# EUROPEAN PATENT APPLICATION

(11) **EP 4 810 483 A1**
(43) Date of publication of application: **23.09.2026**
(21) Application number: 24890761.0
(22) Date of filing: 14.11.2024
(51) Int. Cl.: C07D 209/30, C07D 209/32, C07D 401/04, C07D 471/04, C07D 235/00, C07D 235/02

(54) **6- AND 5-HETEROARYL DERIVATIVES AND USE THEREOF**

(30) Priority: 15.11.2023 CN 202311531006; 07.12.2023 CN 202311689492; 28.12.2023 CN 202311855147; 18.01.2024 CN 202410077837; 21.03.2024 CN 202410329802; 08.11.2024 CN 202411597445
(71) Applicant: CMS Research & Development Pte. Ltd., Singapore 179803 (SG)
(72) Inventor: CHEN, Shuhui, Shanghai 200131 (CN); HE, Haiying, Shanghai 200131 (CN); LI, Peng, Shanghai 200131 (CN); ZHANG, Hongbo, Shanghai 200131 (CN)
(74) Representative: Maiwald GmbH
(86) International application number: PCT/CN2024/132011
(87) International publication number: WO 2025/103406

(57) **Abstract**

Disclosed in the present invention are 6,5-fused heteroaryl derivatives and a use thereof, specifically the compound shown in formula (IV) and a pharmaceutically acceptable salt thereof.

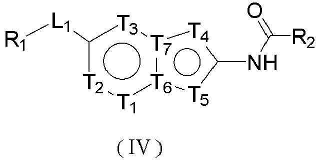

## Description

### The present disclosure claims the priority and benefit of the following:

1) Chinese Patent Application No. 202311531006.7 filed with the China National Intellectual Property Administration on November 15, 2023; 2) Chinese Patent Application No. 202311689492.5 filed with the China National Intellectual Property Administration on December 7, 2023; 3) Chinese Patent Application No. 202311855147.4 filed with the China National Intellectual Property Administration on December 28, 2023; 4) Chinese Patent Application No. 202410077837.X filed with the China National Intellectual Property Administration on January 18, 2024; 5) Chinese Patent Application No. 202410329802.0 filed with the China National Intellectual Property Administration on March 21, 2024; and 6) Chinese Patent Application No. 202411597445.2 filed with the China National Intellectual Property Administration on November 8, 2024. The disclosures of said applications are incorporated herein by reference in their entireties.

### FIELD OF THE INVENTION

The present disclosure relates to a class of 6,5-fused heteroaryl derivatives and uses thereof, and specifically relates to a compound represented by formula (IV) and a pharmaceutically acceptable salt thereof.

### BACKGROUND OF THE INVENTION

Epilepsy is a common neurological disorder affecting approximately 65 million people worldwide. Epilepsy is a group of brain disorders characterized by epileptic seizures resulting from abnormally synchronous and rhythmic neuronal activity in the brain. Spontaneously recurrent seizures are the hallmark of epilepsy. The main clinical symptoms of epilepsy include convulsions, loss of consciousness, myoclonus, hypotonia, and prolonged duration of muscle contractions. Epilepsies are life-altering as they cause learning disabilities and developmental abnormalities in children. Epileptic events can also be life-threatening because tonic-clonic (convulsive) seizures can cause uncontrolled muscle contractions, leading to injury.

Activation of Kv7, which includes five members (Kv7.1-5), can reduce neuronal excitability and has been shown to be an effective treatment for epilepsy. The Kv7 (KCNQ) subfamily of potassium ion channels comprises five members, each exhibiting a distinct tissue distribution and physiological role. Kv7.2 and Kv7.3 are the most abundant Kv7 subunits in the central and peripheral nervous systems; heteromeric channels composed of Kv7.2 and Kv7.3 subunits are believed to underlie the potassium ion current known as the M-current, whose activation reduces neuronal excitability by driving the membrane potential towards a value close to the potassium equilibrium potential, thereby limiting repetitive firing and leading to spike frequency adaptation. Retigabine (RTG) is a Kv7 potassium ion channel activator that can recognize a hydrophobic pocket located in the pore domain, opening and stabilizing the potassium channel. It was the first approved neuronal potassium channel agonist for the treatment of epilepsy. However, RTG was withdrawn from the market in 2017 due to its ability to cause permanent retinal abnormalities, peripheral skin discoloration, bladder dysfunction, and other side effects.

XEN1101 is a novel positive allosteric modulator of Kv7.2-7.3 (KCNQ2/3) potassium channels in neurons. Phase 3 clinical trials are designed to evaluate XEN1101 as adjunctive therapy for focal onset seizures (FOS) and primary generalized tonic-clonic seizures (PGTCS). In a Phase 2b clinical trial, XEN1101 demonstrated a statistically significant and dose-dependent reduction in monthly focal seizure frequency from baseline compared to placebo. A small molecule oral selective KCNQ2/3 opener (CB03) for the treatment of refractory epilepsy has completed first-in-human dosing in healthy subjects in a Phase 1 clinical trial in the United States. These next-generation Kv7 potassium ion channel openers will bring new hope to clinical patients.

### SUMMARY OF THE INVENTION

The present disclosure provides a compound represented by formula (IV) or a pharmaceutically acceptable salt thereof, 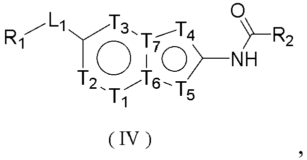 wherein,
L₁ is selected from CH₂, NH, O and S;
T₁, T₂ and T₃ are each independently selected from CR₃ and N;
T₄ and T₅ are each independently selected from CR₄, N and NR₄;
T₆ and T₇ are each independently selected from C and N;
R₁ is selected from phenyl, 5- to 10-membered heteroaryl, 6- to 10-membered heterocycloalkyl and 6- to 10-membered heterocycloalkenyl, wherein said phenyl, 5- to 10-membered heteroaryl, 6- to 10-membered heterocycloalkyl and 6- to 10-membered heterocycloalkenyl are each independently optionally substituted with 1, 2 or 3 R₁ₐ;
or, L₁ is absent, and R₁ is selected from 6- to 10-membered heterocycloalkyl and 6- to 10-membered heterocycloalkenyl, wherein said 6- to 10-membered heterocycloalkyl and 6- to 10-membered heterocycloalkenyl are each independently optionally substituted with 1, 2 or 3 R₁ₐ;
R₂ is selected from C₁₋₆ alkyl, -C₁₋₃ alkyl-C₃₋₆ cycloalkyl, -C₁₋₃ alkyl-(4- to 6-membered heterocycloalkyl) and C₃₋₆ cycloalkyl, wherein said C₁₋₆ alkyl, -C₁₋₃ alkyl-C₃₋₆ cycloalkyl, -C₁₋₃ alkyl-(4- to 6-membered heterocycloalkyl) and C₃₋₆ cycloalkyl are each independently optionally substituted with 1, 2, 3, 4 or 5 R₂ₐ;
R₃ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₃ₐ;
R₄ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₄ₐ;
each R₁ₐ, each R₂ₐ, each R₃ₐ and each R₄ₐ are each independently selected from H, F, Cl, Br, I, CN, C₁₋₄ alkyl and C₁₋₄ alkoxy, wherein said C₁₋₄ alkyl and C₁₋₄ alkoxy are each independently optionally substituted with 1, 2, 3, 4 or 5 R;
or, two R₁ₐ together with the atoms to which they are attached form a C₅₋₆ cycloalkyl, C₅₋₆ cycloalkenyl, phenyl or 5- to 6-membered heteroaryl, wherein said C₅₋₆ cycloalkyl, C₅₋₆ cycloalkenyl, phenyl or 5- to 6-membered heteroaryl is optionally substituted with 1, 2 or 3 R;
each R is independently selected from H, F, Cl, Br and I.

The present disclosure also provides a compound represented by formula (III) or a pharmaceutically acceptable salt thereof, 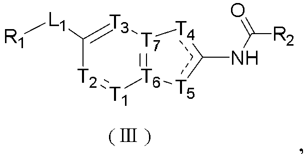 wherein,
-̅ -̅ -̅ is selected from a single bond and a double bond, and the structural unit 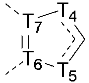 is an aromatic ring;
L₁ is selected from CH₂, NH, O and S;
T₁, T₂ and T₃ are each independently selected from CR₃ and N;
T₄ and T₅ are each independently selected from CR₄, N and NR₄;
T₆ and T₇ are each independently selected from C and N;
R₁ is selected from phenyl, 5- to 10-membered heteroaryl, 6- to 10-membered heterocycloalkyl and 6- to 10-membered heterocycloalkenyl, wherein said phenyl, 5- to 10-membered heteroaryl, 6- to 10-membered heterocycloalkyl and 6- to 10-membered heterocycloalkenyl are each independently optionally substituted with 1, 2 or 3 R₁ₐ;
or, L₁ is absent, and R₁ is selected from 6- to 10-membered heterocycloalkyl and 6- to 10-membered heterocycloalkenyl, wherein said 6- to 10-membered heterocycloalkyl and 6- to 10-membered heterocycloalkenyl are each independently optionally substituted with 1, 2 or 3 R₁ₐ;
R₂ is selected from C₁₋₆ alkyl, -C₁₋₃ alkyl-C₃₋₆ cycloalkyl and -C₁₋₃ alkyl-(4- to 6-membered heterocycloalkyl), wherein said C₁₋₆ alkyl, -C₁₋₃ alkyl-C₃₋₆ cycloalkyl and -C₁₋₃ alkyl-(4- to 6-membered heterocycloalkyl) are each independently optionally substituted with 1, 2, 3, 4 or 5 R₂ₐ;
R₃ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₃ₐ;
R₄ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₄ₐ;
each R₁ₐ, each R₂ₐ, each R₃ₐ and each R₄ₐ are each independently selected from H, F, Cl, Br, I, CN, C₁₋₄ alkyl and C₁₋₄ alkoxy, wherein said C₁₋₄ alkyl and C₁₋₄ alkoxy are each independently optionally substituted with 1, 2, 3, 4 or 5 R;
or, two R₁ₐ together with the atoms to which they are attached form a C₅₋₆ cycloalkyl, C₅₋₆ cycloalkenyl, phenyl or 5- to 6-membered heteroaryl, wherein said C₅₋₆ cycloalkyl, C₅₋₆ cycloalkenyl, phenyl or 5- to 6-membered heteroaryl is optionally substituted with 1, 2 or 3 R;
each R is independently selected from H, F, Cl, Br and I.

The present disclosure provides a compound represented by formula (I) or a pharmaceutically acceptable salt thereof, 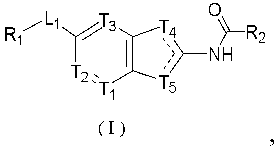 wherein,
-̅-̅-̅ is selected from a single bond and a double bond, and the structural unit 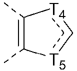 is an aromatic ring;
L₁ is selected from CH₂, NH, O and S;
T₁, T₂ and T₃ are each independently selected from CR₃ and N;
T₄ and T₅ are each independently selected from CR₄, N and NR₄;
R₁ is selected from phenyl, 5- to 10-membered heteroaryl, 6- to 10-membered heterocycloalkyl and 6- to 10-membered heterocycloalkenyl, wherein said phenyl, 5- to 10-membered heteroaryl, 6- to 10-membered heterocycloalkyl and 6- to 10-membered heterocycloalkenyl are each independently optionally substituted with 1, 2 or 3 R₁ₐ;
or, L₁ is absent, and R₁ is selected from 6- to 10-membered heterocycloalkyl and 6- to 10-membered heterocycloalkenyl, wherein said 6- to 10-membered heterocycloalkyl and 6- to 10-membered heterocycloalkenyl are each independently optionally substituted with 1, 2 or 3 R₁ₐ;
R₂ is selected from C₁₋₆ alkyl, -C₁₋₃ alkyl-C₃₋₆ cycloalkyl and -C₁₋₃ alkyl-(4- to 6-membered heterocycloalkyl), wherein said C₁₋₆ alkyl, -C₁₋₃ alkyl-C₃₋₆ cycloalkyl and -C₁₋₃ alkyl-(4- to 6-membered heterocycloalkyl) are each independently optionally substituted with 1, 2, 3, 4 or 5 R₂ₐ;
R₃ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₃ₐ;
R₄ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₄ₐ;
each R₁ₐ, each R₂ₐ, each R₃ₐ and each R₄ₐ are each independently selected from H, F, Cl, Br, I, CN, C₁₋₄ alkyl and C₁₋₄ alkoxy, wherein said C₁₋₄ alkyl and C₁₋₄ alkoxy are each independently optionally substituted with 1, 2, 3, 4 or 5 R;
or, two R₁ₐ together with the atoms to which they are attached form a C₅₋₆ cycloalkyl, C₅₋₆ cycloalkenyl, phenyl or 5- to 6-membered heteroaryl, wherein said C₅₋₆ cycloalkyl, C₅₋₆ cycloalkenyl, phenyl or 5- to 6-membered heteroaryl is optionally substituted with 1, 2 or 3 R;
each R is independently selected from H, F, Cl, Br and I.

The present disclosure also provides a compound represented by formula (I) or a pharmaceutically acceptable salt thereof, 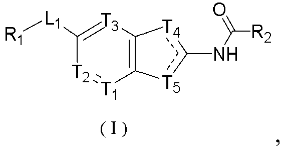 wherein,
-̅-̅-̅ is selected from a single bond and a double bond, and the structural unit 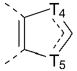 is an aromatic ring;
L₁ is selected from CH₂, NH, O and S;
T₁, T₂, T₃, T₄ and T₅ are each independently selected from CR₃ and N;
R₁ is selected from phenyl and 5- to 10-membered heteroaryl, wherein said phenyl and 5- to 10-membered heteroaryl are each independently optionally substituted with 1, 2 or 3 R₁ₐ;
R₂ is selected from C₁₋₆ alkyl, -C₁₋₃ alkyl-C₃₋₆ cycloalkyl and -C₁₋₃ alkyl-(4- to 6-membered heterocycloalkyl), wherein said C₁₋₆ alkyl, -C₁₋₃ alkyl-C₃₋₆ cycloalkyl and -C₁₋₃ alkyl-(4- to 6-membered heterocycloalkyl) are each independently optionally substituted with 1, 2, 3, 4 or 5 R₂ₐ;
R₃ is selected from H, F, Cl, Br, I, CN, C₁₋₄ alkyl, C₁₋₄ alkoxy, C₃₋₆ cycloalkyl and 4-to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₃ₐ;
each R₁ₐ, each R₂ₐ and each R₃ₐ are each independently selected from H, F, Cl, Br, I, CN, C₁₋₄ alkyl and C₁₋₄ alkoxy, wherein said C₁₋₄ alkyl and C₁₋₄ alkoxy are each independently optionally substituted with 1, 2, 3, 4 or 5 R;
or, two R₁ₐ on adjacent atoms are taken together to form a C₅₋₆ cycloalkyl or C₅₋₆ cycloalkenyl, wherein said C₅₋₆ cycloalkyl or C₅₋₆ cycloalkenyl is optionally substituted with 1, 2 or 3 R;
each R is independently selected from H, F, Cl, Br and I.

In some technical solutions of the present disclosure, the above-mentioned each R₁ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R, and other variables are as defined in the present disclosure.

In some technical solutions of the present disclosure, the above-mentioned each R₁ₐ is independently selected from H, F, Cl, CH₃, CH₂F, CHF₂, CF₃ and OCH₃, and other variables are as defined in the present disclosure.

In some technical solutions of the present disclosure, the above-mentioned each R₁ₐ is independently selected from H, F, Cl, CH₃, CH₂F, CHF₂ and CF₃, and other variables are as defined in the present disclosure.

In some technical solutions of the present disclosure, the above-mentioned two R₁ₐ together with the atoms to which they are attached form a cyclopentenyl, cyclohexenyl, phenyl or thienyl, wherein the cyclopentenyl, cyclohexenyl, phenyl or thienyl is optionally substituted with 1, 2 or 3 R, and other variables are as defined in the present disclosure.

In some technical solutions of the present disclosure, the above-mentioned two R₁ₐ on adjacent atoms are taken together to form a cyclopentenyl or cyclohexenyl, wherein the cyclopentenyl or cyclohexenyl is optionally substituted with 1, 2 or 3 R, and other variables are as defined in the present disclosure.

In some technical solutions of the present disclosure, the above-mentioned each R₂ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R, and other variables are as defined in the present disclosure.

In some technical solutions of the present disclosure, the above-mentioned each R₂ₐ is independently selected from H, F, Cl, CH₃, CH₂F, CHF₂ and CF₃, and other variables are as defined in the present disclosure.

In some technical solutions of the present disclosure, the above-mentioned each R₃ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R, and other variables are as defined in the present disclosure.

In some technical solutions of the present disclosure, the above-mentioned each R₃ₐ is independently selected from H, F, Cl, CH₃, CH₂F, CHF₂ and CF₃, and other variables are as defined in the present disclosure.

In some technical solutions of the present disclosure, the above-mentioned each R₄ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R, and other variables are as defined in the present disclosure.

In some technical solutions of the present disclosure, the above-mentioned each R₄ₐ is independently selected from H, F, Cl, CH₃, CH₂F, CHF₂ and CF₃, and other variables are as defined in the present disclosure.

In some technical solutions of the present disclosure, the above-mentioned L₁ is selected from NH, O and S, and other variables are as defined in the present disclosure.

In some technical solutions of the present disclosure, the above-mentioned L₁ is selected from NH, and other variables are as defined in the present disclosure.

In some technical solutions of the present disclosure, the above-mentioned R₁ is selected from phenyl, pyridinyl, pyrimidinyl, pyrazinyl, pyridazinyl, thienyl, thiazolyl, pyrrolyl, imidazolyl, pyrazolyl, 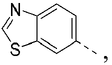 piperidinyl, piperazinyl, morpholinyl, homopiperidinyl, homopiperazinyl, 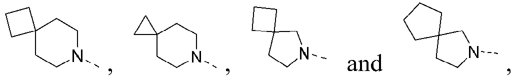 wherein said phenyl, pyridinyl, pyrimidinyl, pyrazinyl, pyridazinyl, thienyl, thiazolyl, pyrrolyl, imidazolyl, pyrazolyl, 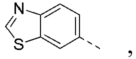 piperidinyl, piperazinyl, morpholinyl, homopiperidinyl, homopiperazinyl, 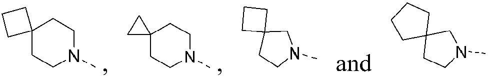 are each independently optionally substituted with 1, 2 or 3 R₁ₐ, and other variables are as defined in the present disclosure.

In some technical solutions of the present disclosure, the above-mentioned R₁ is selected from 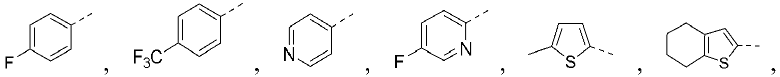 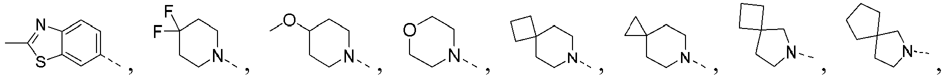 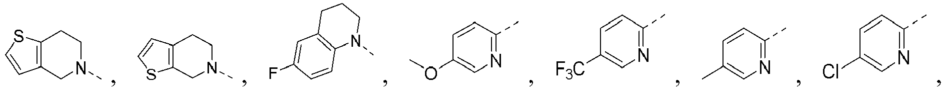 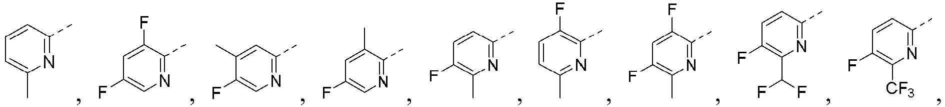 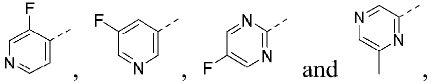 and other variables are as defined in the present disclosure.

In some technical solutions of the present disclosure, the above-mentioned R₁ is selected from 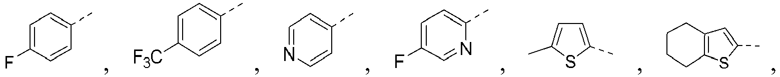 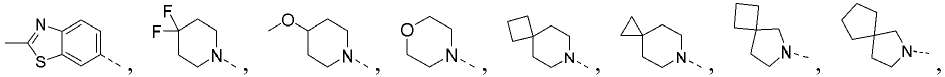 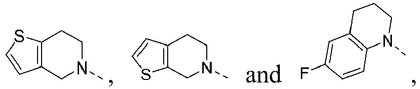 and other variables are as defined in the present disclosure.

In some technical solutions of the present disclosure, the above-mentioned R₁ is selected from 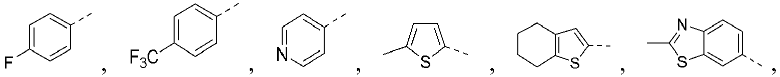 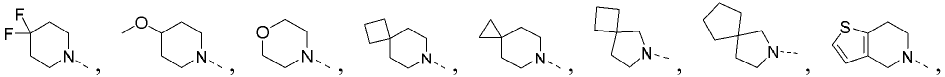 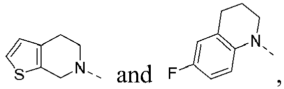 and other variables are as defined in the present disclosure.

In some technical solutions of the present disclosure, the above-mentioned R₁ is selected from phenyl, pyridinyl, pyrimidinyl, pyrazinyl, pyridazinyl, thienyl, thiazolyl, pyrrolyl, imidazolyl, pyrazolyl and 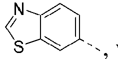 wherein said phenyl, pyridinyl, pyrimidinyl, pyrazinyl, pyridazinyl, thienyl, thiazolyl, pyrrolyl, imidazolyl, pyrazolyl and 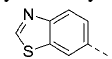 are each independently optionally substituted with 1, 2 or 3 R₁ₐ, and other variables are as defined in the present disclosure.

In some technical solutions of the present disclosure, the above-mentioned R₁ is selected from 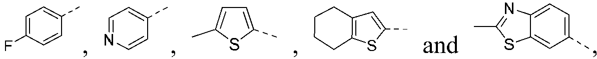 and other variables are as defined in the present disclosure.

In some technical solutions of the present disclosure, the above-mentioned R₁ is selected from phenyl and pyridinyl, wherein said phenyl and pyridinyl are each independently optionally substituted with 1, 2 or 3 R₁ₐ, and other variables are as defined in the present disclosure.

In some technical solutions of the present disclosure, the above-mentioned R₁ is selected from 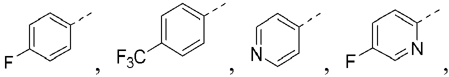 and other variables are as defined in the present disclosure.

In some technical solutions of the present disclosure, the above-mentioned R₂ is selected from CH₂CH₃, CH₂C(CH₃)₃, -CH₂-cyclopropyl, -CH₂-cyclobutyl, 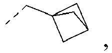 cyclopropyl, cyclobutyl, cyclopentyl, cyclohexyl and 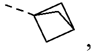 wherein said CH₂CH₃, CH₂C(CH₃)₃, -CH₂-cyclopropyl, -CH₂-cyclobutyl, 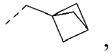 cyclopropyl, cyclobutyl, cyclopentyl, cyclohexyl and 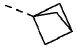 are each independently optionally substituted with 1, 2, 3, 4 or 5 R₂ₐ, and other variables are as defined in the present disclosure.

In some technical solutions of the present disclosure, the above-mentioned R₂ is selected from CH₂CH₃, CH₂C(CH₃)₃, 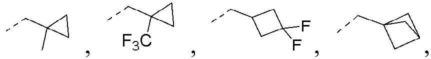 cyclopropyl, cyclobutyl, cyclopentyl and 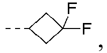 and other variables are as defined in the present disclosure.

In some technical solutions of the present disclosure, the above-mentioned R₂ is selected from CH₂C(CH₃)₃, -CH₂-cyclopropyl, -CH₂-cyclobutyl and 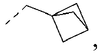 wherein said CH₂C(CH₃)₃, -CH₂-cyclopropyl, -CH₂-cyclobutyl and 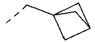 are each independently optionally substituted with 1, 2, 3, 4 or 5 R₂ₐ, and other variables are as defined in the present disclosure.

In some technical solutions of the present disclosure, the above-mentioned R₂ is selected from CH₂C(CH₃)₃, 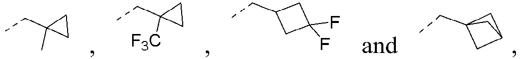 and other variables are as defined in the present disclosure.

In some technical solutions of the present disclosure, the above-mentioned R₃ is selected from H, F, Cl, CN, CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 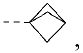 azetidinyl and oxetanyl, wherein said CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 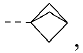 azetidinyl and oxetanyl are each independently optionally substituted with 1, 2 or 3 R₃ₐ, and other variables are as defined in the present disclosure.

In some technical solutions of the present disclosure, the above-mentioned R₃ is selected from H, F, Cl, CN, CH₃ and CF₃, and other variables are as defined in the present disclosure.

In some technical solutions of the present disclosure, the above-mentioned R₄ is selected from H, F, Cl, CN, CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 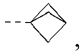 azetidinyl and oxetanyl, wherein said CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 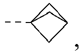 azetidinyl and oxetanyl are each independently optionally substituted with 1, 2 or 3 R₄ₐ, and other variables are as defined in the present disclosure.

In some technical solutions of the present disclosure, the above-mentioned R₄ is selected from H, F, Cl, CN, CH₃, CF₃, CH₂CH₃, C(CH₃)₃, 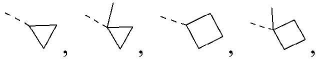 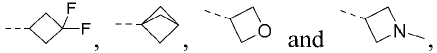 and other variables are as defined in the present disclosure.

In some technical solutions of the present disclosure, the above-mentioned R₄ is selected from H, F, Cl, CN, CH₃, CF₃, CH₂CH₃, C(CH₃)₃, 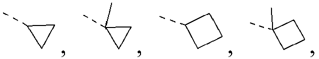 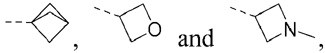 and other variables are as defined in the present disclosure.

In some technical solutions of the present disclosure, the above-mentioned structural unit 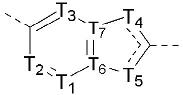 is selected from 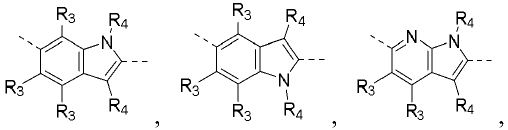 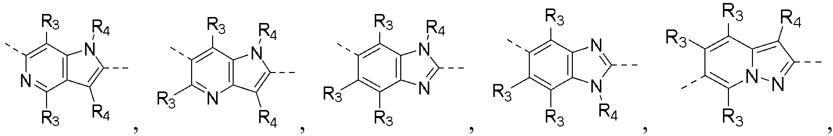 and other variables are as defined in the present disclosure.

In some technical solutions of the present disclosure, the above-mentioned structural unit 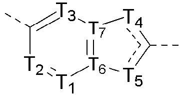 is selected from 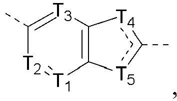 and other variables are as defined in the present disclosure.

In some technical solutions of the present disclosure, the above-mentioned structural unit 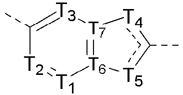 is selected from 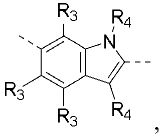 and other variables are as defined in the present disclosure.

In some technical solutions of the present disclosure, the above-mentioned structural unit 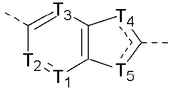 is selected from 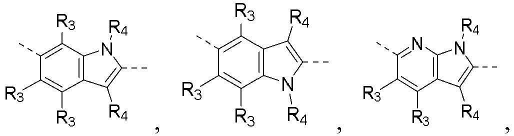 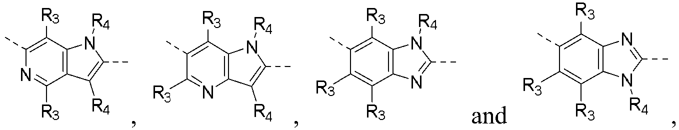 and other variables are as defined in the present disclosure.

In some technical solutions of the present disclosure, the above-mentioned structural unit 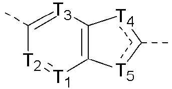 is selected from 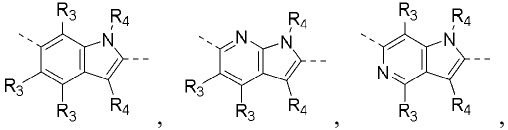 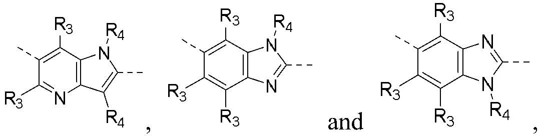 and other variables are as defined in the present disclosure.

In some technical solutions of the present disclosure, the above-mentioned compound or a pharmaceutically acceptable salt thereof is selected from: 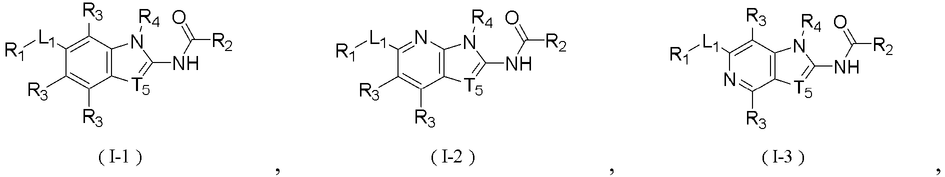 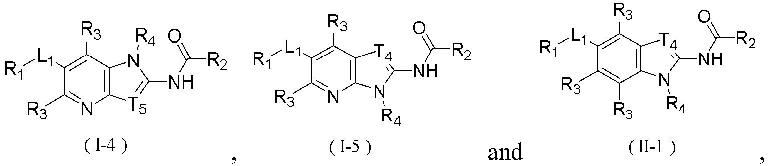
wherein, T₄ and T₅ are each independently selected from CR₄ and N;
R₁, R₂, R₃, R₄ and L₁ are as defined in the present disclosure.

In some technical solutions of the present disclosure, the above-mentioned compound represented by formula (I-1) or (II-1), or a pharmaceutically acceptable salt thereof, is selected from: 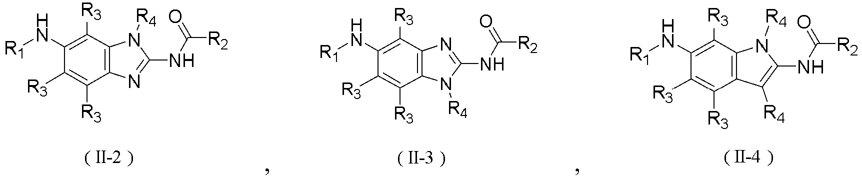 and 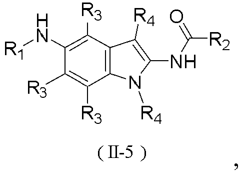 wherein,
R₁ is selected from phenyl, 5- to 6-membered heteroaryl and 6- to 10-membered heterocycloalkyl, wherein said phenyl, 5- to 6-membered heteroaryl and 6- to 10-membered heterocycloalkyl are each independently optionally substituted with 1, 2 or 3 R₁ₐ;
R₂ is selected from CH₂C(CH₃)₃, 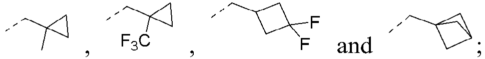
R₃ is selected from H, F, Cl, CN, CH₃ and CF₃;
R₄ is selected from H, F, Cl, CH₃, C(CH₃)₃, cyclopropyl, cyclobutyl, 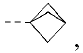 azetidinyl and oxetanyl, wherein said CH₃, C(CH₃)₃, cyclopropyl, cyclobutyl, 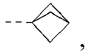 azetidinyl and oxetanyl are each independently optionally substituted with 1, 2 or 3 R₄ₐ;
each R₁ₐ is independently selected from H, F, CH₃, CF₃ and OCH₃;
each R₄ₐ is independently selected from H, F, CH₃ and CF₃.

In some technical solutions of the present disclosure, the above-mentioned compound represented by formula (I), (I-1), (II-1), (II-2) or (II-3), or a pharmaceutically acceptable salt thereof, wherein, R₁ is selected from phenyl and 5- to 6-membered heteroaryl, wherein said phenyl and 5- to 6-membered heteroaryl are each independently optionally substituted with 1, 2 or 3 R₁ₐ, and other variables are as defined in the present disclosure.

In some technical solutions of the present disclosure, the above-mentioned compound represented by formula (I), (I-1), (II-1), (II-2) or (II-3), or a pharmaceutically acceptable salt thereof, wherein, R₁ is selected from phenyl and pyridinyl, wherein said phenyl and pyridinyl are each independently optionally substituted with 1, 2 or 3 R₁ₐ, and other variables are as defined in the present disclosure.

In some technical solutions of the present disclosure, the above-mentioned compound represented by formula (I-1) or (II-4), or a pharmaceutically acceptable salt thereof, is selected from: 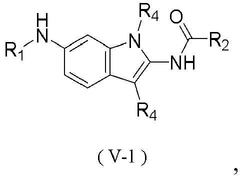 wherein,
R₁ is phenyl and 5- to 6-membered heteroaryl, wherein said phenyl and 5- to 6-membered heteroaryl are each independently optionally substituted with 1, 2 or 3 R₁ₐ;
R₂ is selected from CH₂C(CH₃)₃ and 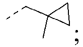
R₄ is selected from H, CH₃, cyclopropyl, cyclobutyl and 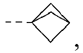 wherein said CH₃, cyclopropyl, cyclobutyl and 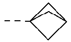 are each independently optionally substituted with 1, 2 or 3 R₄ₐ;
each R₁ₐ is independently selected from H, F, CH₃, CF₃ and OCH₃;
each R₄ₐ is independently selected from H and F.

In some technical solutions of the present disclosure, the above-mentioned compound represented by formula (V-1), or a pharmaceutically acceptable salt thereof, wherein, R₁ is selected from phenyl and pyridinyl, wherein said phenyl and pyridinyl are each independently optionally substituted with 1, 2 or 3 R₁ₐ, and other variables are as defined in the present disclosure.

In some technical solutions of the present disclosure, the above-mentioned compound represented by formula (V-1), or a pharmaceutically acceptable salt thereof, wherein, R₁ is selected from pyridinyl, wherein the pyridinyl is optionally substituted with 1, 2 or 3 R₁ₐ, and other variables are as defined in the present disclosure.

The present disclosure also includes some solutions obtained by any combination of the above variables.

The present disclosure also provides a compound represented below or a pharmaceutically acceptable salt thereof.

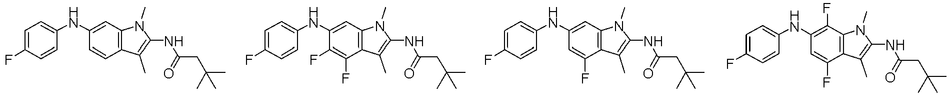

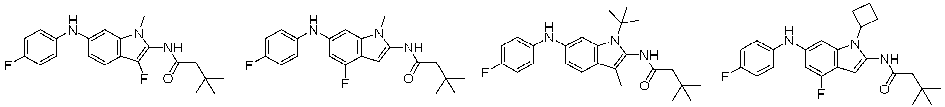

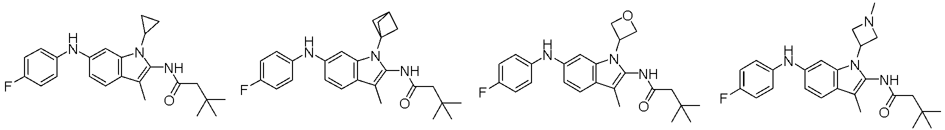

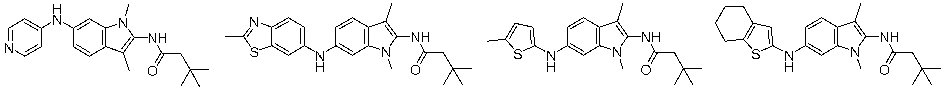

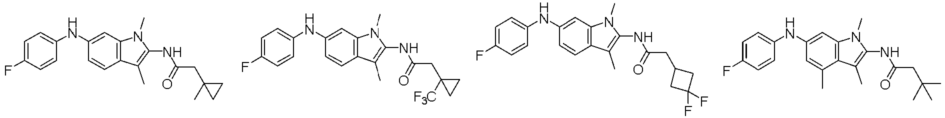

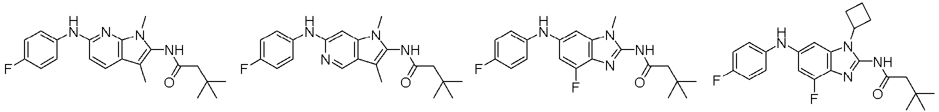

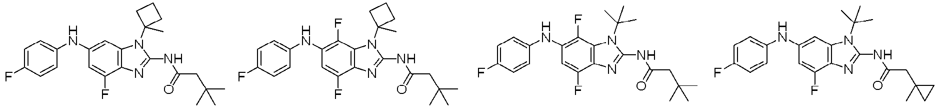

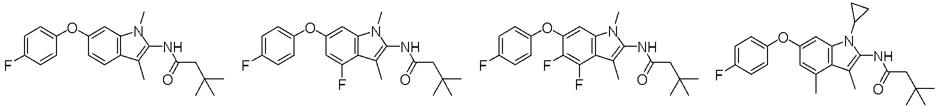

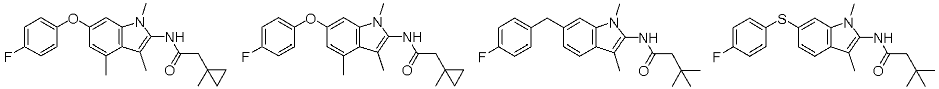

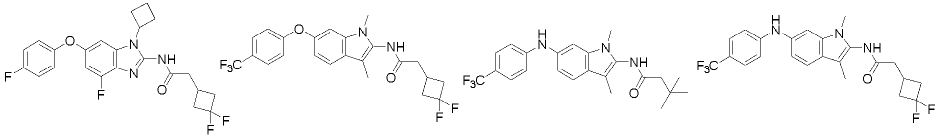

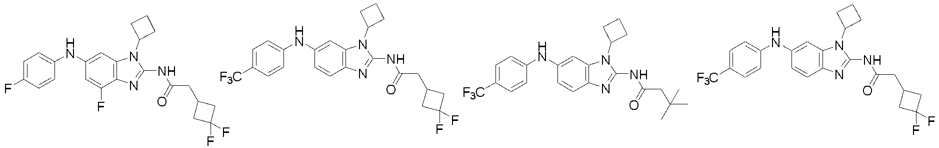

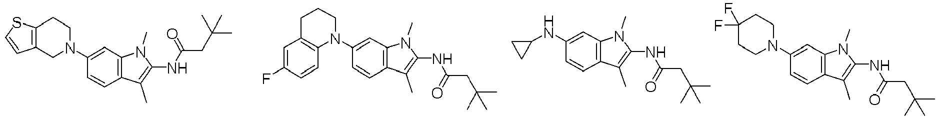

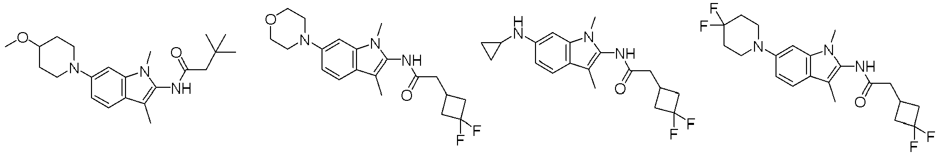

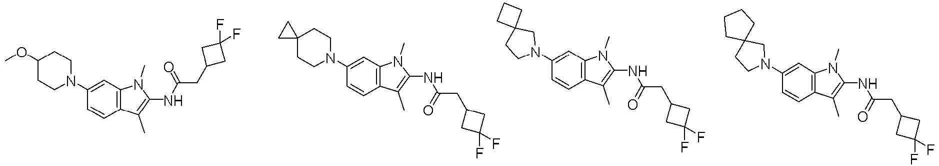

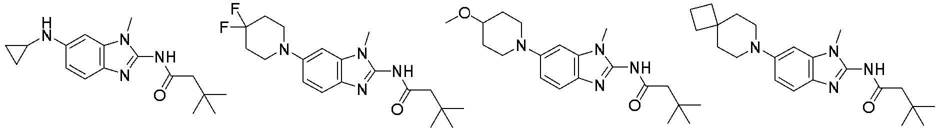

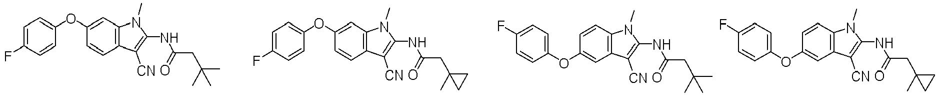

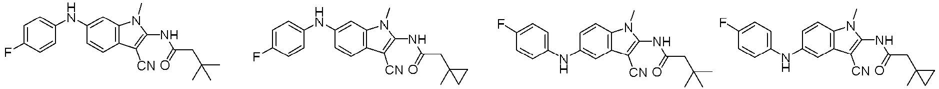

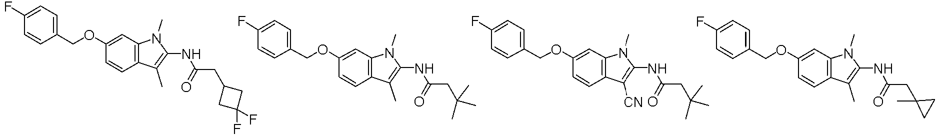

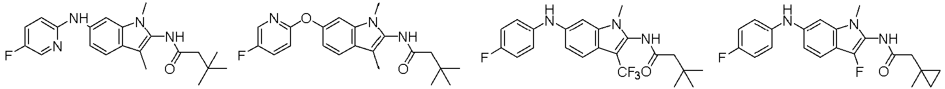

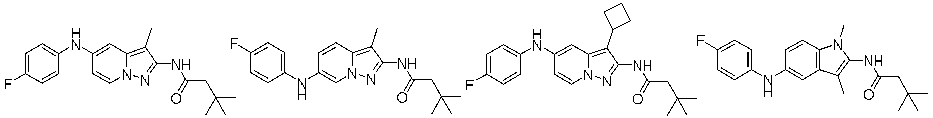

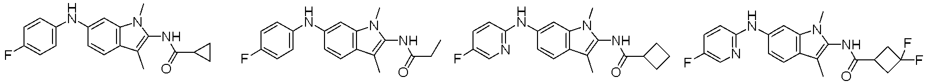

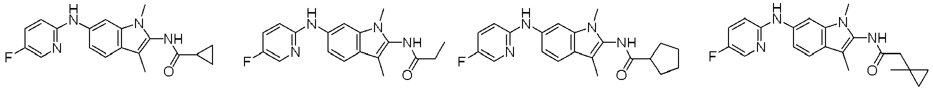

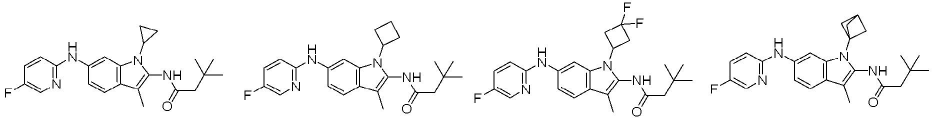

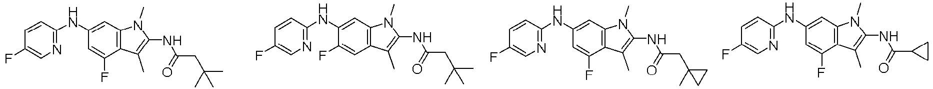

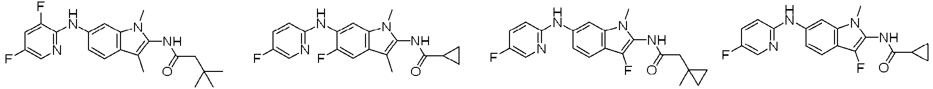

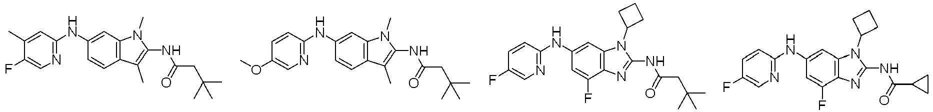

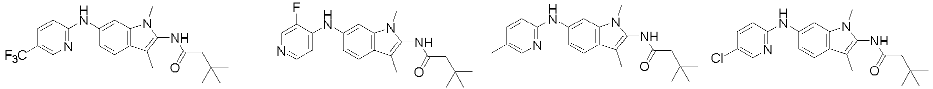

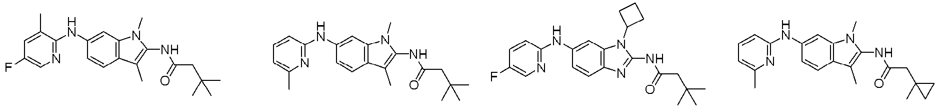

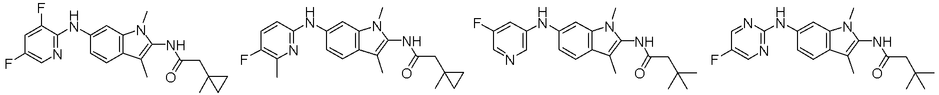

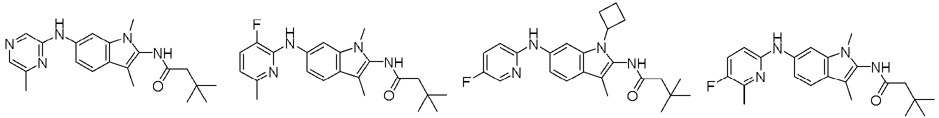

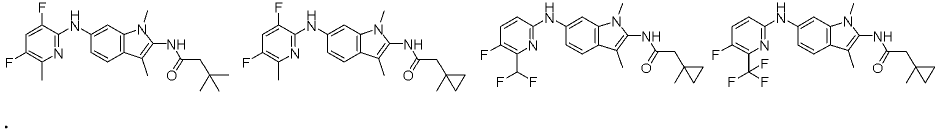

The present disclosure also provides a pharmaceutical composition, which comprises a compound according to any one of the preceding claims or a pharmaceutically acceptable salt thereof.

The present disclosure also provides the use of the compound or a pharmaceutically acceptable salt thereof, or the pharmaceutical composition as described above, in the manufacture of a medicament for treating a disease associated with a Kv7 potassium ion channel opener.

In some embodiments of the present disclosure, in the above use, the disease associated with a Kv7 potassium channel opener is selected from epilepsy.

The present disclosure also provides the following test methods:

### Test Method 1: hKCNQ2/3 Thallium Flux Assay

Test Objective: To test the in vitro concentration-response relationship of compounds using FLIPR thallium flux assay in CHO cell lines stably expressing hKCNQ2/3.

### Test Cells and Reagents:

(1) Cell line: CHO cells stably transfected with hKCNQ2/3.
(2) Cell culture medium reagents: including F12 medium, fetal bovine serum (FBS, 10%), penicillin-streptomycin (Invitrogen, Cat. No. 15140-122), G418 (Invitrogen, Cat. No. 10131027).
(3) Cell culture:
   This cell line was passaged three times per week at a split ratio of 1:2 to 1:3. When the cells grew to >80% confluence in a T-75 flask, they were digested with 0.25% trypsin-EDTA solution for approximately 1 minute, and then transferred from the flask for passaging. According to the split ratio, the cells were transferred to another T-75 flask containing complete cell growth medium. Note: To maintain logarithmic growth, the cells should be kept in a subconfluent monolayer state. Based on the cell doubling time, the cells were passaged every 2-3 days.

### Assay Procedure:

(1) Day 1 - Cell preparation: Cells were digested as described above, and cell density and viability were determined using a Cell Countess. The volume of the single-cell suspension was adjusted with complete cell growth medium. Then, hKCNQ2/3_CHO cells were seeded onto PDL-precoated 384-well plates at a density of approximately 20,000 cells/well (30 µL/well). The plates were then incubated overnight in a 37°C, 5% CO₂ humidified air incubator.
(2) Day 2 - Compound preparation: Test compounds were diluted with dimethyl sulfoxide (DMSO) to stock concentrations and stored in a -20°C freezer. A compound addition program was set up on the ECHO liquid handler, and compounds were added to the plates using the ECHO liquid handler. Retigabine (RTG) was used as a positive control at a highest concentration of 100 µM with 3-fold serial dilutions for a total of 9 doses. Buffer containing 5 mM K⁺ and 1 mM Tl⁺ was added to the plates.
   Notes: 1) K₂SO₄ and Tl₂SO₄ were prepared in 1× chloride-free buffer. Depending on the voltage-gated potassium channel involved, the concentrations of Tl⁺ and K⁺ should be adjusted. Tl⁺ concentration is approximately 0.5-5 mM, and K⁺ concentration is approximately 5-30 mM. 2) Each 1 mM solution of K₂SO₄ and Tl₂SO₄ contains 2 mM K⁺ and Tl⁺; the final concentration should be calculated based on the ion concentration. 3) TlCl readily precipitates and a chloride-free buffer is required.
(3) Day 2 -Assay testing: Once the cells reached the desired confluence, the cell assay plates were removed from the incubator. Assay buffer and diluted Tl⁺ dye (Molecular Devices # R8222) were prepared. The medium was discarded using a Bravo liquid handler, and 25 µL of Tl⁺ dye was added to each well. After 1 hour of incubation, the cell assay plates, composite plates and FLIPR tips were placed in the FLIPR^{TETRA} (Molecular Devices, USA) for FLIPR detection. After setting up the tips, baseline readings were taken for 60 seconds, then 12.5 µL was added from the composite plate (3×) to the cell assay plate, and data were recorded for at least 5 minutes.
(4) Data processing:
   Maximum signals were generated by the FLIPR software. Data analysis was performed using Excel (2013) and Prism 6.01. Data quality control: S/B > 2.00, Z factor > 0.50. IC₅₀ or EC₅₀ was defined as the midpoint between the lowest and highest plateaus, calculated using Prism 6.01 with a four-parameter logistic model

### Test Method 2: Pharmacokinetic Evaluation in Mice

Assay method: Test compounds were mixed with 5% DMSO / 60% polyethylene glycol (PEG400) / 35% water, vortexed and sonicated to obtain a clear 0.2 mg/mL solution, which was then filtered through a microporous membrane before use. Male CD-1 mice weighing 25-35 g were selected. The candidate compound solution was administered intravenously at a dose of 1 mg/kg. The candidate compound solution was orally administered at a dose of 5 mg/kg after fasting. Blood samples (25 µL each) were collected at 0.083, 0.25, 0.5, 1, 2, 4, 8, 12 and 24 hours after administration, and placed into commercial anticoagulant tubes pre-containing EDTA-K2. The tubes were centrifuged for 10 minutes to separate plasma, which was then stored at -60°C. The content of the target compound in plasma samples was determined by LC/MS/MS method, and pharmacokinetic parameters were calculated using Phoenix WinNonlin software (Pharsight, USA).

Definitions of parameters: IV: intravenous administration; PO: oral administration; C₀: instantaneous plasma concentration immediately after IV administration; Cmax: maximum plasma concentration after administration; Tmax: time to reach peak plasma concentration after administration; T_{1/2}: time required for the blood drug concentration to decrease by half; Vdss: (apparent Volume of Distribution) refers to the proportionality constant between the amount of drug in the body and the plasma concentration once the drug has reached a state of dynamic equilibrium within the body; CL: (Clearance) refers to the apparent volume of distribution of drug cleared from the body per unit of time. Tₗₐₛₜ: time of the last detection point. AUC₀₋ₗₐₛₜ: (Area Under the Concentration-Time Curve) refers to the area enclosed by the plasma drug concentration curve and the time axis. F (Bioavailability) is a measure of the rate and extent to which a drug is absorbed into the systemic circulation, serving as a critical indicator for evaluating the degree of drug absorption.

### Technical Effects

The compounds of the present disclosure exhibit good binding to both the Kv7.2 protein and the human Kv7.3 protein with highly homologous binding sites, and show significant agonistic activity on hKCNQ2/3 potassium ion channel, thereby achieving a favorable pharmacological effect.

### Definition and Explanations

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commercial products or active ingredients thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (*R*)- and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is generated by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, a wedged solid bond ( 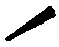 ) and a wedged dashed bond ( 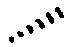 ) indicate the absolute configuration of a stereocenter; a straight solid bond ( 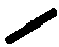 ) and a straight dashed bond ( 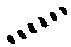 ) indicate the relative configuration of a stereocenter; a wavy line ( 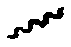 ) indicates a wedged solid bond ( 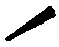 ) or a wedged dashed bond ( 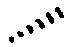 ); or a wavy line ( 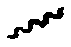 ) indicates a straight solid bond ( 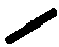 ) or a straight dashed bond ( 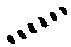 ).

Unless otherwise specified, the term "enriched in one isomer", "isomerically enriched", "enriched in one enantiomer" or "enantiomerically enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomeric excess" or "enantiomeric excess" means the difference between the relative percentages of two isomers or two enantiomers. For example, if one isomer or enantiomer is present in an amount of 90% and the other isomer or enantiomer is present in an amount of 10%, the isomeric or enantiomeric excess (ee value) is 80%.

Optically active (R)- and (S)-isomer, or D and L isomer can be prepared using chiral synthesis or chiral reagents or other conventional techniques. If one kind of enantiomer of certain compound disclosed herein is to be obtained, the pure desired enantiomer can be obtained by asymmetric synthesis or derivatization with chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomer which is then subjected to diastereomeric resolution through the conventional method in the art to afford the pure enantiomer after recovery. In addition, the enantiomer and the diastereoisomer are generally isolated through chromatography which uses a chiral stationary phase and optionally combines with a chemical derivatization method (for example, carbamate generated from amine).

Some compounds of the present disclosure can exist as atropisomers, which are conformational isomers that occur when the rotation around a single bond in the molecule is prevented or significantly slowed down due to the steric interactions with other parts of the molecule. The compounds disclosed herein include all the atropisomers, which can be pure, single atropisomers, or mixtures enriched in one of the atropisomers, or nonspecific mixtures of each. Separation of the isomers can be permitted if the rotational potential around the single bond is sufficiently high and the interconversion between the conformations is sufficiently slow.

Compounds disclosed herein may contain an unnatural proportion of atomic isotopes at one or more of the atoms that constitutes the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "substituted" means that any one or more hydrogen atoms on a particular atom are substituted with a substituent, which can include deuterium and hydrogen variants, provided that the valence state of the particular atom is normal and the substituted compound is stable. When the substituent is oxygen (i.e., =O), it means that two hydrogen atoms are substituted. The term "optionally substituted" means that it may or may not be substituted, and unless otherwise specified, the type and number of substituents can be arbitrary on a chemically feasible basis.

When any variable (e.g., R) appears once or more than once in the composition or structure of a compound, its definition is independent in each case. Thus, for example, if a group is substituted with 0 to 2 R groups, the group can optionally be substituted with at most two R groups, and the R in each case has independent options. Furthermore, a combination of substituents and/or the variants thereof is allowed only in the case that such combination can result in a stable compound.

When the number of a linking group is 0, such as -(CRR)₀-, it indicates that the linking group is a single bond.

When the number of a substituent is 0, it means that the substituent is absent. For example, -A-(R)₀ indicates that the structure is actually -A.

When a substituent is vacant, it means that the substituent is absent. For example, when X is vacant in A-X, it means that the structure is actually A.

When one of the variables is a single bond, it means that the two groups it connects are directly connected. For example, when L in A-L-Z represents a single bond, it means that the structure is actually A-Z.

When an enumerated linking group does not indicate its linking direction, its linking direction is arbitrary. For example, when the linking group L in 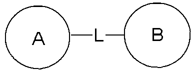 is -M-W-, the -M-W- can be linked to the ring A and the ring B in the same direction as the reading order from left to right to constitute 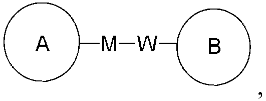 or can be linked to the ring A and the ring B in the reverse direction as the reading order from left to right to constitute 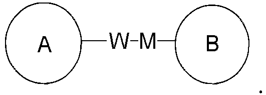 A combination of the linking groups, substituents and/or variants thereof is allowed only when such combination can result in a stable compound.

Unless otherwise specified, when a group has one or more connectable sites, any one or more sites of the group can be connected to other groups through chemical bonds. When the connection mode of the chemical bond is unlocated, and there is H atom(s) at a connectable site(s), when connecting the chemical bond, the number of hydrogen atoms at said site decreases correspondingly with the number of the connected chemical bond, and the group will become a group of corresponding valence. The chemical bond between the site and other groups can be represented by a straight solid bond 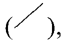 a straight dashed bond 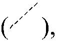 or a wavy line 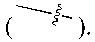 For example, the straight solid bond in -OCH₃ indicates that the group is connected to other groups through the oxygen atom in the group; the straight dashed bond in 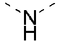 indicates that the group is connected to other groups through two ends of the nitrogen atom in the group; the wavy line in 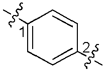 indicates that the group is connected to other groups through the carbon atoms at the 1- and 2-positions in the phenyl group; 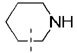 indicates that any connectable site on the piperidyl can be connected to other groups via one chemical bond, including at least four connection modes: 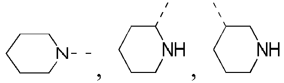 and 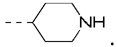 Even if an H atom is drawn on -N-, 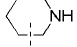 still includes the groups with the connection mode of 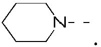 It is just that, when one chemical bond is connected, the H at that site will be reduced by one, and it becomes the corresponding monovalent piperidyl group.

Unless otherwise specified, the number of atoms in a ring is usually defined as the number of the members of the ring. For example, "5- to 7-membered ring" refers to a "ring" in which 5 to 7 atoms are arranged in a circular arrangement.

Unless otherwise specified, the term "aromatic ring" or "aryl ring" refers to a cyclic group having a conjugated π-electron system, in which the interatomic regions are covered by delocalized π-electron clouds. In structural formulas, provided that the valences of atoms and the rules of covalent bonding are satisfied, it may be depicted as alternating single and double bonds, or the delocalized π-electron cloud may be represented by ○. For example, the structural formulas 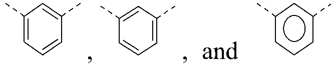 all represent the same structure; the structural formulas 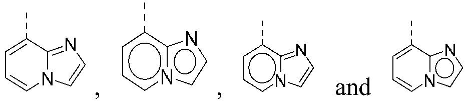 all represent the same structure. It may be a monocyclic or fused polycyclic system, wherein each ring is aromatic. Unless otherwise specified, the ring optionally contains 0, 1 or more heteroatoms independently selected from O, S and N.

Unless otherwise specified, the term "halo" or "halogen" by itself or as a part of another substituent, means a fluorine, chlorine, bromine, or iodine atom.

Unless otherwise specified, the term "C₁₋₆ alkyl", used alone or in combination with other terms, denotes a straight or branched saturated hydrocarbon group having 1 to 6 carbon atoms; the term "C₁₋₄ alkyl", used alone or in combination with other terms, denotes a straight or branched saturated hydrocarbon group having 1 to 4 carbon atoms; the term "C₁₋₃ alkyl", used alone or in combination with other terms, denotes a straight or branched saturated hydrocarbon group having 1 to 3 carbon atoms. It may be monovalent (e.g., methyl), divalent (e.g., methylene) or polyvalent (e.g., methine). Said C₁₋₆ alkyl includes C₁₋₂, C₁₋₃, C₁₋₄, C₂₋₃, C₂₋₄, C₂₋₅, C₄, C₅, and C₆ alkyl, etc.; the C₁₋₄ alkyl includes C₁₋₂, C₁₋₃, and C₂₋₃ alkyl, etc.; the C₁₋₃ alkyl includes C₁, C₂ and C₃ alkyl, etc. Examples of C₁₋₆ alkyl include, but are not limited to, methyl (Me), ethyl (Et), propyl (including n-propyl and isopropyl), butyl (including n-butyl, isobutyl, sec-butyl and tert-butyl), pentyl, etc.; examples of C₁₋₄ alkyl include, but are not limited to, methyl (Me), ethyl (Et), propyl (including n-propyl and isopropyl), butyl (including n-butyl, isobutyl, sec-butyl and tert-butyl), etc.; examples of C₁₋₃ alkyl include, but are not limited to, methyl (Me), ethyl (Et), propyl (including n-propyl and isopropyl), etc.

Unless otherwise specified, the term "C₁₋₄ alkoxy", used alone or in combination with other terms, denotes an alkyl group containing 1 to 4 carbon atoms attached to the remainder of the molecule through an oxygen atom. The C₁₋₄ alkoxy includes C₁₋₃, C₁₋₂, C₂₋₄, C₄ and C₃ alkoxy, etc. It may be monovalent, divalent or polyvalent. Examples of C₁₋₄ alkoxy include, but are not limited to, methoxy, ethoxy, propoxy (including n-propoxy and isopropoxy), butoxy (including n-butoxy, isobutoxy, sec-butoxy and tert-butoxy), etc.

Unless otherwise specified, "C₃₋₆ cycloalkyl" denotes a saturated cyclic hydrocarbon group composed of 3 to 6 carbon atoms, which may be monocyclic or bicyclic. The C₃₋₆ cycloalkyl includes C₃₋₅, C₄₋₅ and C₅₋₆ cycloalkyl, etc.; it may be monovalent, divalent or polyvalent. Examples of C₃₋₆ cycloalkyl include, but are not limited to, cyclopropyl, cyclobutyl, cyclopentyl, cyclohexyl, 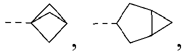 etc.

Unless otherwise specified, "C₅₋₆ cycloalkyl" denotes a saturated cyclic hydrocarbon group composed of 5 to 6 carbon atoms, which may be monocyclic or bicyclic. Said C₅₋₆ cycloalkyl includes C₅ and C₆ cycloalkyl, etc.; it may be monovalent, divalent or polyvalent. Examples of C₅₋₆ cycloalkyl include, but are not limited to, cyclopentyl, cyclohexyl, 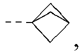 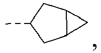 etc.

Unless otherwise specified, "C₅₋₆ cycloalkenyl" denotes a partially unsaturated cyclic hydrocarbon group composed of 5 to 6 carbon atoms containing at least one carbon-carbon double bond, which may be monocyclic or bicyclic, and wherein any ring in the system is non-aromatic. Said C₅₋₆ cycloalkenyl includes C₅ or C₆ cycloalkenyl, etc.; it may be monovalent, divalent or polyvalent. Examples of C₅₋₆ cycloalkenyl include, but are not limited to, cyclopentenyl, cyclopentadienyl, cyclohexenyl, cyclohexadienyl, etc.

Unless otherwise specified, the term "4- to 6-membered heterocycloalkyl", used alone or in combination with other terms, denotes a saturated cyclic group having 4 to 6 ring atoms, in which 1, 2, 3 or 4 ring atoms are heteroatoms independently selected from O, S and N, the remainder being carbon atoms, wherein carbon atoms are optionally oxo-substituted (i.e., C(O)), nitrogen atoms are optionally quaternized, and nitrogen and sulfur heteroatoms may be optionally oxidized (i.e., NO and S(O)p, where p is 1 or 2). It includes monocyclic and bicyclic ring systems. Furthermore, for said "4- to 6-membered heterocycloalkyl", a heteroatom may occupy the position of attachment of the heterocycloalkyl to the rest of the molecule. Said 4-to 6-membered heterocycloalkyl includes 5- to 6-membered, 4-membered, 5-membered and 6-membered heterocycloalkyl, etc. It may be monovalent, divalent or polyvalent. Examples of 4-to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl or hexahydropyridazinyl, etc.

Unless otherwise specified, the term "6- to 10-membered heterocycloalkyl", used alone or in combination with other terms, denotes a saturated cyclic group having 6 to 10 ring atoms, of which 1, 2, 3 or 4 ring atoms are heteroatoms independently selected from O, S and N, the remainder being carbon atoms, wherein carbon atoms are optionally oxo-substituted (i.e., C(O)), nitrogen atoms are optionally quaternized, and nitrogen and sulfur heteroatoms may be optionally oxidized (i.e., NO and S(O)p, where p is 1 or 2). It includes monocyclic, bicyclic and tricyclic ring systems, wherein bicyclic and tricyclic systems include spiro, fused and bridged rings. Furthermore, for said "6- to 10-membered heterocycloalkyl", a heteroatom may occupy the position of attachment of the heterocycloalkyl to the rest of the molecule. Said 6-to 10-membered heterocycloalkyl includes 6- to 8-membered, 6- to 9-membered, 6-membered, 7-membered, 8-membered, 9-membered and 10-membered heterocycloalkyl, etc. Examples of 6- to 10-membered heterocycloalkyl include, but are not limited to, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), hexahydropyridazinyl, homopiperazinyl, homopiperidinyl, dioxepanyl, 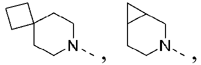 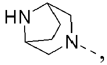 etc.

Unless otherwise specified, the term "6- to 10-membered heterocycloalkenyl", used alone or in combination with other terms, denotes a partially unsaturated cyclic group having 6 to 10 ring atoms containing at least one carbon-carbon double bond, of which 1, 2, 3 or 4 ring atoms are heteroatoms independently selected from O, S and N, the remainder being carbon atoms, wherein carbon atoms are optionally oxo-substituted (i.e., C(O)), nitrogen atoms are optionally quaternized, and nitrogen and sulfur heteroatoms may be optionally oxidized (i.e., NO and S(O)p, where p is 1 or 2). It includes monocyclic, bicyclic and tricyclic ring systems, wherein bicyclic and tricyclic systems include spiro, fused and bridged rings, and any ring in the system is non-aromatic. Furthermore, for said "6- to 10-membered heterocycloalkenyl", a heteroatom may occupy the position of attachment of the heterocycloalkenyl to the rest of the molecule. Said 6- to 10-membered heterocycloalkenyl includes 6- to 8-membered, 6- to 9-membered, 6-membered, 7-membered, 8-membered, 9-membered and 10-membered heterocycloalkenyl, etc. Examples of 6- to 10-membered heterocycloalkenyl include, but are not limited to, tetrahydropyridinyl, dihydropyridinyl, tetrahydropyridazinyl, dihydropyridazinyl, 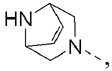 etc.

Unless otherwise specified, the terms "5- to 10-membered heteroaromatic ring" and "5- to 10-membered heteroaryl" are used interchangeably herein. The term "5- to 10-membered heteroaryl" denotes a cyclic group having a conjugated π-electron system composed of 5 to 10 ring atoms, of which 1, 2, 3 or 4 ring atoms are heteroatoms independently selected from O, S and N, the remainder being carbon atoms. Nitrogen atoms are optionally quaternized, and nitrogen and sulfur heteroatoms may be optionally oxidized (i.e., NO and S(O)p, where p is 1 or 2). It may be a monocyclic, bicyclic or tricyclic ring system, wherein each ring is aromatic. The 5- to 10-membered heteroaryl group may be attached to the rest of the molecule through a heteroatom or a carbon atom. Said 5- to 10-membered heteroaryl includes 5- to 9-membered, 5- to 8-membered, 5- to 7-membered, 5- to 6-membered, 5-membered, 6-membered, 7-membered, 8-membered, 9-membered and 10-membered heteroaryl, etc. Examples of said 5-10 membered heteroaryl include, but are not limited to, pyrrolyl (including N-pyrrolyl, 2-pyrrolyl and 3-pyrrolyl, etc.), pyrazolyl (including 2-pyrazolyl and 3-pyrazolyl, etc.), imidazolyl (including N-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1H-1,2,3-triazolyl, 2H-1,2,3-triazolyl, 1H-1,2,4-triazolyl and 4H-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furanyl (including 2-furanyl and 3-furanyl, etc.), thienyl (including 2-thienyl and 3-thienyl, etc.), pyridinyl (including 2-pyridinyl, 3-pyridinyl and 4-pyridinyl, etc.), pyrazinyl, pyrimidinyl (including 2-pyrimidinyl and 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolinyl and 5-isoquinolinyl, etc.), quinoxalinyl (including 2-quinoxalinyl and 5-quinoxalinyl, etc.), or quinolinyl (including 3-quinolinyl and 6-quinolinyl, etc.).

Unless otherwise specified, the terms "5- to 6-membered heteroaromatic ring" and "5-to 6-membered heteroaryl" may be used interchangeably. The term "5- to 6-membered heteroaryl" means a monocyclic group having a conjugated pi electron system and composed of 5 to 6 ring atoms, in which 1, 2, 3 or 4 ring atoms are heteroatoms independently selected from O, S and N, and the remainder ring atoms are carbon atoms, wherein the nitrogen atom is optionally quaternized and the nitrogen and sulfur heteroatoms may be optionally oxidized (i.e., NO and S(O)ₚ, wherein p is 1 or 2). The 5- to 6-membered heteroaryl group may be attached to the remainder of a molecule by a heteroatom or a carbon atom. The 5- to 6-membered heteroaryl group includes 5-membered and 6-membered heteroaryl groups. Examples of the 5-to 6-membered heteroaryl group include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like).

Compounds disclosed herein can be prepared by a variety of synthetic methods well known to those skilled in the art, including the following enumerated embodiment, the embodiment formed by the following enumerated embodiment in combination with other chemical synthesis methods, and equivalent alternatives well known to those skilled in the art. Preferred embodiments include, but are not limited to examples disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-ray diffraction (SXRD). In the single crystal X-ray diffraction (SXRD), the diffraction intensity data of the cultivated single crystal are collected using a Bruker D8 venture diffractometer with a light source of CuKα radiation in a scanning mode of φ/ω scan; after collecting the relevant data, the crystal structure is further analyzed by the direct method (Shelxs97) to confirm the absolute configuration.

The abbreviations used in the present disclosure: prep-HPLC stands for preparative high-performance liquid chromatography separation; prep-TLC stands for preparative thin layer chromatography separation; PE stands for petroleum ether; EA stands for ethyl acetate.

The solvents used in the present disclosure are commercially available. Compounds are named according to general naming principles in the art or by ChemDraw^{®} software, and commercially available compounds are named with their vendor directory names.

### DETAILED DESCRIPTION OF THE INVENTION

The present disclosure is described in detail below by means of examples. However, it is not intended that these examples have any disadvantageous limitations to the present disclosure. Compounds disclosed herein can be prepared by a variety of synthetic methods well known to those skilled in the art, including the following enumerated embodiment, the embodiment formed by the following enumerated embodiment in combination with other chemical synthesis methods, and equivalent well known to those skilled in the art. Preferred embodiments include, but are not limited to examples disclosed herein. It will be apparent to those skilled in the art that various changes and modifications may be made to the embodiments disclosed herein without departing from the spirit and scope disclosed herein.

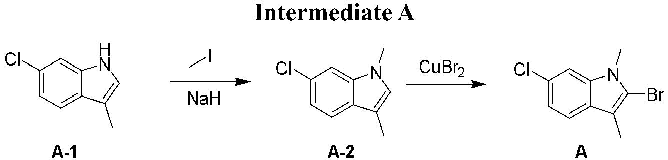

### Step 1

Compound **A-1** (2.5 g, 15.09 mmol) was dissolved in tetrahydrofuran (30 mL), cooled to 0 °C. Sodium hydride (905.69 mg, 22.64 mmol, 60% purity) was added in portions, and the mixture was stirred at 0 °C for 30 min. A solution of iodomethane (30.19 mmol, 1.88 mL) in THF (5 mL) was added dropwise. After the addition, the mixture was slowly warmed to 20 °C and stirred for 2 h. The reaction was quenched by adding water (50 mL) under ice-bath cooling, and extracted with ethyl acetate (50 mL × 3). The organic phase was concentrated under reduced pressure to give compound **A-2.** LCMS (ESI):179.8 [M+H]⁺; ¹HNMR (400 MHz, CDCl₃) δ 7.47 (d, *J*=8.5 Hz, 1H), 7.29 (s, 1H), 7.08 (dd, *J*=2.0, 8.5 Hz, 1H), 6.83 (d, *J*=1.0 Hz, 1H), 3.75 (s, 3H), 2.32 (s, 3H).

### Step 2

Compound **A-2** (1 g, 5.57 mmol), copper(II) bromide (2.49 g, 11.13 mmol) and tetrabutylammonium bromide (179.45 mg, 556.65 µmol) were dissolved in a mixture of water (0.8 mL) and 1,2-dichloroethane (20 mL). The mixture was stirred at 20 °C for 2 h. The mixture was then filtered, and the filtrate was concentrated under reduced pressure. The residue was purified by column chromatography (petroleum ether) to give compound **A.** ¹HNMR (400 MHz, CDCl₃) δ 7.42 (d, *J*=8.5 Hz, 1H), 7.29 - 7.27 (m, 1H), 7.12 - 7.07 (m, 1H), 3.72 (s, 3H), 2.28 (s, 3H).

### Example 1

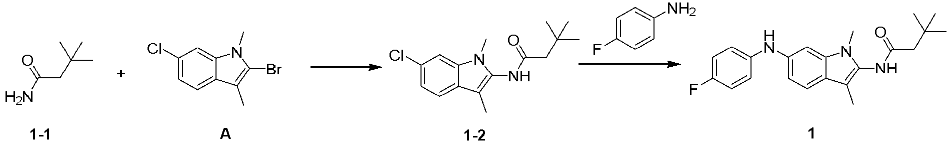

### Step 1

Compound **A** (200 mg, 773.57 µmol), compound **1-1** (178.19 mg, 1.55 mmol), tris(dibenzylideneacetone)dipalladium(0) (70.84 mg, 77.36 µmol), 4,5-bis(diphenylphosphino)-9,9-dimethylxanthene (89.52 mg, 154.71 µmol) and cesium carbonate (756.13 mg, 2.32 mmol) were added to dioxane (8 mL). The mixture was purged with nitrogen three times, heated to 110 °C, and stirred for 12 h. After filtration, the filtrate was concentrated under reduced pressure, and the residue was purified by column chromatography (petroleum ether/ethyl acetate = 10:1) to give compound **1-2.** LCMS (ESI): 292.9 [M+H]⁺; ¹HNMR (400 MHz, CDCl₃) δ 7.44 (d, *J*=8.5 Hz, 1H), 7.26 (s, 1H), 7.12 - 7.04 (m, 1H), 6.84 (br s, 1H), 3.55 (s, 3H), 2.37 (s, 2H), 2.22 (s, 3H), 1.20 (s, 9H).

### Step 2

Compound **1-2** (20.00 mg, 68.31 µmol), 4-fluoroaniline (15.18 mg, 136.61 µmol), 2-dicyclohexylphosphino-2',6'-diisopropoxy-1,1'-biphenyl (6.37 mg, 13.66 µmol) and potassium tert-butoxide (15.33 mg, 136.61 µmol) were dissolved in dioxane (2 mL). Then methanesulfonato(2-dicyclohexylphosphino-2',6'-diisopropoxy-1,1'-biphenyl)(2-amino-1,1'-biphenyl-2-yl)palladium(II) (5.71 mg, 6.83 µmol) was added. After purging with nitrogen three times, the mixture was stirred at 110 °C for 12 h. The reaction mixture was filtered, and the filtrate was concentrated under reduced pressure. The residue was purified by preparative TLC (petroleum ether/ethyl acetate = 3:1) and then by preparative HPLC (column: Welch Xtimate C18 150*25 mm*5 µm; mobile phase: [water (ammonia + ammonium bicarbonate) - acetonitrile]; gradient (acetonitrile%): 50%-80%) to give compound **1.** LCMS (ESI): 368.3 [M+H]⁺; ¹HNMR (400 MHz, CDCl₃) δ 7.46 - 7.40 (m, 1H), 7.07 - 6.93 (m, 5H), 6.91 - 6.81 (m, 2H), 5.70 - 5.58 (m, 1H), 3.51 (s, 3H), 2.35 (s, 2H), 2.22 (s, 3H), 1.20 (s, 9H).

### Example 2

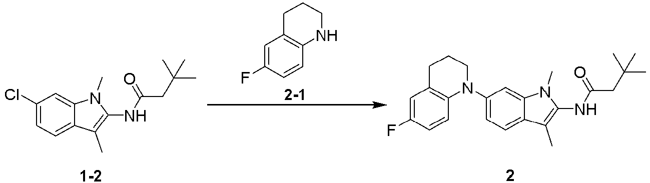

Compound **1-2** (100 mg, 341.53 µmol), compound 2-1 (103.26 mg, 683.05 µmol), 2-dicyclohexylphosphino-2',6'-diisopropoxy-1,1'-biphenyl (31.87 mg, 68.31 µmol) and cesium carbonate (333.83 mg, 1.02 mmol) were dissolved in dioxane (6 mL). Then methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol) was added. After purging with nitrogen three times, the mixture was stirred at 110 °C for 12 h. The mixture was then cooled to 0 °C, and the reaction was quenched by pouring into water (5 mL). The mixture was extracted with ethyl acetate (5 mL × 3). The combined organic phases were concentrated under reduced pressure. The residue was purified by column chromatography (petroleum ether/ethyl acetate = 10:1) to give compound **2.** LCMS (ESI): 408.2 [M+H]⁺. ¹HNMR (400 MHz, DMSO-d₆) δ 9.66 (s, 1H), 7.46 (d, J=8.0 Hz, 1H), 7.22 (d, J=1.5 Hz, 1H), 6.92 - 6.82 (m, 2H), 6.64 (dt, J=3.0, 8.8 Hz, 1H), 6.32 (dd, J=5.0, 9.0 Hz, 1H), 3.63 - 3.54 (m, 2H), 3.47 (s, 3H), 2.83 (t, J=6.3 Hz, 2H), 2.28 (s, 2H), 2.10 (s, 3H), 2.04 - 1.95 (m, 2H), 1.13 - 1.03 (m, 9H).

### Example 3

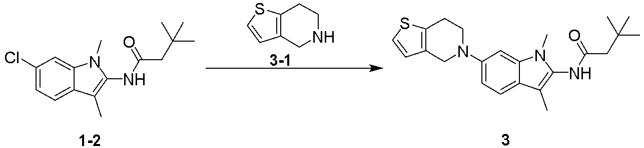

Compound **1-2** (100 mg, 341.53 µmol), compound **3-1** (95.09 mg, 683.05 µmol), 2-dicyclohexylphosphino-2',6'-diisopropoxy-1,1'-biphenyl (31.87 mg, 68.31 µmol) and cesium carbonate (333.83 mg, 1.02 mmol) were dissolved in dioxane (6 mL). Then methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol) was added. After purging with nitrogen three times, the mixture was stirred at 110 °C for 12 h. The mixture was cooled to 0 °C, quenched by pouring into water (5 mL), and extracted with ethyl acetate (5 mL × 3). The combined organic phases were concentrated under reduced pressure. The residue was purified by column chromatography (petroleum ether/ethyl acetate = 5:1) to give compound 3. LCMS (ESI): 396.1 [M+H]⁺; ¹HNMR (400 MHz, DMSO-d₆) δ 9.53 (s, 1H), 7.39 - 7.26 (m, 2H), 7.01 - 6.84 (m, 3H), 4.26 (s, 2H), 3.57 (t, J=5.8 Hz, 2H), 3.46 (s, 3H), 2.96 (br t, J=5.3 Hz, 2H), 2.26 (s, 2H), 2.06 (s, 3H), 1.09 (s, 9H).

### Example 4

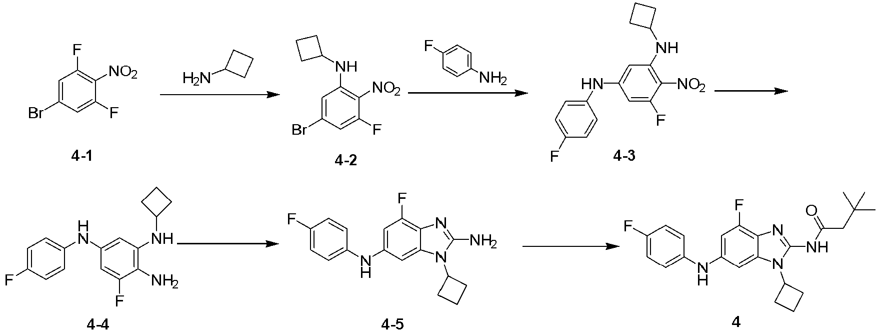

### Step 1

Compound 4-1 (1 g, 4.2 mmol) was added to THF (15 mL), then cyclobutylamine (268.96 mg, 3.78 mmol) and N,N-diisopropylethylamine (1.63 g, 12.61 mmol) were added, and the mixture was stirred at 25 °C for 5 h. Water (20 mL) was added, and the mixture was extracted with ethyl acetate (20 mL × 2). The combined organic layers were washed with saturated brine (20 mL), dried over sodium sulfate, filtered, and the filtrate was concentrated to give compound 4-2.

### Step 2

Compound 4-2 (1 g, 3.46 mmol) was added to dioxane (15 mL), followed by tris(dibenzylideneacetone)dipalladium(0) (316.75 mg, 345.90 µmol), 4,5-bis(diphenylphosphino)-9,9-dimethylxanthene (200.14 mg, 345.90 µmol), Cesium carbonate (2.82 g, 8.65 mmol) and 4-fluoroaniline (461.22 mg, 4.15 mmol) in sequence. The mixture was heated to 90 °C under nitrogen atmosphere to react for 5 h. After cooling to room temperature, the mixture was extracted with ethyl acetate (50 mL × 2). The combined organic layers were washed with saturated brine (50 mL), dried over sodium sulfate, and filtered. The filtrate was concentrated to obtain a crude product. The crude product was purified by column chromatography (petroleum ether/ethyl acetate, 1/0 ~ 2/1, V/V) to give compound 4-3. LCMS (ESI) m/z: 320.1 [M+H]⁺.

### Step 3

Compound 4-3 (0.6 g, 1.88 mmol) was added to ethanol (40 mL) and water (10 mL), followed by reduced iron powder (524.69 mg, 9.40 mmol) and ammonium chloride (502.57 mg, 9.4 mmol). The mixture was stirred at 90 °C for 16 h. After cooling to room temperature, the mixture was filtered. Water (50 mL) was added to the filtrate and the mixture was extracted with ethyl acetate (50 mL × 2). The combined organic layers were washed with saturated brine (50 mL), dried over sodium sulfate, and filtered. The filtrate was concentrated to give compound 4-4. LCMS (ESI) m/z: 290.1 [M+H]⁺.

### Step 4

Compound 4-4 (0.45 g, 1.56 mmol) was added to ethanol (20 mL), then cyanogen bromide (329.49 mg, 3.11 mmol) was added, and the mixture was stirred at 25 °C for 16 h. The aqueous layer was diluted with sodium bicarbonate solution (50 mL) and extracted with ethyl acetate (50 mL × 2). The combined organic layers were washed with saturated brine (50 mL), dried over sodium sulfate, and filtered. The filtrate was concentrated to give compound 4-5. LCMS (ESI) m/z: 315.2 [M+H]⁺.

### Step 5

Compound 4-5 (0.4 g, 1.27 mmol) was added to acetone (10 mL), then N,N-diisopropylethylamine (493.39 mg, 3.82 mmol) and tert-butylacetyl chloride (171.29 mg, 1.27 mmol) were added, and the mixture was stirred at 0 °C for 1 h. The reaction mixture was concentrated under reduced pressure to give a crude product. 1N NaOH (20 mL) and acetone (5 mL) were added, and the mixture was stirred at 25 °C for 1 h, then extracted with ethyl acetate (50 mL × 2). The combined organic layers were washed with saturated brine (50 mL), dried over sodium sulfate, and filtered. The filtrate was concentrated to obtain a crude product. The crude product was purified by column chromatography (petroleum ether/ethyl acetate, 1/0 ~ 2/1, V/V) to give compound 4. LCMS (ESI) m/z: 413.2 [M+H]⁺. ¹H NMR (CDCl3, 400MHz): δ 7.08-7.04 (m, 1H), 7.04 (d, J=4.0 Hz, 2H), 7.02 (s, 1H), 6.96 (d, J=1.5 Hz, 1H), 6.66 (dd, J=11.9, 1.4 Hz, 1H), 5.68 (br s, 1H), 4.94 (quin, J=8.7 Hz, 1H), 2.80-2.68 (m, 2H), 2.51 (br d, J=3.8 Hz, 2H), 2.48 (s, 2H), 1.98-1.84 (m, 2H), 1.10 (s, 9H).

### Example 5

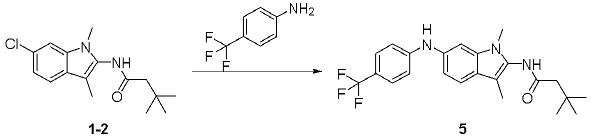

Compound 1-2 (200 mg, 683.05 µmol) was added to dioxane (4 mL), followed by methanesulfonato(2-dicyclohexylphosphino-2',6'-diisopropoxy-1,1'-biphenyl)(2-amino-1,1'-biphenyl-2-yl)palladium(II) (57.13 mg, 68.31 µmol), 2-dicyclohexylphosphino-2',6'-diisopropoxy-1,1'-biphenyl (63.75 mg, 136.61 µmol), cesium carbonate (667.66 mg, 2.05 mmol) and 4-(trifluoromethyl)aniline (220.11 mg, 1.37 mmol). The mixture was heated to 110 °C under nitrogen for 12 h. After cooling to room temperature, the mixture was filtered, the filter cake was rinsed with ethyl acetate (10 mL). The filtrates were collected and concentrated under reduced pressure to give a crude product. The crude product was purified by column chromatography (petroleum ether/ethyl acetate, 100/0 ~ 20/1, V/V) to give compound 5. LCMS (ESI) m/z: 418.0 [M+H]⁺. ¹H NMR (400 MHz, DMSO-d₆) δ ppm 9.60 (s, 1H), 8.54 (s, 1H), 7.46-7.41 (m, 3H), 7.16 (d, J = 1.6 Hz, 1H), 7.05 (d, J = 8.6 Hz, 2H), 6.88 (dd, J = 8.0, 1.6 Hz, 1H), 3.46 (s, 3H), 2.27 (s, 2H), 2.08 (s, 3H), 1.09 ppm (s, 9H).

### Example 6

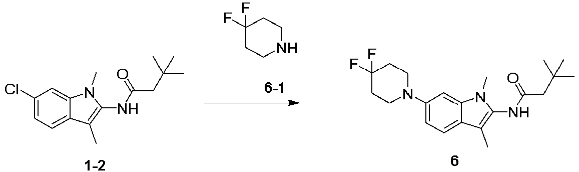

1-2 (100 mg, 341.53 µmol), 6-1 (82.74 mg, 683.05 µmol), 2-dicyclohexylphosphino-2',6'-diisopropoxy-1,1'-biphenyl (31.87 mg, 68.31 µmol), and cesium carbonate (333.83 mg, 1.02 mmol) were dissolved in dioxane (6 mL). Then methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol) was added. Under nitrogen, the mixture was stirred at 110 °C to react for 12 h. After the reaction was complete, the mixture was cooled to room temperature. The mixture was quenched with water (10 mL) and extracted with ethyl acetate (10 mL × 3). The combined organic phases were concentrated under reduced pressure. The residue was purified by column chromatography (petroleum ether/ethyl acetate = 5:1) to give compound 6. LCMS (ESI) m/z: 378.2 [M+H]⁺. ¹HNMR (400 MHz, DMSO-d₆) δ 9.52 (s, 1H), 7.30 (d, J=8.5 Hz, 1H), 6.90 (d, J=1.5 Hz, 1H), 6.81 (dd, J=2.0, 8.5 Hz, 1H), 3.44 (s, 3H), 3.30 - 3.26 (m, 4H), 2.25 (s, 2H), 2.17 - 2.06 (m, 4H), 2.04 (s, 3H), 1.07 (s, 9H).

### Example 7

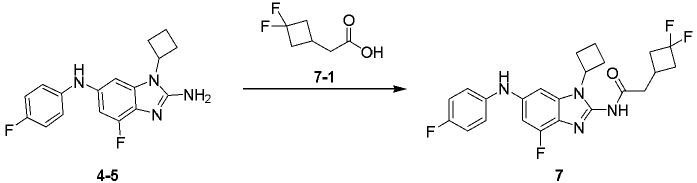

Compound 4-5 (0.3 g, 0.954 mmol) was added to ethyl acetate (10 mL), then 7-1 (0.172 g, 1.15 mmol), pyridine (754.93 mg, 9.54 mmol) and n-butylphosphonic anhydride (50% in ethyl acetate) (1.38 g, 3.82 mmol) were added. The mixture was stirred at 50 °C for 3 h. Water (50 mL) was added to the reaction mixture, and the mixture was extracted with ethyl acetate (50 mL × 2). The combined organic layers were washed with saturated brine (50 mL), dried over sodium sulfate, and filtered. The filtrate was concentrated to obtain a crude product. The crude product was purified by column chromatography (petroleum ether/ethyl acetate, 1/0 ~ 2/1, V/V) to give compound 7. LCMS (ESI) m/z: 447.1 [M+H]⁺. ¹H NMR (CDCl₃, 400MHz): δ ppm 7.10-7.01 (m, 4H), 6.90 (d, J=1.8 Hz, 1H), 6.65 (dd, J=11.8, 1.6 Hz, 1H), 5.68 (br s, 1H), 5.30-5.14 (m, 1H), 2.91 - 2.72 (m, 6H), 2.66 (br s, 1H), 2.48 (br d, J=7.8 Hz, 2H), 2.41-2.30 (m, 2H), 2.00-1.89 (m, 2H).

### Example 8

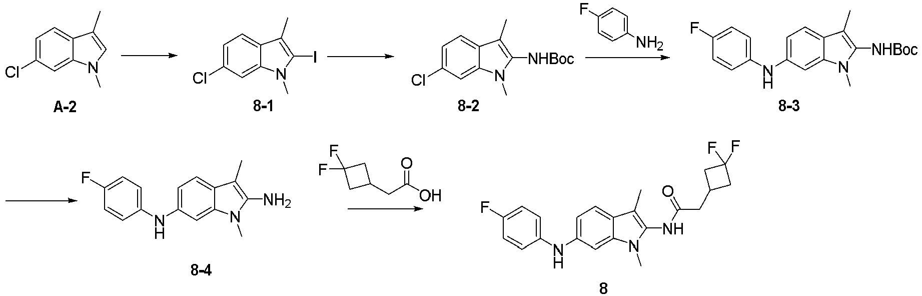

### Step 1

Compound A-2 (1.5 g, 7.51 mmol), silver triflate (2.32 g, 9.02 mmol) and sodium bicarbonate (757.58 mg, 9.02 mmol) were added to a reaction flask, followed by tetrahydrofuran (15 mL). The reaction system was cooled to 0 °C, and iodine (1.91 g, 7.51 mmol) was added to the reaction system while maintaining the internal temperature at 0 °C. The flask was evacuated and purged with nitrogen three times, then the mixture was reacted for 2 h. The reaction system was cooled to room temperature, filtered, and the filter cake was washed with ethyl acetate. Sodium sulfite solution (30 mL) was added to the filtrate, and the layers were separated. The organic phase was collected, dried over anhydrous sodium sulfate and concentrated under reduced pressure. The residue was purified by column chromatography (PE/EA = 1/0) to give compound 8-1.

### Step 2

Compound 8-1 (1.5 g, 4.91 mmol), tert-butyl carbamate (1.15 g, 9.82 mmol), tris(dibenzylideneacetone)dipalladium(0) (449.56 mg, 490.93 µmol), 4,5-bis(diphenylphosphino)-9,9-dimethylxanthene (568.13 mg, 981.86 µmol) and Cs₂CO₃ (4.80 g, 14.73 mmol) were added to a reaction flask, and dioxane (15 mL) was added. The flask was evacuated and purged with nitrogen three times. The reaction system was heated to 90 °C and stirred for 16 h. The reaction system was cooled to room temperature, and filtered. The filtrate was concentrated under reduced pressure, and the residue was purified by column chromatography (PE/EA = 1/0 ~ 10/1) to give compound 8-2. LCMS (ESI) m/z: 295.1 [M+H]⁺.

### Step 3

Compound 8-2 (0.6 g, 2.04 mmol) was dissolved in dioxane (10 mL). Then 4-fluoroaniline (452.34 mg, 4.07 mmol), methanesulfonato(2-dicyclohexylphosphino-2',6'-diisopropoxy-1,1'-biphenyl)(2-amino-1,1'-biphenyl-2-yl)palladium(II) (170.24 mg, 203.54 µmol), 2-dicyclohexylphosphino-2',6'-diisopropoxy-1,1'-biphenyl (189.96 mg, 407.09 µmol) and cesium carbonate (1.33 g, 4.07 mmol) were added. The flask was evacuated and purged with nitrogen three times. Under protection, the mixture was stirred at 110 °C for 4 h. The reaction system was cooled to room temperature, and filtered. The filtrate was concentrated under reduced pressure, and the residue was purified by column chromatography (PE/EA = 20/1 ~ 10/1) to give compound 8-3.LCMS (ESI) m/z: 370.1 [M+H]⁺.

### Step 4

Compound 8-3 (0.6 g, 1.62 mmol) was dissolved in ethyl acetate (5 mL). HCl in ethyl acetate (2 M, 4.06 mL) was added to the reaction system, and the reaction system was heated to 50 °C for 2 h. The mixture was concentrated under reduced pressure to give crude compound 8-4 as a hydrochloride salt. LCMS (ESI) m/z: 270.1 [M+H]⁺.

### Step 5

Compound 8-4 (0.5 g, as Crude hydrochloride) was added to tetrahydrofuran (10 mL). Then 2-(3,3-difluorocyclobutyl)acetic acid (294.57 mg, 1.96 mmol), N,N,N',N'-tetramethyl-O-(7-azabenzotriazol-1-yl)uronium hexafluorophosphate (746.09 mg, 1.96 mmol), and N,N-diisopropylethylamine (1.06 g, 8.18 mmol) were added. Under nitrogen, the reaction system was heated to 50 °C and stirred for 16 h. The reaction system was cooled to room temperature, and filtered, and the filtrate was concentrated. The residue was purified by column chromatography (PE/EA = 10/1 ~ 3/1) to give compound 8. LCMS (ESI) m/z: 402.1[M+H]⁺. 1H NMR (400 MHz, DMSO-d₆) δ ppm 9.67 (s, 1 H), 7.92 (s, 1 H), 7.34 (d, J=8.40 Hz, 1 H), 7.04-6.99 (m, 5 H), 6.82 - 6.76 (m, 1 H), 3.39 (s, 3 H), 2.83- 2.66 (m, 2 H), 2.65 - 2.60 (m, 2 H), 2.85- 2.58 (m, 1 H), 2.46 - 2.34 (m, 2 H), 2.07 - 2.02 (m, 3 H).

### Example 9

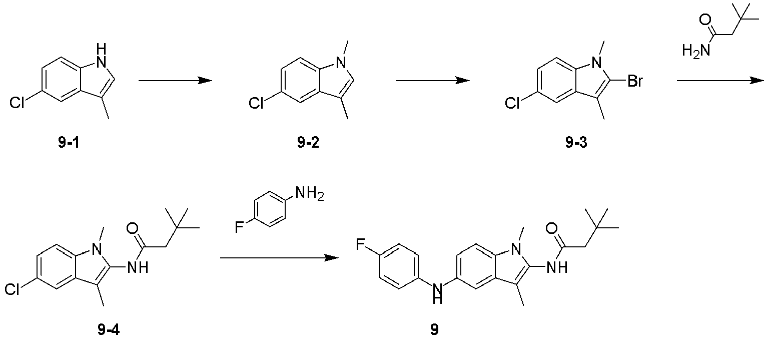

### Step 1

Compound 9-1 (2 g, 12.08 mmol) was dissolved in tetrahydrofuran (30 mL), and the mixture was cooled to 0 °C. Sodium hydride (724.48 mg, 18.11 mmol, 60% purity) was added in portions, and the mixture was stirred at 0 °C for 30 min. Iodomethane (3.43 g, 24.15 mmol) was then added, and the reaction mixture was stirred at 20 °C for an additional 2 h. The reaction was quenched with water (50 mL) at 0 °C, and extracted with ethyl acetate (50 mL × 3). The combined organic phases were washed with saturated brine (50 mL × 1), dried over anhydrous sodium sulfate, and filtered. The filtrate was concentrated under reduced pressure to give compound 9-2. LCMS (ESI) m/z: 180 [M+H]⁺. ¹HNMR (400 MHz, CDCl₃) δ 7.56 - 7.50 (m, 1H), 7.22 - 7.13 (m, 2H), 6.84 (s, 1H), 3.72 (s, 3H), 2.29 (s, 3H).

### Step 2

Compound 9-2 (500 mg, 2.78 mmol), copper(II) bromide (1.24 g, 5.57 mmol) and tetrabutylammonium bromide (89.72 mg, 278.33 µmol) were dissolved in a mixture of water (0.4 mL) and 1,2-dichloroethane (10 mL). The mixture was stirred at 20 °C for 2 h. After the reaction was complete, the mixture was filtered, the filter cake was washed with dichloromethane (30 mL), and the filtrate was concentrated under reduced pressure. The residue was purified by silica gel column chromatography (petroleum ether) to give compound 9-3. LCMS (ESI) m/z: 258, 260 [M+H]⁺.

### Step 3

Compound 9-3 (170 mg, 657.53 µmol), 3,3-dimethylbutanamide (151.46 mg, 1.32 mmol), cesium carbonate (642.71 mg, 1.97 mmol), 4,5-bis(diphenylphosphino)-9,9-dimethylxanthene (76.09 mg, 131.51 µmol) and tris(dibenzylideneacetone)dipalladium(0) (60.21 mg, 65.75 µmol) were added to dioxane (5 mL). Under nitrogen, the mixture was heated to 110 °C and stirred for 12 h. After the reaction was complete, the reaction system was cooled to room temperature, and filtered. The filter cake was washed with ethyl acetate (30 mL), and the filtrate was concentrated under reduced pressure. The residue was purified by silica gel column chromatography to give compound 9-4. LCMS (ESI) m/z: 293 [M+H]⁺.

### Step 4

Compound 9-4 (67 mg, 228.82 µmol), 4-fluoroaniline (50.85 mg, 457.65 µmol), 2-dicyclohexylphosphino-2',6'-diisopropoxy-1,1'-biphenyl (21.36 mg, 45.76 µmol), potassium tert-butoxide (51.35 mg, 457.65 µmol) were dissolved in dioxane (2 mL). Finally, methanesulfonato(2-dicyclohexylphosphino-2',6'-diisopropoxy-1,1'-biphenyl)(2-amino-1,1'-biphenyl-2-yl)palladium(II) (19.14 mg, 22.88 µmol) was added. After purging with nitrogen three times, the mixture was stirred at 110 °C for 12 h. After the reaction was complete, the reaction system was cooled to room temperature. Water (30 mL) was added, and the mixture was extracted with ethyl acetate (30 mL × 2). The combined organic phases were washed with saturated brine (50 mL × 1), dried over anhydrous sodium sulfate, and filtered. The filtrate was concentrated under reduced pressure to give a crude product. The crude product was purified by preparative HPLC (column: Welch Xtimate C18 150*25 mm*5 µm; mobile phase: [water (ammonia + ammonium bicarbonate) - acetonitrile]; acetonitrile ratio: 47%-77%) to give compound 9. LCMS (ESI) m/z: 386.3 [M+H]⁺. ¹HNMR (400 MHz, CDCl₃) δ 7.24 - 6.99 (m, 7H), 3.56 (s, 3H), 2.16 (s, 3H), 2.01 (s, 2H), 1.18 (s, 9H).

### Example 10

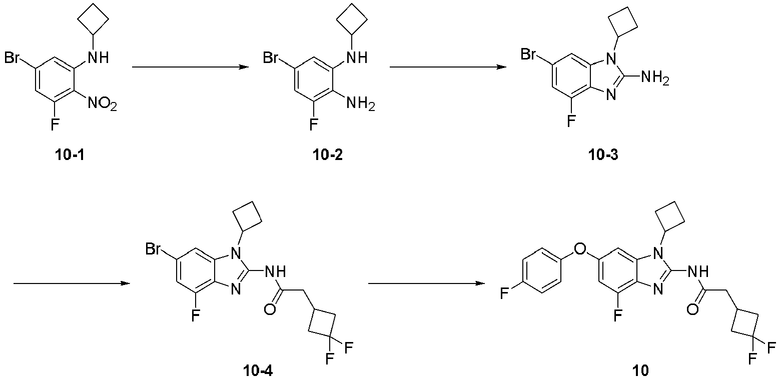

### Step 1

Compound 10-1 (2 g, 6.92 mmol) was added to ethanol (50 mL) and water (10 mL). Iron powder (1.93 g, 34.59 mmol) and ammonium chloride (1.85 g, 34.59 mmol) were added. The mixture was heated to 90 °C under nitrogen and reacted for 12 h. The reaction mixture was filtered. The filter cake was washed with ethyl acetate (50 mL), and the layers were separated. The organic phases were collected, washed with saturated brine (50 mL), dried over anhydrous sodium sulfate, and concentrated under reduced pressure to give a crude product. The crude product was purified by column chromatography (petroleum ether/ethyl acetate, 100/0 ~ 20/1, V/V) to give compound 10-2. LCMS (ESI) m/z: 259.0 [M+H]⁺.

### Step 2

Compound 10-2 (1.27 g, 4.90 mmol) was added to ethanol (15 mL). Cyanogen bromide (1.04 g, 9.80 mmol) was added, and the reaction mixture was reacted at 25 °C under nitrogen for 12 h. The reaction mixture was concentrated under reduced pressure. Ethyl acetate (15 mL) was added to the residue, and a solid precipitated. The solid was filtered and the filter cake was dried to give compound 10-3. LCMS (ESI) m/z: 284.0 [M+H]⁺.

### Step 3

Compound 10-3 (900 mg, 3.17 mmol) was added to N,N-dimethylformamide (15 mL). O-(7-Azabenzotriazol-1-yl)-N,N,N',N'-tetramethyluronium hexafluorophosphate (1.81 g, 4.75 mmol), N,N-diisopropylethylamine (1.23 g, 9.50 mmol) and 2-(3,3-difluorocyclobutyl)acetic acid (570.63 mg, 3.80 mmol) were added. The reaction mixture was heated to 65 °C under nitrogen and reacted for 12 h. The reaction mixture was cooled to room temperature, extracted with ethyl acetate (30 mL), washed with saturated ammonium chloride (15 mL), saturated sodium bicarbonate (15 mL), 10% sodium carbonate solution (15 mL), and saturated brine (15 mL), dried over anhydrous sodium sulfate, and concentrated under reduced pressure. Ethyl acetate (10 mL) was added to the residue, and the mixture was stirred for 30 min. The mixture was filtered, and the filter cake was dried to give compound 10-4. LCMS (ESI) m/z: 416.0 [M+H]⁺.

### Step 4

Compound 10-4 (150 mg, 360.37 µmol) was added to N,N-dimethylacetamide (2 mL). Copper(I) iodide (3.43 mg, 18.02 µmol), iron(III) acetylacetonate (12.73 mg, 36.04 µmol), cesium carbonate (352.25 mg, 1.08 mmol) and 4-fluorocresol (60.60 mg, 540.56 µmol) were added. The reaction mixture was heated to 150 °C under nitrogen and reacted for 12 h. The reaction mixture was cooled to room temperature, and dissolved in ethyl acetate (10 mL). The mixture was washed with saturated sodium chloride (10 mL × 3). The organic phase was collected, dried over anhydrous sodium sulfate, and concentrated under reduced pressure to give a crude product. The crude product was purified by prep-HPLC (column: C18 100×40 mm; mobile phase: [water (TFA) - acetonitrile]; acetonitrile ratio: 36%-66%) to give compound 10. LCMS (ESI) m/z: 448.0 [M+H]⁺. ¹H NMR (400 MHz, DMSO-d₆) δ ppm 10.83-10.48 (m, 1H), 7.26-7.16 (m, 3H), 7.13-7.04 (m, 2H), 6.84-6.73 (m, 1H), 4.81-4.62 (m, 1H), 2.87-2.54 (m, 7H), 2.41- 2.32 (m, 4H), 1.85- 1.72 (m, 2H).

### Example 11

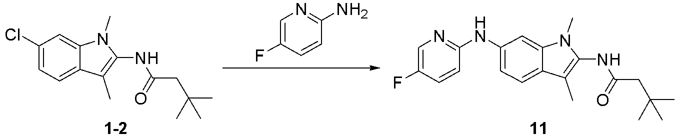

Compound 1-2 (200 mg, 683.05 µmol) was added to dioxane (3 mL). Methanesulfonato(2-dicyclohexylphosphino-2',6'-diisopropoxy-1,1'-biphenyl)(2-amino-1,1'-biphenyl-2-yl)palladium(II) (57.13 mg, 68.31 µmol), 2-dicyclohexylphosphino-2',6'-diisopropoxy-1,1'-biphenyl (63.75 mg, 136.61 µmol), cesium carbonate (667.66 mg, 2.05 mmol) and 2-amino-5-fluoropyridine (153.15 mg, 1.37 mmol) were added. The reaction mixture was heated to 110 °C under nitrogen and reacted for 12 h. The reaction mixture was cooled to room temperature, and filtered. The filter cake was washed with ethyl acetate (10 mL), and the filtrate was collected, concentrated under reduced pressure. The residue was purified by prep-HPLC (column: Welch Xtimate C18 150*25 mm*5 µm; mobile phase: [water (ammonia + ammonium bicarbonate) - acetonitrile]; acetonitrile ratio: 44%-64%) to give a crude product. Ethyl acetate (0.5 mL) was added to the crude product, and the mixture was stirred for 2 h. The mixture was filtered and the filter cake was dried to give compound 11. LCMS (ESI) m/z: 369.0 [M+H]⁺. ¹H NMR (400 MHz, DMSO-d₆) δ ppm 9.56 (s, 1H), 8.92 (s, 1H), 8.10 (d, J = 2.8 Hz, 1H), 7.77 (d, J = 1.2 Hz, 1H), 7.49 (td, J = 8.8, 2.8 Hz, 1H), 7.33 (d, J = 8.0 Hz, 1H), 7.07 (dd, J = 8.4, 1.6 Hz, 1H), 6.84 (dd, J = 9.2, 3.6 Hz, 1H), 3.44 (s, 3H), 2.26 (s, 2H), 2.06 (s, 3H), 1.08 (s, 9H).

### Example 12

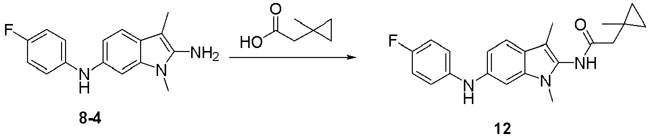

Compound 8-4 (0.2 g, crude hydrochloride salt) was added to tetrahydrofuran (5 mL). 2-(1-Methylcyclopropyl)acetic acid (89.59 mg, 784.89 µmol), N,N,N',N'-tetramethyl-O-(7-azabenzotriazol-1-yl)uronium hexafluorophosphate (298.44 mg, 784.89 µmol) and N,N-diisopropylethylamine (422.66 mg, 3.27 mmol) were added. Under nitrogen, the reaction system was heated to 50 °C and stirred for 16 h. The reaction system was cooled to room temperature, and filtered. The filtrate was concentrated and the residue was purified by column chromatography (petroleum ether/ethyl acetate, 10/1 ~ 3/1) to give compound 12. LCMS (ESI) m/z: 366.1 [M+H]⁺. ¹H NMR (400 MHz, DMSO-d₆) δ ppm 9.47 (s, 1 H), 7.90 (s, 1 H), 7.34 (d, J=8.4 Hz, 1 H), 7.04 - 6.98 (m, 5 H), 6.79 (dd, J=8.4, 1.6 Hz, 1 H), 3.41 (s, 3 H), 2.27 (s, 2 H), 2.06 (s, 3 H), 1.17 (s, 3 H), 0.59 - 0.55 (m, 2 H), 0.38 - 0.31 (m, 2 H).

### Example 13

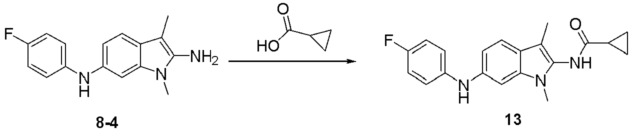

Compound 8-4 (0.1 g, crude hydrochloride salt) was added to tetrahydrofuran (5 mL). Cyclopropanecarboxylic acid (33.79 mg, 392.44 µmol), N,N,N',N'-tetramethyl-O-(7-azabenzotriazol-1-yl)uronium hexafluorophosphate (149.22 mg, 392.44 µmol) and N,N-diisopropylethylamine (211.33 mg, 1.64 mmol) were added to the reaction system. Under nitrogen, the reaction system was heated to 50 °C and stirred for 16 h. The reaction mixture was concentrated and purified by prep-HPLC (column: 2_Phenomenex Gemini C18 75*40 mm*3 µm; mobile phase: [water (ammonia + ammonium bicarbonate) - acetonitrile]; acetonitrile ratio: 31%-61%) to give compound 13. LCMS (ESI) m/z: 338.1 [M+H]⁺. ¹H NMR (400 MHz, DMSO-d₆) δ ppm 9.86 (s, 1 H) 7.90 (s, 1 H) 7.33 (d, J=8.0 Hz, 1 H) 7.02 (d, J=6.8 Hz, 5 H) 6.78-6.76 (m, 1 H) 3.38 (s, 3 H) 2.05 (s, 3 H) 0.85 - 0.80 (m, 5 H).

### Example 14

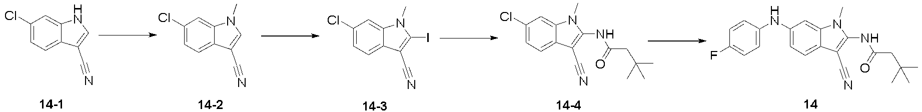

### Step 1

Sodium hydride (339.71 mg, 8.49 mmol, 60% purity) was added to tetrahydrofuran (30 mL). The mixture was cooled to 0 °C in an ice-water bath. Compound 14-1 (1 g, 5.66 mmol) was added in portions and the reaction mixture was stirred to react for 30 min. Iodomethane (11.32 mmol, 705.02 µL) was then added, and the mixture was allowed to warm to 25 °C under nitrogen and reacted for 2 h. Saturated ammonium chloride solution (50 mL) was added dropwise to quench the reaction. The mixture was extracted with ethyl acetate (50 mL), and the layers were separated. The organic phase was washed with saturated brine (50 mL), dried over anhydrous sodium sulfate, and concentrated under reduced pressure to give compound 14-2. LCMS (ESI) m/z: 191.0 [M+H]⁺.

### Step 2

Compound 14-2 (200 mg, 1.05 mmol) was added to tetrahydrofuran (5 mL). The mixture was cooled to 0 °C in an ice-water bath, and lithium bis(trimethylsilyl)amide (1 M, 1.26 mL) was added. After stirring for 30 min, a solution of iodine (292.91 mg, 1.15 mmol) in tetrahydrofuran (2 mL) was added. The reaction mixture was allowed to warm to 25 °C under nitrogen and reacted for 12 h. Saturated sodium sulfite solution (20 mL) was added to quench the reaction, and the mixture was extracted with ethyl acetate (20 mL). The layers were separated. The organic phase was washed with saturated brine (20 mL), dried over anhydrous sodium sulfate, and concentrated under reduced pressure to give compound 14-3. LCMS (ESI) m/z: 317.0[M+H]⁺. ¹H NMR (400 MHz, DMSO-d₆) δ ppm 7.89 (d, J = 1.2 Hz, 1H), 7.59 (d, J = 7.6 Hz, 1H), 7.25 (dd, J = 8.4, 1.2 Hz, 1H), 3.84 (s, 3H).

### Step 3

Compound 14-3 (250 mg, 789.83 µmol) was added to dioxane (10 mL). Tris(dibenzylideneacetone)dipalladium(0) (72.33 mg, 78.98 µmol), 4,5-bis(diphenylphosphino)-9,9-dimethylxanthene (91.40 mg, 157.97 µmol), cesium carbonate (772.02 mg, 2.37 mmol) and 3,3-dimethylbutanamide (181.93 mg, 1.58 mmol) were added. The reaction mixture was heated to 110 °C under nitrogen and reacted for 12 h. The reaction mixture was cooled to room temperature, and filtered. The filter cake was washed with methanol (50 mL), and the filtrate was collected, concentrated under reduced pressure to give a crude product. The crude product was purified by column chromatography (dichloromethane/methanol, 100/0 ~ 50/1, V/V) to give compound 14-4. LCMS (ESI) m/z: 304.0[M+H]⁺.

### Step 4

Compound 14-4 (200 mg, 685.36 µmol) was added to dioxane (4 mL). Methanesulfonato(2-dicyclohexylphosphino-2',6'-diisopropoxy-1,1'-biphenyl)(2-amino-1,1'-biphenyl-2-yl)palladium(II) (55.06 mg, 65.84 µmol), 2-dicyclohexylphosphino-2',6'-diisopropoxy-1,1'-biphenyl (61.44 mg, 131.67 µmol), potassium tert-butoxide (147.75 mg, 1.32 mmol) and 4-fluoroaniline (146.31 mg, 1.32 mmol) were added. The reaction mixture was heated to 110 °C under nitrogen and reacted for 12 h. The reaction system was cooled to room temperature, and filtered. The filter cake was washed with methanol (20 mL), and the filtrate was collected, concentrated under reduced pressure to give a crude product. The crude product was purified by column chromatography (petroleum ether/ethyl acetate, 100/0 ~ 3/1, V/V) to give compound 14. LCMS (ESI) m/z: 379.0[M+H]⁺. ¹H NMR (400 MHz, DMSO-d₆) δ ppm 10.37 (s, 1H), 8.19 (s, 1H), 7.43 (d, J = 8.4 Hz, 1H), 7.17-7.03 (m, 5H), 6.97 (dd, J = 8.8, 2.0 Hz, 1H), 3.52 (s, 3H), 2.30 (s, 2H), 1.08 (s, 9H).

### Example 15

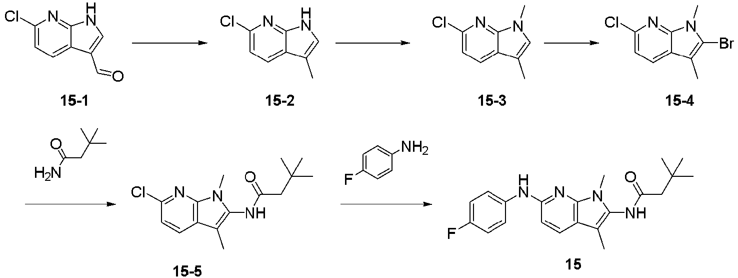

### Step 1

Compound 15-1 (2.10 g, 11.63 mmol) was dissolved in trifluoroacetic acid (20 mL). Triethylsilane (7.03 g, 60.47 mmol) was added, and the mixture was reacted at 20 °C for 12 h. Water (10 mL) was added to quench the reaction, and the mixture was concentrated under reduced pressure to remove most of the TFA. The concentrate was adjusted to pH 8 with saturated sodium carbonate solution, extracted with ethyl acetate (150 mL × 3), and the combined organic phases were washed with saturated brine (150 mL), dried over anhydrous sodium sulfate, and filtered. The filtrate was concentrated under reduced pressure. The residue was purified by silica gel column chromatography to give compound 15-2. LCMS (ESI) m/z: 166.9[M+H]⁺.

### Step 2

Compound 15-2 (1.96 g, 11.76 mmol) was dissolved in tetrahydrofuran (20 mL), cooled to 0 °C. Sodium hydride (705.85 mg, 17.65 mmol, 60% purity) was added in portions. After the addition was complete, the mixture was reacted at 0 °C for 30 min. Iodomethane (4.17 g, 29.41 mmol) was added, and the mixture was stirred at 20 °C for 12 h. After the reaction was complete, the reaction was quenched with water (50 mL) at 0 °C, and extracted with ethyl acetate (150 mL × 3). The combined organic phases were washed with saturated brine (200 mL), dried over anhydrous sodium sulfate, and filtered. The filtrate was concentrated under reduced pressure. The residue was purified by column chromatography to give compound 15-3. LCMS (ESI) m/z: 181.1[M+H]⁺.

### Step 3

Compound 15-3 (1.53 g, 8.47 mmol) was dissolved in acetonitrile (20 mL). N-Bromosuccinimide (1.58 g, 8.89 mmol) was added, and the mixture was stirred at 20 °C for 12 h. After the reaction was complete, saturated sodium thiosulfate solution (5 mL) was added, and the mixture was extracted with ethyl acetate (30 mL × 2). The combined organic phases were washed with saturated brine (30 mL), dried over anhydrous sodium sulfate, and filtered. The filtrate was concentrated under reduced pressure. The residue was purified by silica gel column chromatography to give compound 15-4. LCMS (ESI) m/z: 259.0, 260.8[M+H]⁺.

### Step 4

Compound 15-4 (1.36 g, 5.24 mmol), 3,3-dimethylbutanamide (1.21 g, 10.48 mmol), cesium carbonate (5.12 g, 15.72 mmol), 4,5-bis(diphenylphosphino)-9,9-dimethylxanthene (606.42 mg, 1.05 mmol) and tris(dibenzylideneacetone)dipalladium(0) (479.86 mg, 524.02 µmol) were added to dioxane (20 mL). After purging with nitrogen three times, the mixture was heated to 110 °C and stirred for 12 h for reaction. After the reaction was complete, the reaction system was cooled to room temperature, and water (100 mL) was added. The mixture was extracted with ethyl acetate (100 mL × 2). The combined organic phases were washed with saturated brine (100 mL), dried over anhydrous sodium sulfate, and filtered. The filtrate was concentrated under reduced pressure. The residue was purified by silica gel column chromatography to give compound 15-5. LCMS (ESI) m/z: 294.1[M+H]⁺.

### Step 5

Compound 15-5 (120 mg, 408.45 µmol), 4-fluoroaniline (90.77 mg, 816.91 µmol), cesium carbonate (266.16 mg, 816.91 µmol), 4,5-bis(diphenylphosphino)-9,9-dimethylxanthene (47.27 mg, 81.69 µmol) and tris(dibenzylideneacetone)dipalladium(0) (37.40 mg, 40.85 µmol) were added to dioxane (10 mL). After purging with nitrogen three times, the mixture was stirred at 110 °C for 12 h. After the reaction was complete, the reaction system was cooled to room temperature, and water (50 mL) was added. The mixture was extracted with ethyl acetate (50 mL × 2). The combined organic phases were washed with saturated brine (50 mL), dried over anhydrous sodium sulfate, and filtered. The filtrate was concentrated under reduced pressure to give a crude product. The crude product was purified by preparative HPLC (column: Welch Xtimate C18 150*25 mm*5 µm; mobile phase: [water (ammonia + ammonium bicarbonate) - acetonitrile]; acetonitrile ratio: 45%-75%) to give compound 15. LCMS (ESI) m/z:369.2 [M+H]⁺. ¹HNMR (400 MHz, CDCl₃) δ ppm 7.78 - 7.68 (m, 2H), 7.68 - 7.59 (m, 1H), 7.07 - 6.91 (m, 2H), 6.62 - 6.41 (m, 1H), 3.59 (s, 3H), 2.35 (s, 2H), 2.13 (s, 3H), 1.16 (s, 9H).

### Example 16

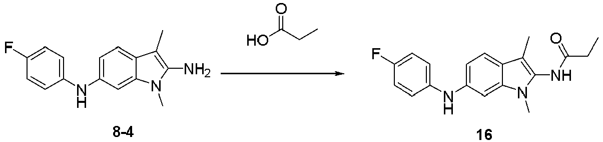

Crude compound 8-4 (0.2 g, as hydrochloride) was added to tetrahydrofuran (5 mL). Propanoic acid (784.89 µmol, 58.55 µL), N,N,N',N'-tetramethyl-O-(7-azabenzotriazol-1-yl)uronium hexafluorophosphate (298.44 mg, 784.89 µmol) and N,N-diisopropylethylamine (422.66 mg, 3.27 mmol) were added. The flask was evacuated and purged with nitrogen three times. The reaction system was heated to 50 °C and stirred for 16 h. The reaction mixture was concentrated and purified by column chromatography (petroleum ether/ethyl acetate, 5/1 ~ 1/1) to give compound 16. LCMS (ESI) m/z:326.1 [M+H]⁺. ¹H NMR (400 MHz, DMSO-d₆) δ ppm 9.56 (s, 1 H), 7.92 (s, 1 H), 7.34 (d, J=8.4 Hz, 1 H) , 7.04 - 6.99 (m, 5 H), 6.79 (dd, J=8.0, 1.6 Hz, 1 H), 3.40 (s, 3 H), 2.40 (q, J=7.2 Hz, 2 H), 2.05 (s, 3 H), 1.15 (t, J=7.6 Hz, 3 H).

### Example 17

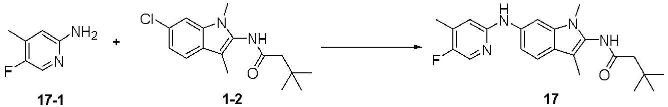

Compound 1-2 (100.00 mg, 341.53 µmol) was added to dioxane (3 mL). Methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and compound 17-1 (86.15 mg, 683.05 µmol) were added. The reaction mixture was heated to 110°C under nitrogen and reacted for 12 h. The reaction system was cooled to room temperature, and filtered. The filter cake was washed with ethyl acetate (10 mL), and the filtrate was collected, concentrated under reduced pressure. The residue was purified by column chromatography (petroleum ether/ethyl acetate, 100/0 ~ 5/1, V/V) to give a crude product, which was then stirred with ethyl acetate (1 mL) at room temperature for 1 h. The mixture was filtered and the filter cake was dried to give compound 17. LCMS (ESI) m/z:383.1 [M+H]⁺. ¹H NMR (400 MHz, DMSO-d₆) δ ppm 9.54 (s, 1H), 8.76 (s, 1H), 7.99 (s, 1H), 7.73 (s, 1H), 7.32 (d, J = 8.4 Hz, 1H), 7.06 (dd, J = 8.4, 1.6 Hz, 1H), 6.70 (d, J = 5.2 Hz, 1H), 3.44 (s, 3H), 2.26 (s, 2H), 2.20 (s, 3H), 2.06 (s, 3H), 1.08 (s, 9H).

### Example 18

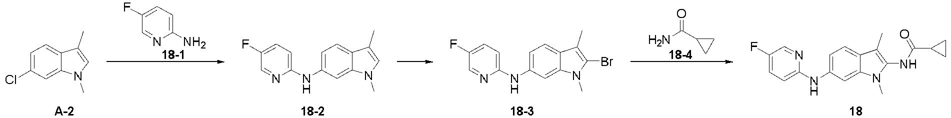

### Step 1

Compound A-2 (2 g, 11.13 mmol) was added to dioxane (20 mL). Compound 18-1 (2.50 g, 22.27 mmol), methanesulfonato(2-dicyclohexylphosphino-2',6'-diisopropoxy-1,1'-biphenyl)(2-amino-1,1'-biphenyl-2-yl)palladium(II) (931.13 mg, 1.11 mmol), 2-dicyclohexylphosphino-2',6'-diisopropoxy-1,1'-biphenyl (1.04 g, 2.23 mmol) and cesium carbonate (10.88 g, 33.40 mmol) were added. After purging with nitrogen three times, the reaction system was heated to 50 °C and stirred for 16 h. The mixture was filtered, and the filtrate was concentrated. The residue was purified by column chromatography (petroleum ether/ethyl acetate, 10/1 ~ 3/1) to give compound 18-2. LCMS (ESI) m/z:256.1 [M+H]⁺.

### Step 2

Compound 18-2 (1.5 g, 4.99 mmol) was added to tetrahydrofuran (30 mL). The reaction system was cooled to -30 °C, and NBS (888.89 mg, 4.99 mmol) was added. After stirring for 5 min, sodium sulfite solution (20 mL) was added to quench the reaction. Ethyl acetate (20 mL) was added and the mixture was stirred for 5 min. The mixture was allowed to stand and layers were separated. The organic phases were collected, washed with saturated brine (20 mL × 2), dried over anhydrous sodium sulfate, and filtered. The filtrate was concentrated. The residue was purified by column chromatography (petroleum ether/ethyl acetate, 10/1 ~ 3/1) to give compound 18-3. LCMS (ESI) m/z: 333.9, 335.9 [M+H]⁺.

### Step 3

Compound 18-3 (50 mg, 149.62 µmol) was added to dioxane (3 mL). Cyclopropanecarboxamide (25.47 mg, 299.23 µmol), tris(dibenzylideneacetone)dipalladium(0) (13.70 mg, 14.96 µmol), 4,5-bis(diphenylphosphino)-9,9-dimethylxanthene (17.31 mg, 29.92 µmol) and cesium carbonate (146.25 mg, 448.85 µmol) were added. After purging with nitrogen three times, the reaction system was heated to 110 °C and stirred for 4 h. The reaction system was cooled to room temperature, and filtered. The filtrate was concentrated and the residue was purified by column chromatography (petroleum ether/ethyl acetate, 5/1 ~ 1/1) to give compound 18. LCMS (ESI) m/z: 339.1 [M+H]⁺. ¹H NMR (400 MHz, DMSO-d₆) δ ppm 9.89 (s, 1 H), 8.93 (s, 1 H), 8.11 (d, J=2.8 Hz, 1 H), 7.78 (d, J=1.2 Hz, 1 H), 7.50 (td, J=8.8, 3.2 Hz, 1 H), 7.34 (d, J=8.28 Hz, 1 H), 7.07 (dd, J=8.4, 1.6 Hz, 1 H), 6.85 (dd, J=9.2, 4.0 Hz, 1 H), 3.43 (s, 3 H), 2.07 (s, 3 H), 1.92 - 1.82 (m, 1 H), 0.87 - 0.81 (m, 4 H).

### Example 19

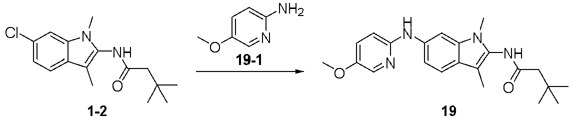

Compound 1-2 (0.1 g, 341.53 µmol) was added to dioxane (3 mL). Methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and compound 19-1 (146.31 mg, 1.32 mmol) were added. The reaction mixture was heated to 110 °C under nitrogen and reacted for 12 h. The reaction system was cooled to room temperature, and filtered. The filter cake was washed with ethyl acetate (10 mL), and the filtrate was collected, concentrated under reduced pressure to give a crude product. The crude product was purified by prep-HPLC (column: Welch Xtimate C18 150*25mm*5µm; mobile phase: [water (ammonia + ammonium bicarbonate) - acetonitrile]; acetonitrile ratio: 38%-68%) and then by column chromatography (dichloromethane/methanol, 100/0 ~ 20/1, V/V) to give compound 19. LCMS (ESI) m/z: 381.3[M+H]⁺. ¹H NMR (400 MHz, DMSO-d₆) δ ppm 9.53 (s, 1H), 8.64 (s, 1H), 7.89 (d, J = 2.8 Hz, 1H), 7.81 (d, J = 1.6 Hz, 1H), 7.33-7.20 (m, 2H), 7.04 (dd, J = 8.8, 2.0 Hz, 1H), 6.82 (d, J = 9.2 Hz, 1H), 3.75 (s, 3H), 3.43 (s, 3H), 2.26 (s, 2H), 2.06 (s, 3H), 1.08 (s, 9H).

### Example 20

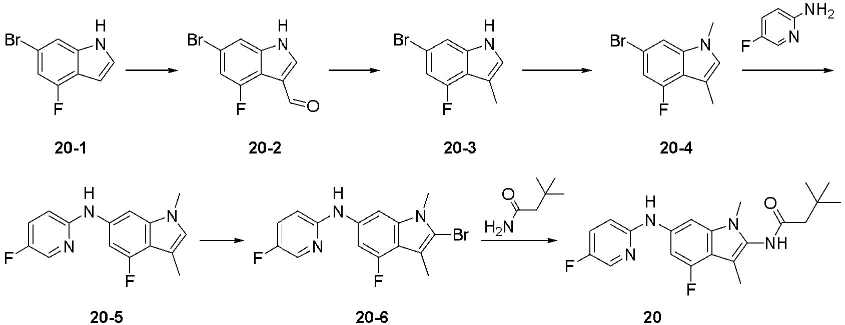

### Step 1

Compound 20-1 (2 g, 9.34 mmol) was dissolved in N,N-dimethylformamide (20 mL). Phosphorus oxychloride (5.01 g, 32.71 mmol) was added at 0 °C, and the mixture was reacted at 20 °C for 2 h. After the reaction was complete, sodium hydroxide solution (2 M, 93.44 mL) was slowly added, and the mixture was reacted at 20 °C for 12 h. After completion, the mixture was extracted with ethyl acetate (200 mL × 3), and the combined organic phases were washed with saturated brine (200 mL × 3), dried over anhydrous sodium sulfate, and filtered. The filtrate was concentrated under reduced pressure. The residue was purified by silica gel column chromatography to give compound 20-2. LCMS (ESI) m/z: 241.8, 243.8 [M+H]⁺. ¹HNMR (400 MHz, CD₃OD) δ 10.01 (d, J=2.8 Hz, 1H), 8.12 (s, 1H), 7.50 (d, J=1.2 Hz, 1H), 7.18 - 7.10 (m, 1H).

### Step 2

Compound 20-2 (770 mg, 3.18 mmol) was dissolved in tetrahydrofuran (15 mL). Lithium aluminum hydride in THF (2.5 M, 3.82 mL, 9.54 mmol) was added at 0 °C, and the mixture was heated to 70 °C and reacted for 1 h. After the reaction was complete, the reaction mixture was cooled to 0-5 °C. Water (20 mL) was added to quench the reaction. The mixture was extracted with ethyl acetate (100 mL × 2), and the combined organic phases were washed with saturated brine (100 mL), dried over anhydrous sodium sulfate, and filtered. The filtrate was concentrated under reduced pressure. The residue was purified by silica gel column chromatography (petroleum ether/ethyl acetate, 10/1 ~ 3/1, V/V) to give compound 20-3. LCMS (ESI) m/z: 228.0, 230.0 [M+H]⁺.

### Step 3

Compound 20-3 (770 mg, 3.38 mmol) was dissolved in tetrahydrofuran (15 mL), and the mixture was cooled to 0 °C. Sodium hydride (202.58 mg, 5.06 mmol, 60% purity) was added in portions. After the addition was complete, the mixture was reacted at 0 °C for 30 min. Iodomethane (1.20 g, 8.44 mmol) was added, and the reaction mixture was stirred at 20 °C for 1 h. After the reaction was complete, the reaction was quenched with water (20 mL) at 0 °C, extracted with ethyl acetate (100 mL × 2), and the combined organic phases were washed with saturated brine (100 mL), dried over anhydrous sodium sulfate, and filtered. The filtrate was concentrated under reduced pressure. The residue was purified by silica gel column chromatography (petroleum ether/ethyl acetate, 10/1 ~ 4/1, V/V) to give compound 20-4. LCMS (ESI) m/z: 242.0, 244.0 [M+H]⁺.

### Step 4

Compound 20-4 (636 mg, 3.38 mmol), 2-amino-5-fluoropyridine (471.23 mg, 4.20 mmol), cesium carbonate (2.57 g, 7.88 mmol), 4,5-bis(diphenylphosphino)-9,9-dimethylxanthene (304.02 mg, 525.43 µmol) and tris(dibenzylideneacetone)dipalladium(0) (240.57 mg, 262.72 µmol) were added to dioxane (20 mL). After purging with nitrogen three times, the reaction system was stirred at 90 °C for 3 h. After the reaction was complete, the reaction system was cooled to room temperature, and water (100 mL) was added. The mixture was extracted with ethyl acetate (100 mL × 2). The combined organic phases were washed with saturated brine (100 mL), dried over anhydrous sodium sulfate, and filtered. The filtrate was concentrated under reduced pressure. The residue was purified by silica gel column chromatography (petroleum ether/ethyl acetate, 10/1 ~ 3/1, V/V) to give compound 20-5. LCMS (ESI) m/z: 274.1 [M+H]⁺.

### Step 5

Compound 20-5 (200 mg, 731.85 µmol) was dissolved in tetrahydrofuran (10 mL). N-Bromosuccinimide (130.26 mg, 731.85 µmol) was added at -40 °C, and the mixture was stirred at -40 °C for 5 min. After the reaction was complete, saturated sodium thiosulfate solution (3 mL) was added. The mixture was extracted with ethyl acetate (30 mL × 2). The combined organic phases were washed with saturated brine (30 mL), dried over anhydrous sodium sulfate, and filtered. The filtrate was concentrated under reduced pressure. The residue was purified by silica gel column chromatography to give compound 20-6. LCMS (ESI) m/z: 351.8, 353.8 [M+H]⁺.

### Step 6

Compound 20-6 (110 mg, 312.34 µmol), 3,3-dimethylbutanamide (53.96 mg, 468.52 µmol), cesium carbonate (305.30 mg, 937.03 µmol), 4,5-bis(diphenylphosphino)-9,9-dimethylxanthene (36.15 mg, 62.47 µmol) and tris(dibenzylideneacetone)dipalladium(0) (28.60 mg, 31.23 µmol) were added to dioxane (10 mL). After purging with nitrogen three times, the reaction system was heated to 90 °C and reacted with stirring for 12 h. After the reaction was complete, the reaction system was cooled to room temperature, and water (30 mL) was added. The mixture was washed with ethyl acetate (40 mL × 2). The combined organic phases were washed with saturated brine (40 mL), dried over anhydrous sodium sulfate, and filtered. The filtrate was concentrated under reduced pressure. The residue was purified by silica gel column chromatography (petroleum ether/ethyl acetate, 10/1 ~ 1/1, V/V) to give compound 20. LCMS (ESI) m/z: 387.2[M+H]⁺. ¹HNMR (400 MHz, DMSO-d₆) δ 9.59 (s, 1H), 9.11 (s, 1H), 8.14 (d, J=3.2 Hz, 1H), 7.57 - 7.49 (m, 2H), 7.09 - 7.03 (m, 1H), 6.86 (dd, J=3.6, 9.2 Hz, 1H), 3.43 (s, 3H), 2.26 (s, 2H), 2.16 (s, 3H), 1.08 (s, 9H).

### Example 21

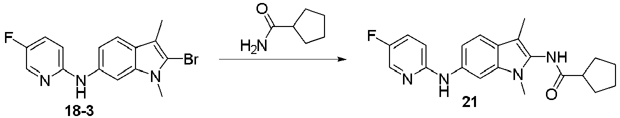

Compound 18-3 (0.1 g, 299.23 µmol) was added to dioxane (5 mL). Then 1-cyclopentanecarboxamide (67.72 mg, 598.47 µmol), tris(dibenzylideneacetone)dipalladium(0) (27.40 mg, 29.92 µmol), 4,5-bis(diphenylphosphino)-9,9-dimethylxanthene (34.63 mg, 59.85 µmol) and cesium carbonate (292.49 mg, 897.70 µmol) were added. After purging with nitrogen three times, the reaction system was stirred at 110 °C for 4 h. Water (30 mL) was added. The mixture was extracted with ethyl acetate (30 mL × 2), washed with saturated brine (30 mL), dried over anhydrous sodium sulfate, and filtered. The filtrate was concentrated to give a crude product. The crude product was purified by column chromatography (petroleum ether/ethyl acetate, 100/0 ~ 50/50, V/V) to give compound 21. LCMS (ESI) m/z: 367.1 [M+H]⁺. ¹H NMR (400 MHz, DMSO-d₆) δ ppm 9.55 (s, 1 H), 8.92 (s, 1 H), 8.10 (d, J=2.8 Hz, 1 H), 7.77 (s, 1 H), 7.49 (td, J=8.4, 2.8 Hz, 1 H), 7.33 (d, J=8.4 Hz, 1 H), 7.07 (dd, J=8.4, 1.6 Hz, 1 H), 6.85 (dd, J=9.2, 3.6 Hz, 1 H) , 3.42 (s, 3 H), 2.92-2.85 (m, 1 H), 2.05 (s, 3 H), 1.96-1.88 (m, 2 H), 1.80 - 1.76 (m, 2 H), 1.71 -1.64 (m, 2 H) 1.62 - 1.56 (m, 2 H).

### Example 22

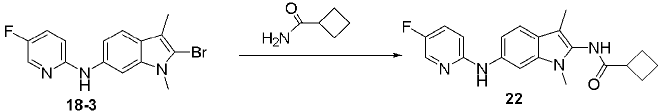

Compound 18-3 (0.05 g, 149.62 µmol) was added to dioxane (5 mL), followed by 1-cyclobutanecarboxamide (29.66 mg, 299.23 µmol), tris(dibenzylideneacetone)dipalladium(0) (13.70 mg, 14.96 µmol), 4,5-bis(diphenylphosphino)-9,9-dimethylxanthene (17.31 mg, 29.92 µmol) and cesium carbonate (146.25 mg, 448.85 µmol). The mixture was purged with nitrogen three times and stirred at 110 °C for 4 h. Water (30 mL) was added. The mixture was extracted with ethyl acetate (30 mL × 2). The organic phase was washed with saturated brine (30 mL), dried over anhydrous sodium sulfate, and the filtrate was concentrated to give a crude product. The crude product was purified by prep-HPLC (column: 2_Phenomenex Gemini C18 75*40 mm*3 µm; mobile phase: [water (ammonia) - acetonitrile]; acetonitrile ratio: 30%-60%) to give compound 22.LCMS (ESI) m/z: 353.2 [M+H]⁺. ¹H NMR (400 MHz, DMSO-d₆) δ ppm 9.44 (s, 1 H), 8.93 (s, 1 H), 8.10 (d, J=2.8 Hz, 1 H), 7.78 (s, 1 H), 7.49 (td, J=8.4, 2.8 Hz, 1 H), 7.33 (d, J=8.4 Hz, 1 H), 7.07 (dd, J=8.4, 1.6 Hz, 1 H), 6.85 (dd, J=9.2, 3.6 Hz, 1 H), 3.41 (s, 3 H), 3.34 (s, 1 H), 2.31-2.24 (m, 2 H), 2.22 - 2.17 (m, 2 H), 2.04 (s, 3 H), 2.02-1.91 (m, 2 H).

### Example 23

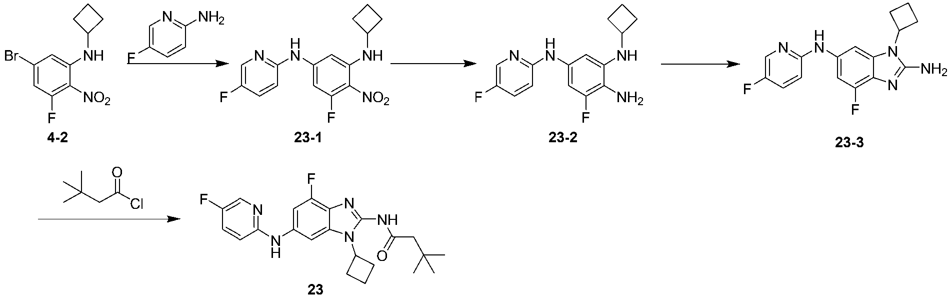

### Step 1

Compound 4-2 (2.4 g, 8.3 mmol) was added to dioxane (20 mL). 5-Fluoropyridin-2-amine (1.4 g, 12.45 mmol), methanesulfonato(2-dicyclohexylphosphino-2',6'-diisopropoxy-1,1'-biphenyl)(2-amino-1,1'-biphenyl-2-yl)palladium(II) (694.27 mg, 830.16 µmol), 2-dicyclohexylphosphino-2',6'-diisopropoxy-1,1'-biphenyl (387.38 mg, 830.16 µmol) and cesium carbonate (8.11 g, 24.90 mmol) were added. The mixture was purged with nitrogen three times, heated to 100 °C and reacted for 12 h. The mixture was diluted with water (20 mL), and extracted with ethyl acetate (20 mL × 2). The combined organic layers were washed with saturated brine (20 mL), dried over anhydrous sodium sulfate, and filtered. The filtrate was concentrated under reduced pressure to give a crude product, which was purified by column chromatography (petroleum ether/ethyl acetate, 1/0 ~ 2/1, V/V) to give compound 23-1. LCMS (ESI) m/z: 321.0 [M+H]⁺.

### Step 2

Compound 23-1 (0.6 g, 1.87 mmol) was added to ethanol (40 mL) and water (10 mL). Reduced iron powder (523.11 mg, 9.37 mmol) and ammonium chloride (501.01 mg, 9.37 mmol) were added, and the mixture was stirred at 90 °C for 16 h. The reaction system was cooled to room temperature, and filtered. Water (50 mL) was added to the filtrate, and the mixture was extracted with ethyl acetate (50 mL × 2). The combined organic layers were washed with saturated brine (50 mL), dried over sodium sulfate, and filtered. The filtrate was concentrated to give compound 23-2. LCMS (ESI) m/z: 291.0 [M+H]⁺.

### Step 3

Compound 23-2 (0.3 g, 1.03 mmol) was added to ethanol (10 mL), followed by cyanogen bromide (218.91 mg, 2.07 mmol). The mixture was stirred at 25 °C for 16 h. The aqueous layer was diluted with saturated sodium bicarbonate solution (50 mL) and extracted with ethyl acetate (50 mL × 2). The combined organic layers were washed with saturated brine (50 mL), dried over anhydrous sodium sulfate, and filtered. The filtrate was concentrated to give compound 23-3. LCMS (ESI) m/z: 316.0 [M+H]⁺.

### Step 4

Compound 23-3 (0.3 g, 0.951 mmol) was added to acetone (10 mL). N,N-Diisopropylethylamine (368.88 mg, 2.85 mmol) and tert-butylacetyl chloride (153.68 mg, 1.14 mmol) were added, and the mixture was stirred at 25 °C for 16 h. The reaction mixture was concentrated to give a crude product. 1N NaOH (20 mL) and acetone (5 mL) were added, and the mixture was stirred at 25 °C for 1 h, then extracted with ethyl acetate (50 mL × 2). The combined organic layers were washed with saturated brine (50 mL), dried over sodium sulfate, and filtered. The filtrate was concentrated under reduced pressure to obtain a crude product. The crude product was purified by column chromatography (petroleum ether/ethyl acetate, 1/0 → 2/1, V/V) to give compound 23. LCMS (ESI) m/z: 414.0 [M+H]⁺. ¹H NMR (CDCl₃, 400MHz): δ ppm 8.10 (d, J=3.0 Hz, 1H), 7.52 (d, J=1.6 Hz, 1H), 7.28-7.34 (m, 1H), 6.94 (dd, J=11.8, 1.6 Hz, 1H), 6.76 (dd, J=9.2, 3.4 Hz, 1H), 6.58 (s, 1H), 4.98 (br s, 1H), 2.82 (br s, 2H), 2.51-2.60 (m, 2H), 2.48 (s, 2H), 1.90-2.01 (m, 2H), 1.12 (s, 9H).

### Example 24

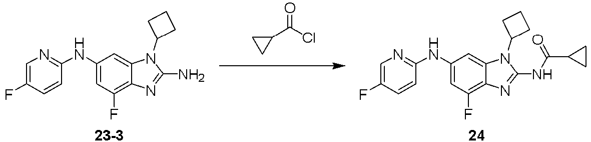

Compound 23-3 (0.3 g, 0.951 mmol) was added to tetrahydrofuran (20 mL). Cyclopropanecarbonyl chloride (0.123 g, 1.43 mmol), N,N-diisopropylethylamine (0.369 g, 2.85 mmol) and N,N,N',N'-tetramethyl-O-(7-azabenzotriazol-1-yl)uronium hexafluorophosphate (0.724 g, 1.9 mmol) were added, and the mixture was stirred at 50 °C for 16 h. The mixture was concentrated under reduced pressure to give a crude product, which was extracted with ethyl acetate (50 mL × 2). The combined organic layers were washed with saturated brine (50 mL), dried over anhydrous sodium sulfate, and filtered. The filtrate was concentrated under reduced pressure to obtain a compound. The crude product was purified by column chromatography (petroleum ether/ethyl acetate, 1/0 → 2/1, V/V) to give compound 24. LCMS (ESI) m/z: 384.2 [M+H]⁺. ¹H NMR (DMSO-d₆, 400MHz): δ 10.73 (br s, 1H), 9.28 (s, 1H), 8.18 (d, J=3.0 Hz, 1H), 7.98 (s, 1H), 7.56 (td, J=8.7, 3.1 Hz, 1H), 7.34 (br d, J=13.3 Hz, 1H), 6.88 (dd, J=9.3, 3.8 Hz, 1H), 4.76 - 4.64 (m, 1H) , 2.64-2.71 (m, 2H), 2.40-2.47 (m, 2H), 1.86-1.93 (m, 3H), 0.90-0.84 (m, 4H).

### Example 25

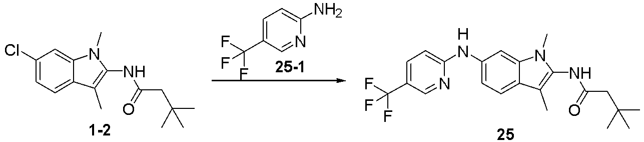

Compound 1-2 (0.1 g, 341.53 µmol) was added to dioxane (3 mL), followed by methanesulfonato(2-dicyclohexylphosphino-2,6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 25-1 (83.05 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, concentrated under reduced pressure to give a crude product. The crude product was purified by prep-HPLC (column: Welch Xtimate C18 150*25 mm*5 µm; mobile phase: [water (ammonia) - acetonitrile]; acetonitrile ratio: 45%-75%) and then concentrated under reduced pressure to obtain a crude product. The crude product was further purified by prep-TLC (dichloromethane/methanol, 10/1, V/V) to give compound 25. LCMS (ESI) m/z: 419.1 [M+H]⁺. ¹H NMR (400 MHz, DMSO-d₆) δ ppm 9.61 (s, 1H), 9.49 (s, 1H), 8.45 (s, 1H), 7.76-7.80 (m, 2H), 7.40 (d, J = 8.4 Hz, 1H), 7.13 (dd, J = 8.4, 1.6 Hz, 1H), 6.89 (d, J = 8.8 Hz, 1H), 3.47 (s, 3H), 2.27 (s, 2H), 2.08 (s, 3H), 1.09 (s, 9H).

### Example 26

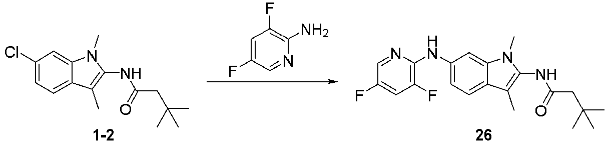

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-3,5-difluoropyridine (66.65 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, concentrated under reduced pressure to give a crude product. Methanol (1 mL) was added to the crude product, and the mixture was stirred at room temperature for 1 h, then filtered. The filter cake was dried to give compound **26.** LCMS (ESI) m/z: 387.1 [M+H]⁺. ¹H NMR (400 MHz, DMSO-d₆) δ ppm 9.58 (s, 1H), 8.70 (s, 1H), 8.02 (d, J = 2.4 Hz, 1H), 7.75-7.82 (m, 2H), 7.32-7.36 (m, 1H), 7.24-7.30 (m, 1H), 3.45 (s, 3H), 2.26 (s, 2H), 2.07 (s, 3H), 1.08 (s, 9H).

### Example 27

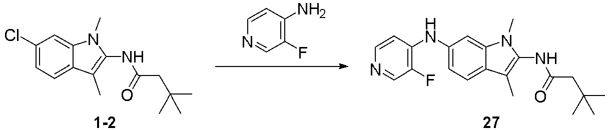

Compound **1-2** (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 4-amino-3-fluoropyridine (57.43 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, concentrated under reduced pressure to give a crude product. The crude product was purified by prep-HPLC (column: Welch Xtimate C18 150*25mm*5µm; mobile phase: [water (ammonia) - acetonitrile]; acetonitrile ratio: 35%-65%) to give compound **27.** LCMS (ESI) m/z: 369.1 [M+H]⁺. ¹H NMR (400 MHz, DMSO-d₆) δ ppm 9.65 (s, 1H), 8.58 (s, 1H), 8.23 (d, J = 4.0 Hz, 1H), 7.95 (d, J = 5.6 Hz, 1H), 7.46 (d, J = 8.4 Hz, 1H), 7.24 (d, J = 1.2 Hz, 1H), 6.88-7.00 (m, 2H), 3.48 (s, 3H), 2.27 (s, 2H), 2.10 (s, 3H), 1.09 (s, 9H).

### Example 28

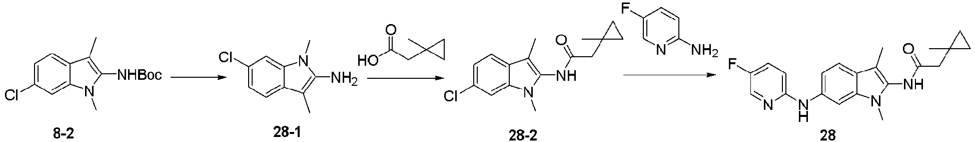

### Step 1

Compound 8-2 (0.2 g, 678.48 µmol) was added to ethyl acetate (5 mL). HCl in ethyl acetate (2 M, 1.70 mL) was added, and the reaction system was heated to 50 °C and stirred for 1 h. The reaction mixture was concentrated, then ethyl acetate (5 mL) was added. The mixture was stirred for 10 min, then filtered. The filter cake was collected and dried to give crude compound 28-1 hydrochloride. LCMS (ESI) m/z: 195.1 [M+H]⁺.

### Step 2

Crude compound 28-1 hydrochloride (120 mg) was added to ethyl acetate (5 mL). 2-(1-Methylcyclopropyl)acetic acid (84.81 mg, 756.57 µmol), 1-butylphosphonic anhydride (1,3,5,2,4,6-trioxatriphosphorinane, CAS: 163755-62-2, 1.50 g, 2.08 mmol, 50% in ethyl acetate) and pyridine (5.19 mmol, 419.08 µL) were sequentially added. The flask was evacuated and purged with nitrogen three times. The reaction system was heated to 50 °C and stirred for 1 h. After cooling to room temperature, the reaction mixture was adjusted to basic pH with sodium bicarbonate solution, and extracted with ethyl acetate (30 mL × 2). The organic phase was collected, washed with saturated brine (30 mL), dried over anhydrous sodium sulfate, and filtered. The filtrate was concentrated, and purified by column chromatography (petroleum ether/ethyl acetate, 10/1 → 3/1) to give compound 28-2.

### Step 3

Compound 28-2 (0.11 g, 378.28 µmol) was added to dioxane (5 mL). 2-Amino-5-fluoropyridine (84.81 mg, 756.57 µmol), methanesulfonato(2-dicyclohexylphosphino-2',6'-diisopropoxy-1,1'-biphenyl)(2-amino-1,1'-biphenyl-2-yl)palladium(II) (31.64 mg, 37.83 µmol), 2-dicyclohexylphosphino-2',6'-diisopropoxy-1,1'-biphenyl (35.30 mg, 75.66 µmol) and cesium carbonate (246.50 mg, 756.57 µmol) were added into the reaction system in sequence. The flask was evacuated and purged with nitrogen three times. The reaction system was heated to 110 °C and stirred for 3 h. After cooling to room temperature, the mixture was filtered by suction. The filter cake was washed with ethyl acetate (2 mL), and the filtrate was concentrated. The residue was purified by column chromatography (petroleum ether/ethyl acetate, 10/1-1/1) to give compound 28. LCMS (ESI) m/z: 367.1 [M+H]⁺. ¹H NMR (400 MHz, DMSO-d₆) δ ppm 9.49 (s, 1 H) 8.93 (s, 1 H) 8.11 (d, J=3.2 Hz, 1 H) 7.77 (d, J=1.6 Hz, 1 H) 7.50 (td, J=8.4, 3.2 Hz, 1 H) 7.34 (d, J=8.4 Hz, 1 H) 7.08 (dd, J=8.4, 1.6 Hz, 1 H) 6.85 (dd, J=9.2, 3.6 Hz, 1 H) 3.45 (s, 3 H) 2.28 (s, 2 H) 2.07 (s, 3 H) 1.18 (s, 3 H) 0.60 - 0.54 (m, 2 H) 0.38 - 0.33 (m, 2 H).

### Example 29

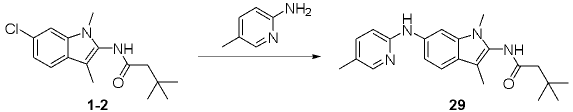

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-5-methylpyridine (55.40 mg, 512.29 µmol) in sequence. The mixture was heated to 110 °C under nitrogen and reacted for 12 h. After cooling to room temperature, the reaction mixture was filtered. The filter cake was washed with ethyl acetate (10 mL), and the filtrate was concentrated under reduced pressure to give a crude product. The crude product was purified by prep-HPLC (column: Welch Xtimate C18 150*30mm*5µm; mobile phase: [water (ammonia) - acetonitrile]; acetonitrile ratio: 20%-50%) and then concentrated under reduced pressure to obtain a crude product. The crude product was further purified by prep-TLC (dichloromethane/methanol, 10/1, V/V) to give compound **29.** LCMS (ESI) m/z: 365.3 [M+H]⁺. ¹H NMR (400 MHz, DMSO-d₆) δ ppm 9.54 (s, 1H), 8.69 (s, 1H), 7.98 (s, 1H), 7.79 (d, J = 1.6 Hz, 1H), 7.28-7.39 (m, 2H), 7.07 (dd, J = 8.4, 1.6 Hz, 1H), 6.76 (d, J = 8.0 Hz, 1H), 3.44 (s, 3H), 2.26 (s, 2H), 2.16 (s, 3H), 2.06 (s, 3H), 1.08 (s, 9H).

### Example 30

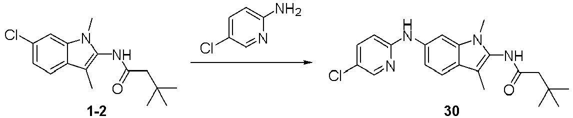

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-5-chloropyridine (65.86 mg, 512.29 µmol) in sequence. The reaction mixture was heated to 110 °C under nitrogen and reacted for 12 h. After cooling to room temperature, the reaction mixture was filtered. The filter cake was washed with ethyl acetate (10 mL), and the filtrate was collected and concentrated under reduced pressure to give a crude product. The crude product was purified by column chromatography (dichloromethane/methanol, 100/0 ~ 10/1, V/V) to give a crude compound 30, which was further purified by prep-HPLC (column: Welch Xtimate C18 150*25mm*5µm; mobile phase: [water (ammonia) - acetonitrile]; acetonitrile ratio: 45%-75%), and concentrated under reduced pressure to give compound 30. LCMS (ESI) m/z: 385.1 [M+H]⁺. ¹H NMR (400 MHz, DMSO-d₆) δ ppm 9.57 (s, 1H), 9.07 (s, 1H), 8.13 (d, J = 2.4 Hz, 1H), 7.73 (d, J = 1.2 Hz, 1H), 7.57 (dd, J = 9.2, 2.8 Hz, 1H), 7.35 (d, J = 8.0 Hz, 1H), 7.09 (dd, J = 8.8, 2.0 Hz, 1H), 6.83 (d, J = 8.8 Hz, 1H), 3.45 (s, 3H), 2.26 (s, 2H), 2.07 (s, 3H), 1.08 (s, 9H).

### Example 31

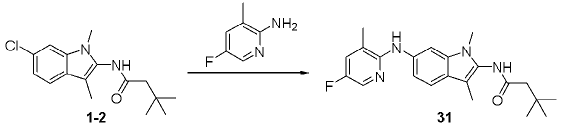

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-3-methyl-5-fluoropyridine (64.62 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, and concentrated under reduced pressure to give a crude product. The crude product was purified by column chromatography (petroleum ether/ethyl acetate, 100/0 → 1/1, V/V) to give a crude product. Methyl tert-butyl ether (1 mL) was added, and the mixture was stirred at room temperature for 1 h, then filtered. The filter cake was dried to give compound 31. LCMS (ESI) m/z: 383.3 [M+H]⁺. ¹H NMR (400 MHz, DMSO-d₆) δ ppm 9.56 (s, 1H), 7.92 (d, *J* = 3.2 Hz, 1H), 7.74 (s, 1H), 7.64 (d, *J =* 1.6 Hz, 1H), 7.43 (dd, *J* = 8.8, 2.0 Hz, 1H), 7.32 (d, *J* = 8.4 Hz, 1H), 7.20 (dd, *J* = 8.4, 1.6 Hz, 1H), 3.44 (s, 3H), 2.29 (s, 3H), 2.26 (s, 2H), 2.07 (s, 3H), 1.08 (s, 9H).

### Example 32

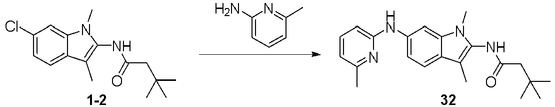

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-6-methylpyridine (55.40 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, and concentrated under reduced pressure to give a crude product. The crude product was purified by column chromatography (petroleum ether/ethyl acetate, 100/0 → 1/1, V/V) to give a crude product. Methanol (1 mL) was added, and the mixture was stirred at room temperature for 1 h, then filtered. The filter cake was dried to give compound 32. LCMS (ESI) m/z: 365.3 [M+H]⁺. ¹H NMR (400 MHz, DMSO-d₆) δ ppm 9.54 (s, 1H), 8.75 (s, 1H), 7.90 (s, 1H), 7.39 (t, J = 8.0 Hz, 1H), 7.32 (d, J = 8.4 Hz, 1H), 7.06 (dd, J = 8.4, 1.6 Hz, 1H), 6.62 (d, J = 8.0 Hz, 1H), 6.54 (d, J = 7.6 Hz, 1H), 3.45 (s, 3H), 2.37 (s, 3H), 2.26 (s, 2H), 2.07 (s, 3H), 1.09 (s, 9H).

### Example 33

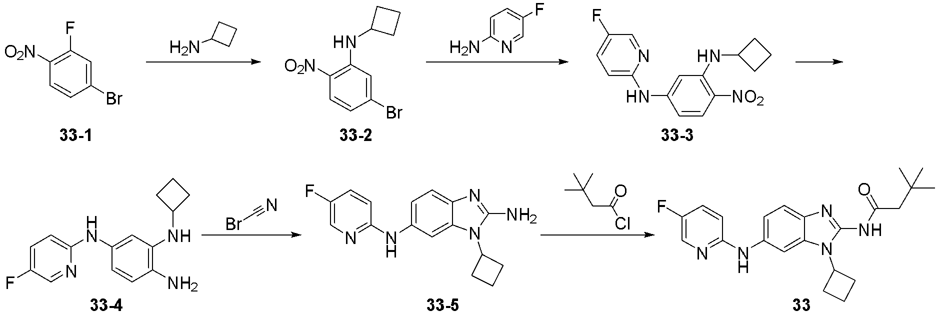

### Step 1

Compound 33-1 (1 g, 4.55 mmol) was added to tetrahydrofuran (20 mL). Cyclobutylamine (387.94 mg, 5.45 mmol) and N,N-diisopropylethylamine (1.76 g, 13.64 mmol) were added, and the mixture was stirred at 25 °C for 12 h. The mixture was diluted with water (100 mL), and extracted with ethyl acetate (100 mL × 2). The combined organic layers were washed with saturated brine (50 mL), dried over anhydrous sodium sulfate, filtered, and concentrated under reduced pressure to give compound 33-2. LCMS (ESI) m/z: 271.0, 273.0 [M+H]⁺.

### Step 2

Compound 33-2 (1.35 g, 4.98 mmol) was added to dioxane (20 mL). 2-Amino-5-fluoropyridine (1.12 g, 9.96 mmol), cesium carbonate (3.24 g, 9.96 mmol), 2-dicyclohexylphosphino-2',6'-diisopropoxy-1,1'-biphenyl (464.72 mg, 995.91 µmol) and methanesulfonato(2-dicyclohexylphosphino-2',6'-diisopropoxy-1,1'-biphenyl)(2-amino-1,1'-biphenyl-2-yl)palladium(II) (416.47 mg, 497.95 µmol) were added in sequence. The reaction mixture was purged with nitrogen three times, heated to 110 °C and reacted for 3 h. After cooling to room temperature, water (100 mL) was added, and the mixture was extracted with ethyl acetate (100 mL × 2). The combined organic layers were washed with saturated brine (100 mL), dried over anhydrous sodium sulfate. The residue was purified by silica gel column chromatography (petroleum ether/ethyl acetate, 10/1~4/1, V/V) to give compound 33-3. LCMS (ESI) m/z: 303.0[M+H]⁺.

### Step 3

To compound 33-3 (300 mg, 992.38 µmol) were added ethanol (20 mL) and water (5 mL), followed by iron powder (277.10 mg, 4.96 mmol) and ammonium chloride (265.42 mg, 4.96 mmol). The mixture was stirred at 90 °C for 3 h. After cooling to room temperature, the reaction mixture was filtered, and the filter cake was washed with ethyl acetate (30 mL). Water (50 mL) was added to the filtrate, and the mixture was extracted with ethyl acetate (50 mL × 2). The combined organic layers were washed with saturated brine (50 mL), dried over anhydrous sodium sulfate. The residue was purified by silica gel column chromatography (petroleum ether/ethyl acetate, 10/1~1/1, V/V) to give compound 33-4. LCMS (ESI) m/z: 273.0 [M+H]⁺.

### Step 4

Compound 33-4 (206 mg, 756.46 µmol) was added to ethanol (10 mL). Cyanogen bromide (160.25 mg, 1.51 mmol) was added, and the mixture was stirred at 25 °C for 4 h. The aqueous layer was diluted with saturated sodium bicarbonate solution (50 mL) and extracted with ethyl acetate (50 mL × 1). The combined organic layer was washed with saturated brine (50 mL), dried over anhydrous sodium sulfate, filtered, and concentrated under reduced pressure to give compound 33-5. MS-ESI LCMS (ESI) m/z: 298.0 [M+H]⁺.

### Step 5

Compound 33-5 (100 mg, 336.33 µmol) was added to acetone (6 mL). N,N-Diisopropylethylamine (130.40 mg, 1.01 mmol) and tert-butylacetyl chloride (49.80 mg, 369.96 µmol) were added, and the mixture was stirred at 0 °C for 1 h. Water (50 mL) was added, and the reaction mixture was extracted with ethyl acetate (50 mL × 2). The combined organic layers were washed with saturated brine (50 mL), dried over anhydrous sodium sulfate. The residue was purified by silica gel column chromatography (petroleum ether/ethyl acetate, 10/1-1/1) to give compound 33. LCMS (ESI) m/z: 396.1[M+H]⁺. ¹H NMR (CDCl₃, 400MHz): δ ppm 8.39 (d, *J*=1.6 Hz, 1H), 8.02 (d, *J*=2.8 Hz, 1H), 7.48 - 7.35 (m, 2H), 7.23 - 7.16 (m, 1H), 6.87 - 6.81 (m, 1H), 4.62 - 4.54 (m, 1H), 3.03 - 2.91 (m, 2H), 2.58 - 2.48 (m, 2H), 2.35 (s, 2H), 2.10 - 1.92 (m, 2H), 1.14 (s, 9H).

### Example 34

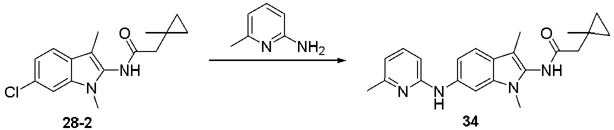

Compound 28-2 (0.1 g, 343.89 µmol) was added to dioxane (5 mL), followed by 2-amino-6-methylpyridine (74.38 mg, 687.79 µmol), methanesulfonato(2-dicyclohexylphosphino-2',6'-diisopropoxy-1,1'-biphenyl)(2-amino-1,1'-biphenyl-2-yl)palladium(II) (28.76 mg, 34.39 µmol), 2-dicyclohexylphosphino-2',6'-diisopropoxy-1,1'-biphenyl (32.09 mg, 68.78 µmol) and cesium carbonate (224.10 mg, 687.79 µmol) in sequence. The flask was evacuated and purged with nitrogen three times. The reaction system was heated to 110 °C and stirred for 2.5 h. After cooling to room temperature, the mixture was filtered. The filter cake was washed with ethyl acetate (3 mL), and the filtrate was concentrated. The residue was purified by column chromatography (petroleum ether/ethyl acetate, 10/1-1/1) to give compound 34. LCMS (ESI) m/z: 363.6[M+H]⁺. ¹HNMR (400MHz, DMSO-d₆) δ ppm 9.48 (s, 1H), 8.76 (s, 1H), 7.92 (d, J=2.0Hz, 1H), 7.39 (t , J=8.0, 1H), 7.33 (d, J=8.8 Hz, 1H), 7.07 (dd, J=8.8, 2.0Hz, 1H), 6.62 (d, J=8.4 Hz, 1H), 6.54 (d, J=7.2 Hz, 1H), 3.45(s, 3H), 2.37(s, 3H), 2.28(s, 2H), 2.07(s, 3H), 1.18(s, 3H), 0.60-0.53(m, 2H), 0.39-0.31 (m, 2H).

### Example 35

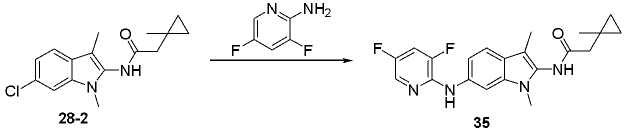

Compound 28-2 (0.1 g, 343.89 µmol) was added to dioxane (5 mL), followed by 2-amino-3,5-difluoropyridine (89.48 mg, 687.79 µmol), methanesulfonato(2-dicyclohexylphosphino-2',6'-diisopropoxy-1,1'-biphenyl)(2-amino-1,1'-biphenyl-2-yl)palladium(II) (28.76 mg, 34.39 µmol), 2-dicyclohexylphosphino-2',6'-diisopropoxy-1,1'-biphenyl (32.09 mg, 68.78 µmol) and cesium carbonate (224.10 mg, 687.79 µmol) in sequence. The flask was evacuated and purged with nitrogen three times. The reaction system was heated to 110 °C and stirred for 2.5 h. After cooling to room temperature, the mixture was filtered. The filter cake was washed with ethyl acetate (3 mL), and the filtrate was concentrated. The residue was purified by column chromatography (petroleum ether/ethyl acetate, 10/1 → 1/1) to give compound 35. LCMS (ESI) m/z: 384.9[M+H]⁺. ¹H NMR (400 MHz, DMSO-*d*₆) δ ppm 9.51 (s, 1 H), 8.70 (d, *J*=1.2 Hz, 1 H), 8.03 (d, *J*=2.4 Hz, 1 H), 7.82 - 7.74 (m, 2 H), 7.37 - 7.33 (m, 1 H), 7.31 - 7.24 (m, 1 H), 3.45 (s, 3 H), 2.29 (s, 2 H), 2.08 (s, 3 H), 1.18 (s, 3 H), 0.59 - 0.55 (m, 2 H), 0.38 - 0.34 (m, 2H).

### Example 36

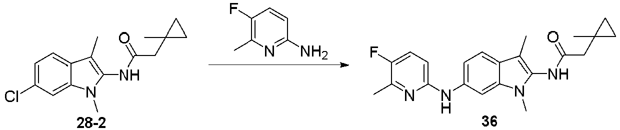

Compound 28-2 (50 mg, 171.95 µmol) was added to dioxane (3 mL), followed by 2-amino-5-fluoro-6-methylpyridine (43.38 mg, 343.89 µmol), methanesulfonato(2-dicyclohexylphosphino-2',6'-diisopropoxy-1,1'-biphenyl)(2-amino-1,1'-biphenyl-2-yl)palladium(II) (14.38 mg, 17.19 µmol), 2-dicyclohexylphosphino-2',6'-diisopropoxy-1,1'-biphenyl (16.05 mg, 34.39 µmol) and potassium tert-butoxide (57.88 mg, 515.84 µmol) in sequence. The flask was evacuated and purged with nitrogen three times. The reaction mixture was heated to 110 °C and stirred for 2.5 h. After cooling to room temperature, the reaction mixture was filtered. The filter cake was washed with ethyl acetate (3 mL), and the filtrate was concentrated. The residue was purified by column chromatography (petroleum ether/ethyl acetate, 10/1 - 1/1) to give compound 36. LCMS(ESI) m/z: 381.2[M+H]⁺. ¹HNMR (400MHz, DMSO-d₆) δ ppm 9.49 (s, 1H), 8.84 (s, 1H), 7.89(d, J=1.6 Hz, 1H), 7.40(t, J=9.2 Hz, 1H), 7.33(d, J=8.4 Hz, 1 H), 7.04(dd, J=8.8, 2.0Hz, 1H), 6.68(dd, J=9.2, 2.4 Hz, 1H), 3.45(s, 3H), 2.37(d, J=2.8Hz, 3H), 2.29(s, 2H), 2.07(s, 3H), 1.18(s, 3H), 0.60-0.54(m, 2H), 0.37-0.35(m, 2H).

### Example 37

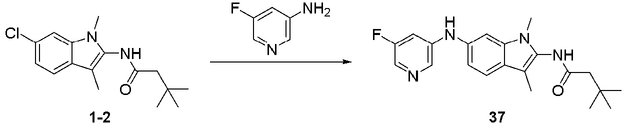

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 3-amino-5-fluoropyridine (57.94 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, and concentrated under reduced pressure to give a crude product. The crude product was purified by column chromatography (petroleum ether/ethyl acetate, 100/0 ~ 1/1, V/V) to give a crude product. Ethyl acetate (1 mL) was added, and the mixture was stirred at room temperature for 1 h, then filtered. The filter cake was dried to give compound 37. LCMS(ESI) m/z: 369.2 [M+H]⁺. ¹HNMR (400 MHz, DMSO-d₆) δ ppm 9.62 (s, 1H), 8.54 (s, 1H), 8.13 (br s, 1H), 7.84 (s, 1H), 7.42 (br d, J = 8.0 Hz, 1H), 7.08-7.18 (m, 2H), 6.87 (br d, J = 8.4 Hz, 1H), 3.47 (s, 3H), 2.27 (s, 2H), 2.08 (s, 3H), 1.06-1.10 (m, 9H).

### Example 38

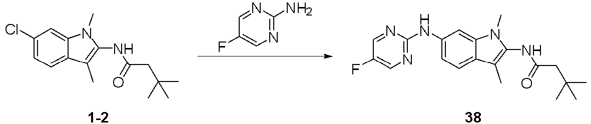

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-5-fluoropyrimidine (57.94 mg, 512.29 µmol) in sequence. The reaction mixture was heated to 110 °C under nitrogen and reacted for 12 h. After cooling to room temperature, the reaction mixture was filtered. The filter cake was washed with ethyl acetate (10 mL), and the filtrate was collected, and concentrated under reduced pressure to give a crude product. The crude product was purified by column chromatography (petroleum ether/ethyl acetate, 100/0 ~ 1/1, V/V) to give compound **38.** LCMS(ESI) m/z: 370.0[M+H]⁺. ¹H NMR (400 MHz, DMSO-d₆) δ ppm 9.59 (d, J = 9.0 Hz, 2H), 8.54 (d, J = 0.8 Hz, 2H), 7.83 (d, J = 1.8 Hz, 1H), 7.34 (d, J = 8.5 Hz, 1H), 7.22 (dd, J = 8.5, 1.8 Hz, 1H), 3.45 (s, 3H), 2.26 (s, 2H), 2.07 (s, 3H), 1.08 (s, 9H).

### Example 39

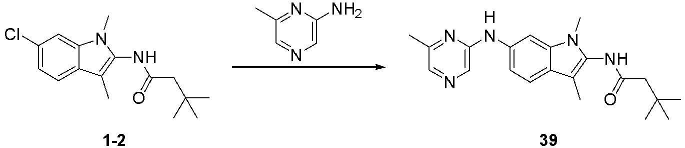

Compound 1-2 (0.1 g, 341.53 µmol) was added to dioxane (3 mL), followed by methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol), 2-dicyclohexylphosphino-2',6'-diisopropoxy-1,1'-biphenyl (31.87 mg, 68.31 µmol), cesium carbonate (333.83 mg, 1.02 mmol) and 2-amino-6-methylpyrazine (55.91 mg, 512.29 µmol) in sequence. The reaction mixture was heated to 110 °C under nitrogen and reacted for 12 h. After cooling to room temperature, the mixture was filtered. The filter cake was washed with ethyl acetate (10 mL), and the filtrate was collected, and concentrated under reduced pressure to give a crude product.. The crude product was purified by column chromatography (petroleum ether/ethyl acetate, 100/0~1/1, V/V) to give a crude product. Methyl tert-butyl ether (1 mL) was added, and the mixture was stirred at room temperature for 1 h, then filtered. The filter cake was dried to give compound 39. LCMS(ESI) m/z: 366.2 [M+H]⁺. ¹H NMR (400 MHz, DMSO-d₆) δ ppm 9.59 (s, 1H), 9.26 (s, 1H), 8.01 (s, 1H), 7.93 (d, J = 1.6 Hz, 1H), 7.75 (s, 1H), 7.37 (d, J = 8.4 Hz, 1H), 7.13 (dd, J = 8.4, 1.6 Hz, 1H), 3.46 (s, 3H), 2.37 (s, 3H), 2.27 (s, 2H), 2.07 (s, 3H), 1.08 (s, 9H).

### Example 40

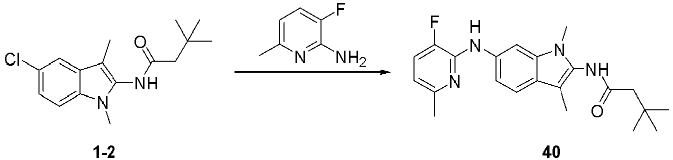

Compound 1-2 (100 mg, 341.53 µmol) was dissolved in dioxane (3 mL). 3-Fluoro-6-methylpyridin-2-amine (51.69 mg, 409.83 µmol), cesium carbonate (333.83 mg, 1.02 mmol), 2-dicyclohexylphosphino-2',6'-diisopropoxy-1,1'-biphenyl (31.87 mg, 68.31 µmol) and methanesulfonato(2-dicyclohexylphosphino-2',6'-diisopropoxy-1,1'-biphenyl)(2-amino-1,1'-biphenyl-2-yl)palladium(II) (28.56 mg, 34.15 µmol) were added in sequence. The mixture was purged with nitrogen three times and reacted at 110 °C for 12 h. After cooling to room temperature, the mixture was filtered under reduced pressure, and the filtrate was collected, and concentrated under reduced pressure to give a crude product. The crude product was purified by prep-HPLC (column: Welch Xtimate C18 150*25mm*5µm; mobile phase: [water (ammonia) - acetonitrile]; acetonitrile ratio: 47%-77%) to give compound 40. LCMS(ESI) m/z:383.1[M+H]⁺. ¹HNMR (400 MHz, CDCl₃) δ ppm 9.59 (s, 1 H), 8.59 (d, *J*=2 Hz, 1 H), 8.03 (s, 1 H), 7.37 (dd, *J*=12, 8 Hz, 1 H), 7.32 - 7.29 (m, 2 H), 6.57 (dd, *J*=8, 2.8 Hz, 1 H), 3.45 (s, 3 H), 2.36 (s, 3 H), 2.27 (s, 2 H), 2.07 (s, 3 H), 1.09 (s, 9 H).

### Example 41

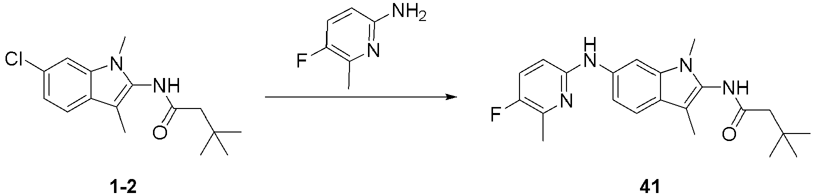

Compound 1-2 (500 mg, 1.71 mmol) was added to dioxane (10 mL), followed by (2-dicyclohexylphosphino-2,6-diisopropoxy-1,1-biphenyl)[2-(2-amino-1,1-biphenyl)] (142.82 mg, 170.76 µmol), 2-dicyclohexylphosphino-2,6-diisopropoxy-1,1-biphenyl (159.37 mg, 341.53 µmol), potassium tert-butoxide (574.85 mg, 5.12 mmol) and 2-amino-5-fluoro-6-methylpyridine (323.08 mg, 2.56 mmol) in sequence. The reaction mixture was heated to 110 °C under nitrogen and reacted for 2 h. The reaction mixture was cooled to room temperature, and filtered. The filter cake was washed with ethyl acetate (10 mL), and the filtrate was collected, and concentrated under reduced pressure to give a crude product. The crude product was purified by column chromatography (petroleum ether/ethyl acetate, 100/0 ~ 1/1, V/V) to give a crude product. Ethyl acetate/methanol (20/1, 10 V) was added, and the mixture was stirred at room temperature for 1 h, then filtered. The filter cake was dried to give compound 41. LCMS (ESI) m/z: 383.2[M+H]⁺. ¹H NMR (400 MHz, DMSO-d₆) δ ppm 9.55 (br s, 1H), 8.82 (br s, 1H), 7.88 (br s, 1H), 7.36 (t, J = 9.2 Hz, 1H), 7.32 (d, J = 8.0 Hz, 1H), 7.03 (dd, J = 8.0, 1.6 Hz, 1H), 6.67 (dd, J = 6.0, 2.8 Hz, 1H), 3.45 (br s, 3H), 2.50 (br s, 3H), 2.36 (d, J = 3.2 Hz, 2H), 2.06 (br s, 3H), 1.08 (br s, 9H).

### Biological Testing

### Assay Example 1: hKCNQ2/3 Thallium Flux Assay

Test Objective: To test the in vitro concentration-response relationship of compounds using FLIPR thallium flux assay in CHO cell lines stably expressing hKCNQ2/3.

### Test Cells and Reagents:

(1) Cell line: CHO cells stably transfected with hKCNQ2/3.
(2) Cell culture medium reagents: including F12 medium, fetal bovine serum (FBS, 10%), penicillin-streptomycin (Invitrogen, Cat. No. 15140-122), G418 (Invitrogen, Cat. No. 10131027).
(3) Cell culture:
   This cell line was passaged three times per week at a split ratio of 1:2 to 1:3. When the cells grew to >80% confluence in a T-75 flask, they were digested with 0.25% trypsin-EDTA solution for approximately 1 minute, and then transferred from the flask for passaging. According to the split ratio, the cells were transferred to another T-75 flask containing complete cell growth medium. Note: To maintain logarithmic growth, the cells should be kept in a subconfluent monolayer state. Based on the cell doubling time, the cells were passaged every 2-3 days.

### Assay Procedure:

(1) Day 1 - Cell preparation: Cells were digested as described above, and cell density and viability were determined using a Cell Countess. The volume of the single-cell suspension was adjusted with complete cell growth medium. Then, hKCNQ2/3_CHO cells were seeded onto PDL-precoated 384-well plates at a density of approximately 20,000 cells/well (30 µL/well). The plates were then incubated overnight in a 37°C, 5% CO₂ humidified air incubator.
(2) Day 2 - Compound preparation: Test compounds were diluted with dimethyl sulfoxide (DMSO) to stock concentrations and stored in a -20°C freezer. A compound addition program was set up on the ECHO liquid handler, and compounds were added to the plates using the ECHO liquid handler. Retigabine (RTG) was used as a positive control at a highest concentration of 100 µM with 3-fold serial dilutions for a total of 9 doses. Buffer containing 5 mM K⁺ and 1 mM Tl⁺ was added to the plates.
   Notes: 1) K₂SO₄ and Tl₂SO₄ were prepared in 1× chloride-free buffer. Depending on the voltage-gated potassium channel involved, the concentrations of Tl⁺ and K⁺ should be adjusted. Tl⁺ concentration is approximately 0.5 mM -5 mM, and K⁺ concentration is approximately 5 mM -30 mM. 2) Each 1 mM solution of K₂SO₄ and Tl₂SO₄ contains 2 mM K⁺ and Tl⁺; the final concentration should be calculated based on the ion concentration. 3) TlCl readily precipitates and a chloride-free buffer is required.
(3) Day 2 - Assay testing: Once the cells reached the desired confluence, the cell assay plates were removed from the incubator. Assay buffer and diluted Tl⁺ dye (Molecular Devices # R8222) were prepared. The medium was discarded using a Bravo liquid handler, and 25 µL of Tl⁺ dye was added to each well. After 1 hour of incubation, the cell assay plates, composite plates and FLIPR tips were placed in the FLIPR^{TETRA} (Molecular Devices, USA) for FLIPR detection. After setting up the tips, baseline readings were taken for 60 seconds, then 12.5 µL was added from the composite plate (3×) to the cell assay plate, and data were recorded for at least 5 minutes.
(4) Data processing:
   Maximum signals were generated by the FLIPR software. Data analysis was performed using Excel (2013) and Prism 6.01. Data quality control: S/B > 2.00, Z factor > 0.50. IC₅₀ or EC₅₀ was defined as the midpoint between the lowest and highest plateaus, calculated using Prism 6.01 with a four-parameter logistic model.

The assay results are shown in Table 1.

**Table 1. Agonistic effect of the compounds of the present disclosure on hKCNQ2/3**

| Compound No. | EC₅₀ (µM) |
|---|---|
| Compound 1 | 0.96 |
| Compound 5 | 0.52 |
| Compound 11 | 0.20 |
| Compound 12 | 0.80 |
| Compound 13 | 0.50 |
| Compound 16 | 0.70 |
| Compound 21 | 0.22 |
| Compound 22 | 0.43 |
| Compound 26 | 0.40 |
| Compound 28 | 0.56 |
| Compound 29 | 0.41 |
| Compound 30 | 0.21 |
| Compound 32 | 0.33 |
| Compound 34 | 0.31 |
| Compound 35 | 0.49 |
| Compound 36 | 0.22 |
| Compound 38 | 0.82 |
| Compound 41 | 0.29 |

**Conclusion:** The compounds of the present disclosure exhibit significant agonistic activity on KCNQ2/3.

### Assay Example 2: Evaluation of the behavioral pharmacodynamics of the test compounds in a maximal electroshock-induced epilepsy model

### Purpose of the assay:

This study used behavioral tests in male CD-1 mice to investigate the effects of test compounds on acute epileptic seizures behavior induced by maximal electroshock.

### Assay materials:

Male CD-1 mice (25 to 35g) were used as the experimental animals and fed with standard feed (Beijing Vital River Laboratory Animal Technology Co., Ltd.).

### Assay procedure:

After arriving at the animal facility, the experimental animals needed to acclimatize for at least one week. The mice were observed daily for vital signs, and randomly divided into groups according to their body weights one day before the drug administration. One hour before the electrical stimulation, different dosages of the test compound were orally administered to each group. On the day of the assay, the male CD-1 mice in each group were anesthetized locally at the cornea with 2% lidocaine. Then, silver bipolar electrodes were used to electrically stimulate the cornea of the mice with 18 mA, 60 Hz, a pulse width of 0.6 ms, and a stimulation time of 1 s to induce seizures. Subsequently, the latency of seizures and the duration of limb rigidity in mice after the electrical stimulation were observed, and the protection rate from seizures for each group was statistically analyzed.

The assay results are shown in the table below:

**Table 2. Pharmacodynamic test results of the compounds of the present disclosure**

| Compound No. | Dose (mg/kg) | Seizure incidence (%) |
|---|---|---|
| Compound 1 | 5 | 16.7 |
| Compound 5 | 0.15; 5 | 66.7; 16.7 |
| Compound 31 | 5 | 16.7 |
| Compound 36 | 5 | 0 |
| Compound 41 | 1.5; 5 | 33.3; 0 |

**Conclusion:** The compounds of the present disclosure exhibit good antiseizure activity in the maximal electroshock-induced seizure model.

### Assay Example 3: Pharmacokinetic evaluation of the compounds

### Test Objective:

The purpose of this study was to evaluate the pharmacokinetic behavior of the test compounds after single intravenous bolus and oral gavage administration, to determine the bioavailability after oral gavage, and to provide animal data for clinical research.

### Test Materials:

CD-1 mice (male, 7-9 weeks old).

### Assay Procedure:

The pharmacokinetic profiles of the test compounds in rodents after intravenous injection and oral administration were evaluated using standard protocols. The test compounds were formulated as clear solutions and administered to mice via a single intravenous injection or oral administration. The vehicle for both intravenous and oral administration was a mixture of 5% DMSO / 60% PEG400 / 35% water. This study utilized four female CD-1 mice: two mice received intravenous administration, with plasma samples collected at 0.083, 0.25, 0.5, 1, 2, 4, 8, and 24 hours post-dosing; the remaining two mice received oral gavage, with plasma samples collected at 0.25, 0.5, 1, 2, 4, 8, and 24 hours post-dosing. Plasma samples were obtained by centrifuging the collected blood at 3,200 × g for 10 minutes at 4°C to separate the supernatant. Protein precipitation was performed by adding a 20-fold volume of methanol solution containing an internal standard to the plasma samples. The mixture was then centrifuged at 12,000 × g for 15 minutes at 4°C; 50 µL of the resulting supernatant was transferred to a 96-well plate, centrifuged again, and the supernatant was injected for analysis. LC-MS/MS was used for quantitative analysis of plasma concentrations, and pharmacokinetic parameters such as peak plasma concentration (Cₘₐₓ), clearance (CL), half-life (T_{1/2}), volume of distribution (Vdss), area under the concentration-time curve (AUC₀₋ₗₐₛₜ), and bioavailability (F) were calculated.

The assay results are shown in Table 3 below:

**Table 3. Pharmacokinetic test results of the compounds of the present disclosure**

| **Compound No.** | Dose mg/kg | Peak plasma concentration Cmax (nM) | Clearance CL (ml/min/kg) | Volume of distribution Vdss (L/kg) | Half-life T_{1/2} (IV, h) | Area under the concentration-time curve AUC0-last PO (nM.hr) | Bioavailability F (%) |
|---|---|---|---|---|---|---|---|
| **Compound 1** | IV:1 | 596 | 41.8 | 5.06 | 1.63 | 4566 | 83.9 |
| | PO:5 | | | | | | |
| **Compound 1** | IV:0.5 | 322 | 34.8 | 5.16 | 1.83 | 2041 | 79.6 |
| | PO:2 | | | | | | |
| **Compound 4** | IV:0.5 | 548 | 17.9 | 2.14 | 1.2 | 2980 | 64.2 |
| | PO:2 | | | | | | |
| **Compound 5** | IV:0.5 | 491 | 9.1 | 3.97 | 5.9 | 5317 | 64.0 |
| | PO:2 | | | | | | |
| **Compound 11** | IV:0.5 | 524 | 50.3 | 2.0 | 0.7 | 1115 | 62.2 |
| | PO:2 | | | | | | |
| **Compound 36** | IV:1 | 4750 | 6.9 | 0.8 | 1.7 | 29452 | 92.7 |
| | PO:5 | | | | | | |
| **Compound 41** | IV:1 | 4580 | 8.9 | 0.6 | 0.7 | 21441 | 78.2 |
| | PO:5 | | | | | | |

**Conclusion:** The compounds of the present disclosure exhibit favorable pharmacokinetic properties, including good oral bioavailability, oral exposure, half-life, and clearance.

### Assay Example 4: Drug-drug interaction evaluation

### Test Objective:

The cocktail method using specific probe substrates for CYP1A2, CYP2C9, CYP2C19, CYP2D6 and CYP3A was used to evaluate the inhibitory effect of the compounds on the activity of human liver microsomal cytochrome P450 isoenzymes (CYP1A2, CYP2C9, CYP2C19, CYP2D6 and CYP3A).

### Test Materials:

Human liver microsomes, probe substrates, positive control inhibitors, and reduced nicotinamide adenine dinucleotide phosphate (NADPH).

### Assay Procedure:

To the corresponding wells of a reaction plate, 20 µL of substrate solution was added; to blank wells, 20 µL of potassium phosphate buffer was added.

To the corresponding wells, 2 µL of test compound and positive control working solution were added; to inhibitor-free and blank wells, 2 µL of solvent was added.

Human liver microsome working solution was prepared, and 158 µL of human liver microsome working solution was added to each well of the reaction plate.

The plate was pre-incubated at 37.0°C for 10 minutes.

To start the reaction, 20 µL of NADPH cofactor working solution was added to the reaction plate.

After incubation in a 37.0°C water bath with mixing for 10 minutes, 400 µL of stop solution was added to terminate the reaction.

The sample plate was centrifuged at 4000 rpm for 20 minutes.

200 µL of supernatant was taken and mixed with 100 µL of pure water as diluent, and the plate was shaken to mix well.

Analysis was performed by liquid chromatography-tandem mass spectrometry (LC-MS/MS).

### Data analysis:

Using the percentage of remaining activity as the ordinate and the test compound concentration as the abscissa, a non-linear regression analysis was performed with a three-parameter or four-parameter model using XL fit software to calculate the IC₅₀ value. When the IC₅₀ value fitted by XL fit was greater than the highest tested concentration (50.0 µM) or the IC₅₀ value could not be obtained by fitting, the IC₅₀ value was marked as ">50.0 µM".

### Three-parameter equation:

$y = \frac{max}{1 + {\left(\frac{x}{{IC}_{50}}\right)}^{- hillslope}}$

### Four-parameter equation:

$y = min + \frac{max - min}{1 + {\left(\frac{x}{{IC}_{50}}\right)}^{- hillslope}}$

max = maximum enzyme activity; min = minimum enzyme activity; x = concentration of test compound or positive control inhibitor; y = enzyme activity at the corresponding concentration; hillslope = slope; IC₅₀ = half-maximal inhibitory concentration. The four-parameter equation was used when the minimum enzyme activity was within ±10%; otherwise the three-parameter equation was used.

The assay results are shown in Table 4 below.

**Table 4. Drug-drug interaction test results of the compounds of the present disclosure**

| Compound No. | CYP(µM)1A2/2C9/2C19/2D6/3A4 |
|---|---|
| Compound 5 | >50/ 10.4/ 18.8/ >50/ >50 |
| Compound 12 | 20/ 11/ 13/ 15/ 37 |
| Compound 26 | 19/ >50/ 44/ >50/ >50 |
| Compound 31 | >50/ >50/ >50/ >50/ >50 |
| Compound 32 | 13/ 11/ 28/ 36/ 21 |
| Compound 34 | 13/ 15/ >50/ >50/ >50 |
| Compound 35 | >50/ >50/ >50/ >50/ >50 |
| Compound 41 | 11/ 11/ >50/ >50/ >50 |

**Conclusion:** The compounds of the present disclosure exhibit low CYP inhibition and a low risk of drug-drug interactions.

### Assay Example 5: Automated patch clamp (Qpatch) assay for hERG potassium channel activity

### Test Method:

CHO-hERG cells were cultured in 175 cm² culture flasks. When the cell density reached 60-80%, the culture medium was removed, the cells were washed once with 7 mL of PBS, and then 3 mL of cell dissociation reagent was added for digestion. After complete digestion, 7 mL of culture medium was added to neutralize, and the cells were centrifuged. The supernatant was aspirated, and the cells were resuspended in 5 mL of culture medium to achieve a cell density of 2~5 × 10⁶/mL.

The compound stock solution was diluted with DMSO. 10 µL of compound stock solution was added to 20 µL of DMSO solution, followed by 3-fold serial dilution to obtain 6 DMSO concentrations. 4 µL of the compound solution at each of the six DMSO concentrations was added to 396 µL of extracellular solution to achieve a 100-fold dilution to obtain six intermediate concentrations. Then, 80 µL of the compound solution at each of the six intermediate concentrations was added to 320 µL of extracellular solution to achieve a 5-fold dilution to the desired final test concentrations. The highest test concentration was 40.00 µM, and there was a total of 6 concentrations: 40.00, 13.33, 4.44, 1.48, 0.49 and 0.16 µM. The DMSO content in the final test concentration did not exceed 0.2%. This concentration of DMSO had no effect on the hERG potassium channel. All dilutions in the compound preparation were performed by a Bravo instrument.

The electrophysiological process was recorded. The single-cell high-impedance sealing and whole-cell pattern formation processes were all automatically performed by a Qpatch instrument. After obtaining the whole-cell recording mode, the cells were clamped at - 80 mV. The cells were applied successively with a pre-voltage of -50 mV for 50 milliseconds and a depolarizing stimulus of +40 mV for 5 seconds, then repolarized to -50 mV for 5 seconds, and then back to -80 millivolts. This voltage stimulation was applied every 15 seconds, and recorded for 2 minutes. The extracellular solution was then given, and recorded for 5 minutes. Then a drug administration process started. The compound concentration started from the lowest test concentration, and each test concentration was administered for 2.5 minutes. At least 3 cells were tested for each concentration (n ≥ 3).

### Data analysis:

The assay data were analyzed using GraphPad Prism 5.0 software. The results are shown in Table 5.

**Table 5. IC₅₀ values of the compounds of the present disclosure on hERG potassium channels**

| **Compound No.** | **IC₅₀(µM)** |
|---|---|
| Compound 1 | >40 |
| Compound 5 | >40 |

**Conclusion:** The compounds of the present disclosure do not significantly inhibit hERG potassium channels and thus have a low risk of cardiotoxicity.

## Claims

1. A compound represented by formula (IV) or a pharmaceutically acceptable salt thereof, 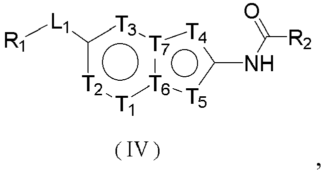 wherein,
L₁ is selected from CH₂, NH, O and S;
T₁, T₂ and T₃ are each independently selected from CR₃ and N;
T₄ and T₅ are each independently selected from CR₄, N and NR₄;
T₆ and T₇ are each independently selected from C and N;
R₁ is selected from phenyl, 5- to 10-membered heteroaryl, 6- to 10-membered heterocycloalkyl and 6- to 10-membered heterocycloalkenyl, wherein said phenyl, 5- to 10-membered heteroaryl, 6- to 10-membered heterocycloalkyl and 6- to 10-membered heterocycloalkenyl are each independently optionally substituted with 1, 2 or 3 R₁ₐ;
or, L₁ is absent, and R₁ is selected from 6- to 10-membered heterocycloalkyl and 6- to 10-membered heterocycloalkenyl, wherein said 6- to 10-membered heterocycloalkyl and 6- to 10-membered heterocycloalkenyl are each independently optionally substituted with 1, 2 or 3 R₁ₐ;
R₂ is selected from C₁₋₆ alkyl, -C₁₋₃ alkyl-C₃₋₆ cycloalkyl, -C₁₋₃ alkyl-(4- to 6-membered heterocycloalkyl) and C₃₋₆ cycloalkyl, wherein said C₁₋₆ alkyl, -C₁₋₃ alkyl-C₃₋₆ cycloalkyl, -C₁₋₃ alkyl-(4- to 6-membered heterocycloalkyl) and C₃₋₆ cycloalkyl are each independently optionally substituted with 1, 2, 3, 4 or 5 R₂ₐ;
R₃ is selected from H, F, Cl, Br, I, CN, C₁₋₄ alkyl, C₁₋₄ alkoxy, C₃₋₆ cycloalkyl and 4- to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₃ₐ;
R₄ is selected from H, F, Cl, Br, I, CN, C₁₋₄ alkyl, C₁₋₄ alkoxy, C₃₋₆ cycloalkyl and 4- to 6-membered heterocycloalkyl, wherein said C₁₋₄ alkyl, C₁₋₄ alkoxy, C₃₋₆ cycloalkyl and 4- to 6-membered heterocycloalkyl are each independently optionally substituted with 1, 2 or 3 R₄ₐ; each R₁ₐ, each R₂ₐ, each R₃ₐ and each R₄ₐ are each independently selected from H, F, Cl, Br, I, CN, C₁₋₄ alkyl and C₁₋₄ alkoxy, wherein said C₁₋₄ alkyl and C₁₋₄ alkoxy are each independently optionally substituted with 1, 2, 3, 4 or 5 R;
or, two R₁ₐ together with the atoms to which they are attached form a C₅₋₆ cycloalkyl, C₅₋₆ cycloalkenyl, phenyl or 5- to 6-membered heteroaryl, wherein said C₅₋₆ cycloalkyl, C₅₋₆ cycloalkenyl, phenyl or 5- to 6-membered heteroaryl is optionally substituted with 1, 2 or 3 R; each R is independently selected from H, F, Cl, Br and I.

2. The compound according to claim 1 or a pharmaceutically acceptable salt thereof, wherein, each R₁ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R; or, each R₁ₐ is independently selected from H, F, Cl, CH₃, CH₂F, CHF₂, CF₃ and OCH₃.

3. The compound according to claim 1 or a pharmaceutically acceptable salt thereof, wherein, two R₁ₐ together with the atoms to which they are attached form a cyclopentenyl, cyclohexenyl, phenyl or thienyl, wherein said cyclopentenyl, cyclohexenyl, phenyl or thienyl is optionally substituted with 1, 2 or 3 R.

4. The compound according to claim 1 or a pharmaceutically acceptable salt thereof, wherein, each R₂ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R; or, each R₂ₐ is independently selected from H, F, Cl, CH₃, CH₂F, CHF₂ and CF₃.

5. The compound according to claim 1 or a pharmaceutically acceptable salt thereof, wherein, each R₃ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R; or, each R₃ₐ is independently selected from H, F, Cl, CH₃, CH₂F, CHF₂ and CF₃.

6. The compound according to claim 1 or a pharmaceutically acceptable salt thereof, wherein, each R₄ₐ is independently selected from H, F, Cl, CN, CH₃, CH₂CH₃, CH₂CH₂CH₃, CH(CH₃)₂, OCH₃, OCH₂CH₃, OCH₂CH₂CH₃ and OCH(CH₃)₂, wherein said CH₃, CH₂CH₃, CH₂CH₂CH₃, CH(CH₃)₂, OCH₃, OCH₂CH₃, OCH₂CH₂CH₃ and OCH(CH₃)₂ are each independently optionally substituted with 1, 2, 3, 4 or 5 R; or, each R₄ₐ is independently selected from H, F, Cl, CH₃, CH₂F, CHF₂ and CF₃.

7. The compound according to claim 1 or a pharmaceutically acceptable salt thereof, wherein, L₁ is selected from NH.

8. The compound according to claim 1 or a pharmaceutically acceptable salt thereof, wherein, R₁ is selected from phenyl, pyridinyl, pyrimidinyl, pyrazinyl, pyridazinyl, thienyl, thiazolyl, pyrrolyl, imidazolyl, pyrazolyl, 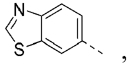 piperidinyl, piperazinyl, morpholinyl, homopiperidinyl, homopiperazinyl, 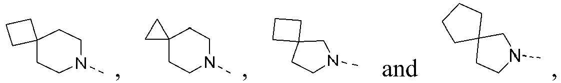 wherein said phenyl, pyridinyl, pyrimidinyl, pyrazinyl, pyridazinyl, thienyl, thiazolyl, pyrrolyl, imidazolyl, pyrazolyl, 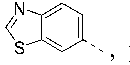 piperidinyl, piperazinyl, morpholinyl, homopiperidinyl, homopiperazinyl, 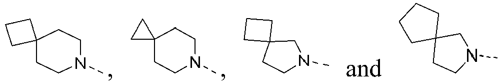 are each independently optionally substituted with 1, 2 or 3 R₁ₐ, or, R₁ is selected from 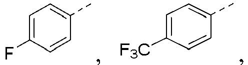 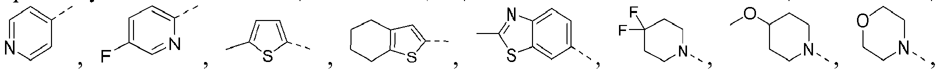 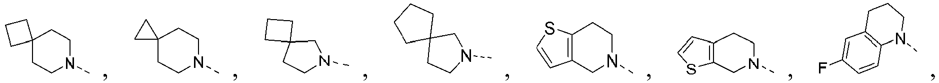 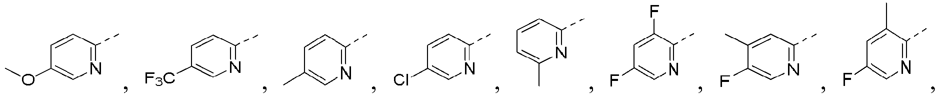 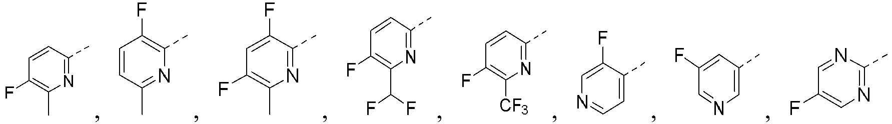 and

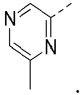

9. The compound according to claim 1 or a pharmaceutically acceptable salt thereof, wherein, R₂ is selected from CH₂CH₃, CH₂C(CH₃)₃, -CH₂-cyclopropyl, -CH₂-cyclobutyl, 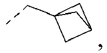 cyclopropyl, cyclobutyl, cyclopentyl, cyclohexyl and 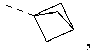 wherein said CH₂CH₃, CH₂C(CH₃)₃, -CH₂-cyclopropyl, -CH₂-cyclobutyl, 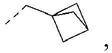 cyclopropyl, cyclobutyl, cyclopentyl, cyclohexyl and 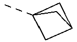 are each independently optionally substituted with 1, 2, 3, 4 or 5 R₂ₐ; or, R₂ is selected from CH₂CH₃, CH₂C(CH₃)₃, 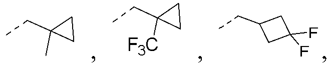 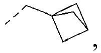 cyclopropyl, cyclobutyl, cyclopentyl and

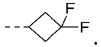

10. The compound according to claim 1 or a pharmaceutically acceptable salt thereof, wherein, R₃ is selected from H, F, Cl, CN, CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 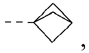 azetidinyl and oxetanyl, wherein said CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 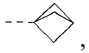 azetidinyl and oxetanyl are each independently optionally substituted with 1, 2 or 3 R₃ₐ; or, R₃ is selected from H, F, Cl, CN, CH₃ and CF₃.

11. The compound according to claim 1 or a pharmaceutically acceptable salt thereof, wherein, R₄ is selected from H, F, Cl, CN, CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 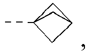 azetidinyl and oxetanyl, wherein said CH₃, CH₂CH₃, CH₂CH₂CH₃, CH(CH₃)₂, C(CH₃)₃, OCH₃, OCH₂CH₃, OCH₂CH₂CH₃, OCH(CH₃)₂, cyclopropyl, cyclobutyl, 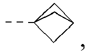 azetidinyl and oxetanyl are each independently optionally substituted with 1, 2 or 3 R₄ₐ; or, R₄ is selected from H, F, Cl, CN, CH₃, CF₃, CH₂CH₃, C(CH₃)₃,

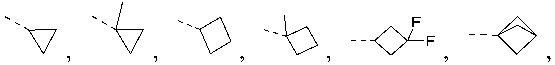

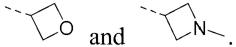

12. The compound according to claim 1 or a pharmaceutically acceptable salt thereof, wherein, the structural unit 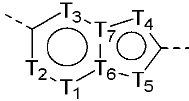 is selected from 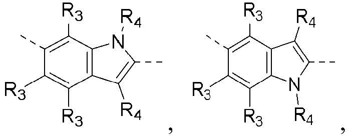 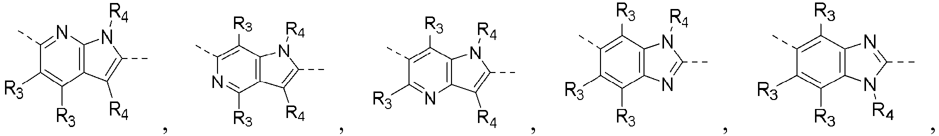 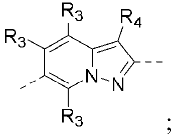 or, the structural unit 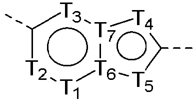 is selected from

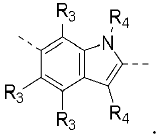

13. A compound according to any one of claims 1 to 12, or a pharmaceutically acceptable salt thereof, which is selected from: 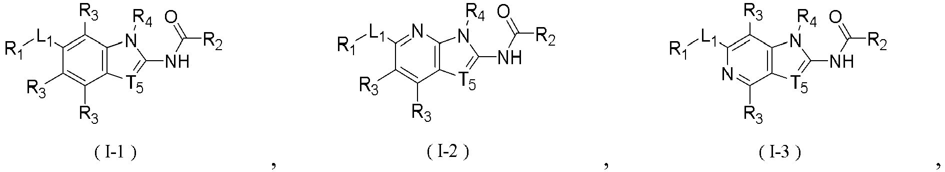 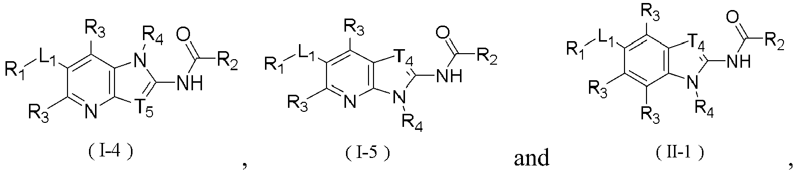 wherein,
T₄ and T₅ are each independently selected from CR₄ and N;
R₁, R₂, R₃, R₄ and L₁ are as defined in any one of claims 1 to 12.

14. The compound according to claim 13, or a pharmaceutically acceptable salt thereof, wherein the compound represented by formula (I-1) or (II-1), or a pharmaceutically acceptable salt thereof, is selected from: 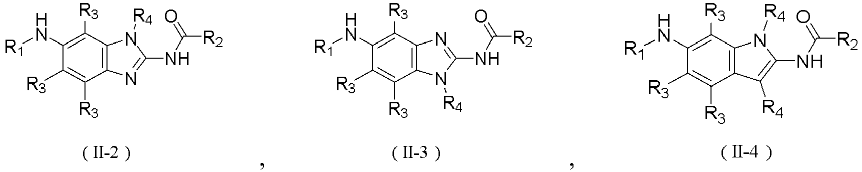 and 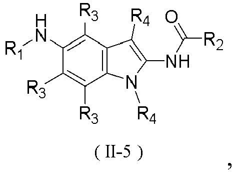 wherein,
R₁ is selected from phenyl, 5- to 6-membered heteroaryl and 6- to 10-membered heterocycloalkyl, wherein said phenyl, 5- to 6-membered heteroaryl and 6- to 10-membered heterocycloalkyl are each independently optionally substituted with 1, 2 or 3 R₁ₐ;
R₂ is selected from CH₂C(CH₃)₃, 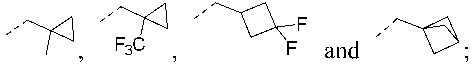
R₃ is selected from H, F, Cl, CN, CH₃ and CF₃;
R₄ is selected from H, F, Cl, CH₃, C(CH₃)₃, cyclopropyl, cyclobutyl, 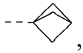 azetidinyl and oxetanyl, wherein said CH₃, C(CH₃)₃, cyclopropyl, cyclobutyl, 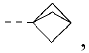 azetidinyl and oxetanyl are each independently optionally substituted with 1, 2 or 3 R₄ₐ;
each R₁ₐ is independently selected from H, F, CH₃, CF₃ and OCH₃;
each R₄ₐ is independently selected from H, F, CH₃ and CF₃.

15. The compound according to claim 13 or 14, or a pharmaceutically acceptable salt thereof, wherein the compound of formula (I-1) or (II-4), or a pharmaceutically acceptable salt thereof, is selected from: 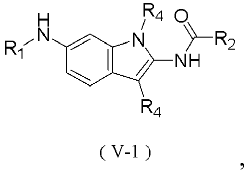 wherein,
R₁ is phenyl and 5- to 6-membered heteroaryl, wherein said phenyl and 5- to 6-membered heteroaryl are each independently optionally substituted with 1, 2 or 3 R₁ₐ;
R₂ is selected from CH₂C(CH₃)₃ and 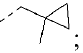
R₄ is selected from H, CH₃, cyclopropyl, cyclobutyl and 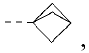 wherein said CH₃, cyclopropyl, cyclobutyl and 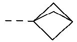 are each independently optionally substituted with 1, 2 or 3 R₄ₐ;
each R₁ₐ is independently selected from H, F, CH₃, CF₃ and OCH₃;
each R₄ₐ is independently selected from H and F.

16. The compound according to claim 15 or a pharmaceutically acceptable salt thereof, wherein, R₁ is 5- to 6-membered heteroaryl, wherein said 5- to 6-membered heteroaryl is optionally substituted with 1, 2 or 3 R₁ₐ; or, R₁ is pyridinyl, wherein said pyridinyl is optionally substituted with 1, 2 or 3 R₁ₐ.

17. A compound as shown below or a pharmaceutically acceptable salt thereof,

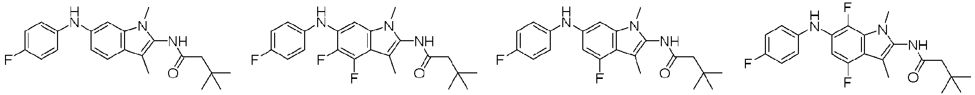

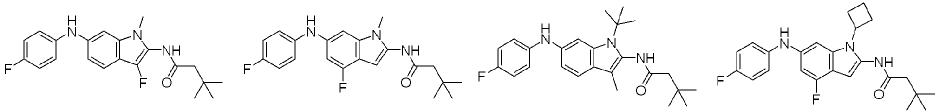

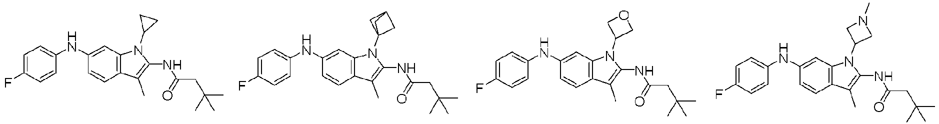

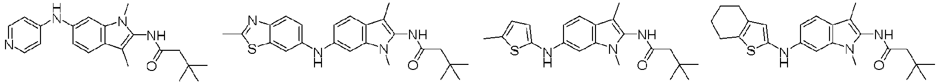

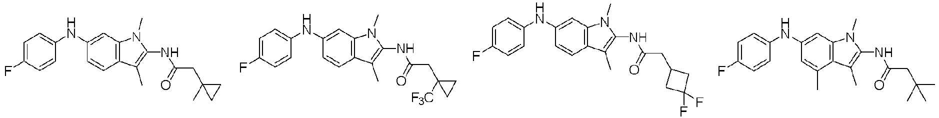

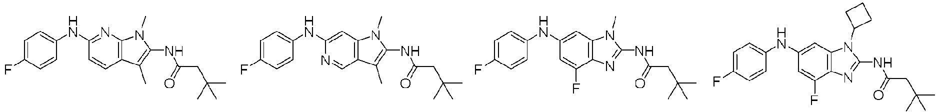

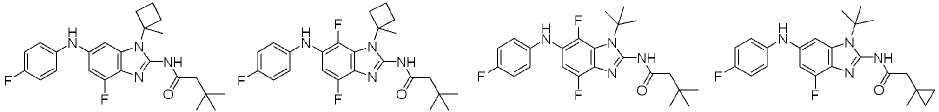

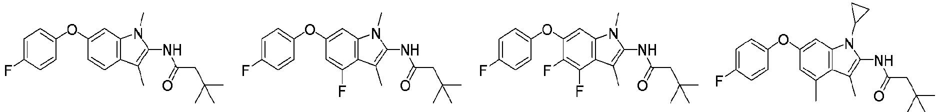

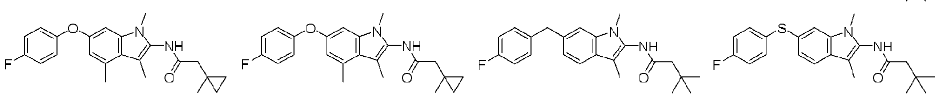

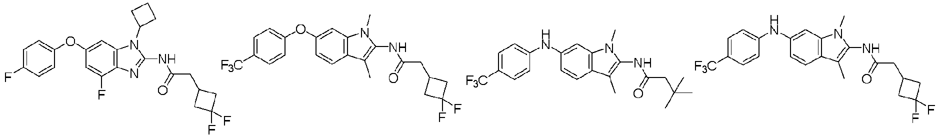

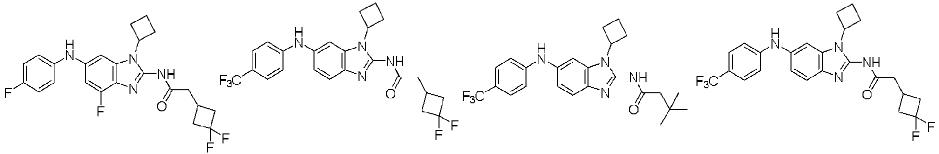

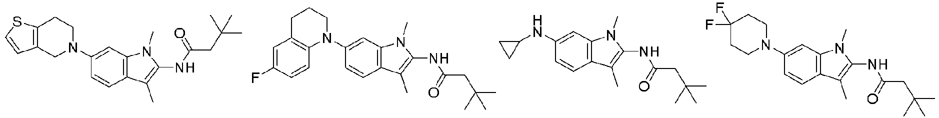

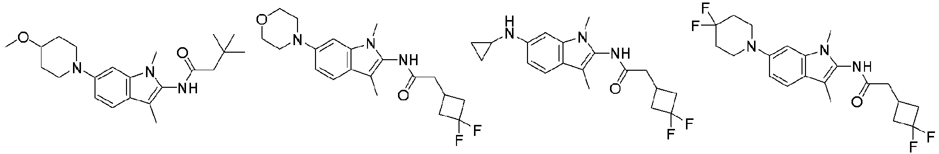

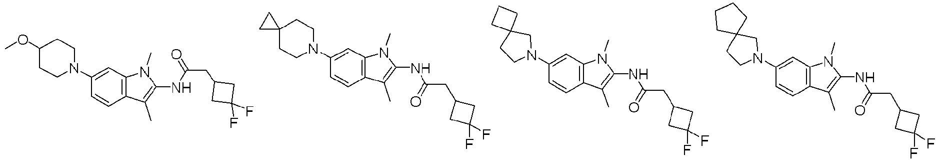

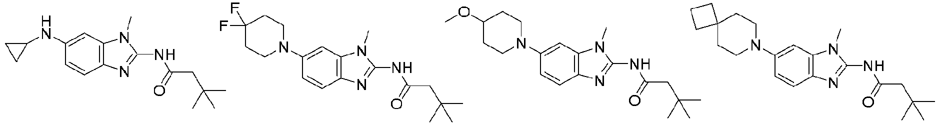

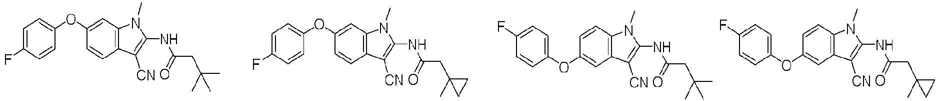

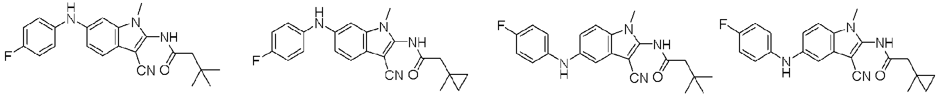

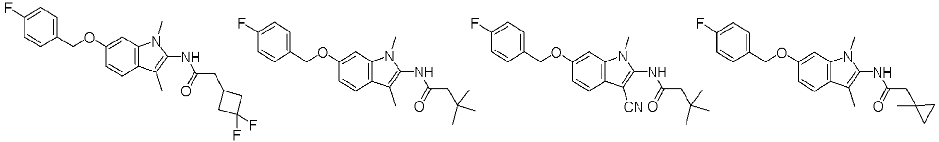

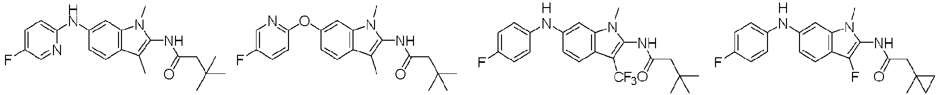

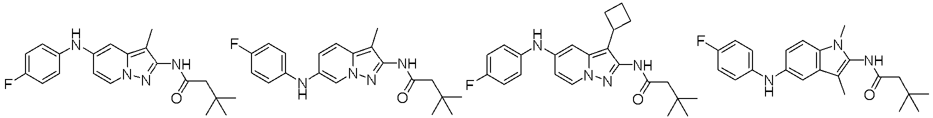

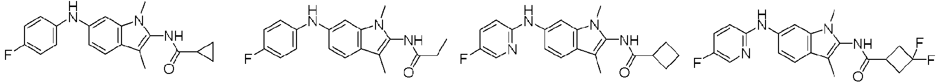

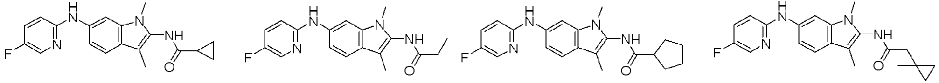

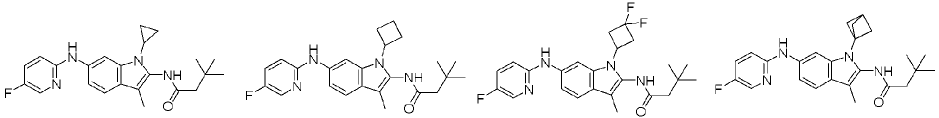

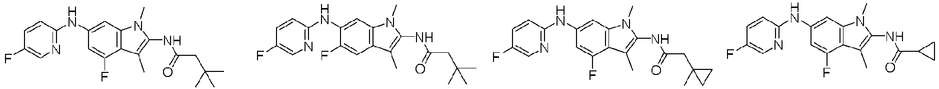

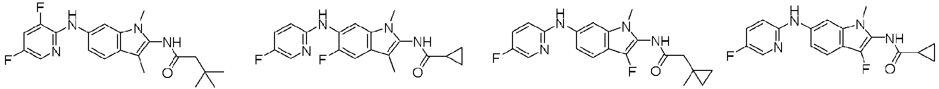

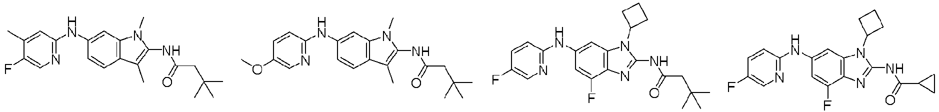

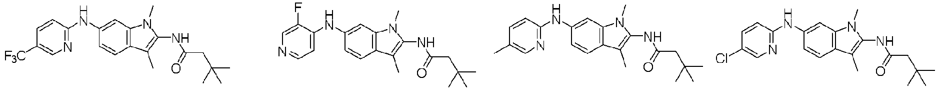

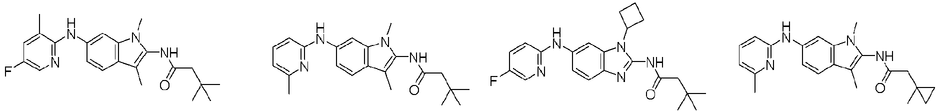

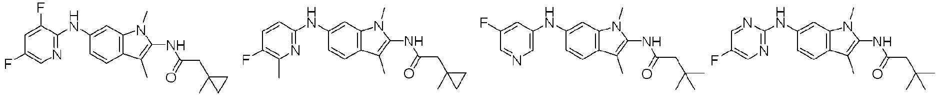

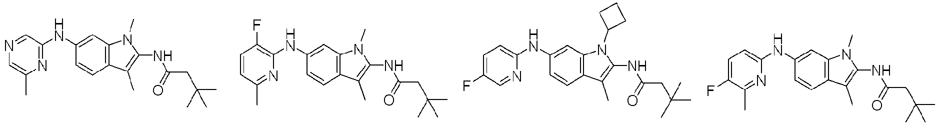

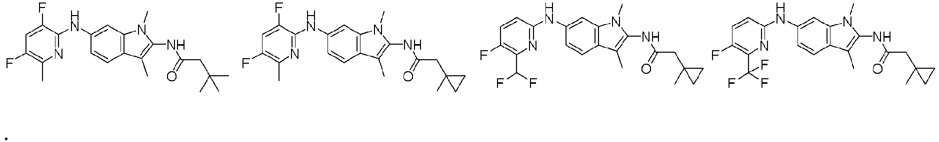

18. A pharmaceutical composition comprising the compound of any one of claims 1-17, or a pharmaceutically acceptable salt thereof.

19. Use of the compound of any one of claims 1-17, or a pharmaceutically acceptable salt thereof, or the pharmaceutical composition of claim 18, in the manufacture of a medicament for treating a disease associated with Kv7 potassium ion channel openers.

20. The use according to claim 19, wherein the disease associated with Kv7 potassium ion channel openers is selected from epilepsy.
